(19) Europäisches Patentamt
European Patent Office
Office européen des brevets

(11) **EP 4 556 011 A1**

(12) **EUROPEAN PATENT APPLICATION**
published in accordance with Art. 153(4) EPC

(43) Date of publication:
**21.05.2025 Bulletin 2025/21**

(21) Application number: **23839067.8**

(22) Date of filing: **14.07.2023**

(51) International Patent Classification (IPC):
**A61K 33/32** (2006.01)  **A61K 33/00** (2006.01)
**A61P 19/06** (2006.01)

(52) Cooperative Patent Classification (CPC):
**A61K 33/00; A61K 33/32; A61P 19/06**

(86) International application number:
**PCT/CN2023/107543**

(87) International publication number:
**WO 2024/012580 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.07.2022 PCT/CN2022/106050**

(71) Applicant: **Shanghai Shiqu Pharmaceutical Technology Co., Ltd.**
**Shanghai 201114 (CN)**

(72) Inventors:
• **ZHANG, Shiyi**
  **Shanghai 201114 (CN)**
• **ZHAO, Xin**
  **Shanghai 201114 (CN)**
• **ZENG, Xiaodong**
  **Shanghai 201114 (CN)**

(74) Representative: **Mewburn Ellis LLP**
**Aurora Building**
**Counterslip**
**Bristol BS1 6BX (GB)**

(54) **USE OF MANGANESE-CONTAINING COMPOUND IN REDUCING LEVEL OF URIC ACID OF MAMMALIAN SUBJECT**

(57)    The present invention relates to use of a manganese-containing compound in reducing the level of uric acid level in a mammalian subject, particularly to use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject, a pharmaceutical composition and a kit for such use, and a method for preventing and/or treating the related diseases or disorders.

**FIG. 22**

**EP 4 556 011 A1**

**Description**

**TECHNICAL FIELD**

The present invention generally relates to use of a manganese-containing compound in reducing uric acid levels in a mammalian subject, particularly to use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject, a pharmaceutical composition and a kit for such use, and a method for preventing and/or treating the related diseases or disorders.

**BACKGROUND ART**

Uric acid, a heterocyclic compound containing nitrogen and oxygen with the molecular formula of $C_5H_4N_4O_3$, is the final metabolite of purine in the body. Disorders associated with abnormally increased uric acid levels in the blood and/or serum mainly include hyperuricemia, gout, and its complications, such as gouty arthritis (with or without gout stones), gouty urinary stones (i.e. gouty urolithiasis), gouty nephropathy (i.e. urate nephropathy), and sequelae of these disorders. Hyperuricemia is associated with an increased risk of developing gout. The more severe and prolonged a subject's hyperuricemia, the higher the risk of developing gout. In addition to gouty arthritis, chronic hyperuricemia can lead to the deposition of urate crystals in the urinary tract, renal parenchyma, and soft tissues, resulting in gouty urolithiasis, gouty nephropathy, and soft tissue tophi, respectively.

At present, the clinical treatment of hyperuricemia or gout mainly includes the administration of drugs for reducing the concentration of uric acid in the blood. In addition, symptoms of acute gout can be controlled by the administration of anti-inflammatory drugs. These drugs are mainly used to fight inflammation but do not have the effect of lowering uric acid levels. Reducing the uric acid level in a subject's blood and/or serum to the normal range reduces the recurrence rate of acute gout and prevents other diseases or disorders associated with increased uric acid levels. To date, there are still many patients who cannot effectively control uric acid levels after standard treatment with existing drugs. In addition, many currently available medications for hyperuricemia or gout are associated with a variety of harmful side effects.

The level of uric acid in a subject's blood and/or serum depends on the balance between the amount of purines obtained through food or drink intake, the amount of uric acid produced in the body, and the amount of uric acid excreted through the urine or digestive tract. Regarding the production of uric acid in the body, about 80% of uric acid is synthesized by the body itself, while about 20% of uric acid enters the body through intestinal ingestion (Current hypertension reports, 2016, 18(10): 1-6.). Some patients with hyperuricemia have increased intestinal uric acid intake due to factors such as dietary habits and intestinal lesions. On the other hand, regarding the excretion of uric acid in the body, in normal people, about 70% of uric acid is excreted through the kidneys, while about 30% of uric acid is still excreted through the intestines. For patients with chronic kidney disease, due to the decreased ability of the kidneys to clear uric acid, intestinal excretion of uric acid becomes the main route of uric acid excretion (Mini Reviews in Medicinal Chemistry, 2020, 20(18): 1857-1866.). It can be seen that by removing uric acid in the digestive tract, it may help to reduce the uric acid level in a subject's body. On the other hand, drugs that only work locally in the digestive tract (such as Veltassa and Lokelma, drugs for treating hyperkalemia) are not absorbed into the blood and do not cause systemic side effects, so they have good safety. Therefore, it is of great significance to develop a uric acid-lowering drug based on the mechanism of clearing uric acid in the digestive tract, which will become a good supplement to existing uric acid-lowering drugs.

**SUMMARY OF THE INVENTION**

The present application is based at least in part on the inventors' unexpected discovery that manganese-containing compounds have excellent properties for adsorbing and/or removing uric acid (including its salts, such as monosodium urate), and that, compared with materials with adsorption properties known in the prior art, the adsorption and/or clearance of uric acid (including its salts, such as monosodium urate) by manganese-containing compounds is not, is substantially not, or is less affected by various substances present in the digestive tract of mammals (including but not limited to digestive juices such as gastric juice, small intestinal juice, and chyme). Furthermore, the inventors have unexpectedly discovered that manganese-containing compounds can be administered at very high safe oral doses without causing unacceptable side effects, thereby having a desirable safety profile. Based on the above findings, a technical solution that meets the above and other needs is provided below.

In one aspect, the present disclosure provides use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject.

In another aspect, the present disclosure provides use of a manganese-containing compound in the preparation of a drug for adsorbing uric acid in the digestive tract of a mammal.

In another aspect, the present disclosure provides a pharmaceutical composition for preventing and/or treating increased uric acid levels in the blood and/or digestive tract or a disease or disorder associated with increased uric acid levels in the blood and/or digestive tract in a mammalian subject, wherein the pharmaceutical composition comprises an insoluble manganese-containing compound and a pharmaceutically acceptable excipient.

In another aspect, the present disclosure provides a kit comprising: (i) any of the pharmaceutical compositions according to the present disclosure; and (ii) drug instructions and/or drug labels.

In another aspect, the present disclosure provides a method for preventing and/or treating increased uric acid levels in the blood and/or digestive tract or a disease or disorder associated with increased uric acid levels in the blood and/or digestive tract in a mammalian subject, which comprises administering an effective amount of an insoluble manganese-containing compound to a subject in need thereof.

In another aspect, the present disclosure provides a method for preventing and/or treating increased uric acid levels in the blood and/or digestive tract or a disease or disorder associated with increased uric acid levels in the blood and/or digestive tract in a mammalian subject, which comprises administering an effective amount of an insoluble manganese-containing compound to a subject in need thereof over an extended period of time.

In another aspect, the present disclosure provides a method for maintaining uric acid levels in the blood in a mammalian subject, comprising administering to a subject in need thereof an effective amount of an insoluble manganese-containing compound over an extended period of time.

In another aspect, the present disclosure provides a pharmaceutical composition, wherein the amount of manganese oxide is sufficient to reduce blood and/or serum uric acid levels by at least about 5% in a mammalian subject whose blood and/or serum uric acid levels are at least 10% higher than 360 $\mu$mol/L or 420 $\mu$mol/L within 24 hours after administration of the pharmaceutical composition.

In another aspect, the present disclosure provides a pharmaceutical composition formulated as at least one solid dose unit for oral administration to a mammalian subject, wherein the solid dose unit comprises manganese oxide, and wherein the manganese oxide has a uric acid clearance capacity measured in a fasted state simulated intestinal fluid (FaSSIF) or fed state simulated intestinal fluid (FeSSIF) assay that is at least about 100 mg/g higher than the uric acid clearance capacity of activated charcoal measured under the same conditions.

Descriptive embodiments of the present disclosure are illustrated and described in details below, from which other aspects and advantages of the present disclosure will be apparent to those skilled in the art. As one skilled in the art will appreciate, the attached drawings and description are exemplary and not restrictive.

## BRIEF DESCRIPTION OF THE DRAWINGS

By referring to the exemplary embodiments and drawings detailed below, it will be possible to better understand the features and advantages of the invention to which the present application relates. The following is a brief description of the information in each of the appended drawings. It is to be understood that the drawings are illustrative in nature and do not constitute a limitation on any aspect of the invention to which the present application relates.

Fig. 1 shows the uric acid clearance capacity (mg/g) and uric acid clearance rate (%) of the test substances as measured in a simulated intestinal fluid (SIF) assay according to Section 2.1 of Example 2.

Fig. 2 shows the uric acid clearance rate (%) of the test substances as measured in a simulated intestinal fluid (SIF) assay according to Section 2.2 of Example 2.

Fig. 3 shows changes in the uric acid clearance rate (%) with mixing time of the test substances as measured in a simulated intestinal fluid (SIF) assay according to Section 2.3 of Example 2.

Fig. 4 shows the uric acid clearance capacity (mg/g) of the test substances as measured in a simulated intestinal fluid (SIF) assay according to Section 2.4 of Example 2.

Fig. 5 shows the uric acid clearance capacity (mg/g) of the test substances at different uric acid concentrations ($\mu$M) as measured in a simulated intestinal fluid (SIF) assay according to the first part of Section 2.5 of Example 2.

Fig. 6 shows the uric acid clearance capacity (mg/g) of the test substances at different uric acid concentrations ($\mu$M) as measured in a fasted state simulated intestinal fluid (FaSSIF) assay according to the first part of Section 2.5 of Example 2.

Fig. 7 shows the uric acid clearance capacity (mg/g) of the test substances at different uric acid concentrations ($\mu$M) as measured in a fed state simulated intestinal fluid (FeSSIF) assay according to the first part of Section 2.5 of Example 2.

Fig. 8 shows the uric acid clearance capacity (mg/g) of the test substances at different uric acid concentrations ($\mu$M) as measured in a simulated intestinal fluid (SIF) assay according to the second part of Section 2.5 of Example 2.

Fig. 9 shows the uric acid clearance rate (%) of the test substances at different uric acid concentrations ($\mu$M) as measured in a simulated intestinal fluid (SIF) assay according to the second part of Section 2.5 of Example 2.

Fig. 10 shows the uric acid clearance capacity (mg/g) of the test substances at different uric acid concentrations ($\mu$M) as measured in a fasted state simulated intestinal fluid (FaSSIF) assay according to the second part of Section 2.5 of Example 2.

Fig. 11 shows the uric acid clearance rate (%) of the test substances at different uric acid concentrations ($\mu$M) as measured in a fasted state simulated intestinal fluid (FaSSIF) assay according to the second part of Section 2.5 of Example 2.

Fig. 12 shows the uric acid clearance capacity (mg/g) of the test substances at different uric acid concentrations ($\mu$M) as measured in a fed state simulated intestinal fluid (FeSSIF) assay according to the second part of Section 2.5 of Example 2.

Fig. 13 shows the uric acid clearance rate (%) of the test substances at different uric acid concentrations ($\mu$M) as measured in a fed state simulated intestinal fluid (FeSSIF) assay according to the second part of Section 2.5 of Example 2.

Fig. 14 shows the uric acid clearance capacity (mg/g) of the test substances as measured in a pork enzymatic hydrolysate assay according to Section 2.6 of Example 2.

Fig. 15 shows the uric acid clearance capacity (mg/g) of the test substances as measured in a food enzymatic hydrolysate assay according to Section 2.7 of Example 2.

Fig. 16 shows the uric acid clearance capacity (mg/g) of the test substances as measured in a simulated intestinal fluid (SIF) assay according to Section 2.8 of Example 2.

Fig. 17 shows the uric acid clearance capacity (mg/g) of the test substances as measured in a simulated intestinal fluid (SIF) assay according to Section 2.9 of Example 2.

Fig. 18 shows the uric acid clearance rate (%) of the test substances as measured in a simulated intestinal fluid (SIF) assay according to Section 2.9 of Example 2.

Fig. 19 shows the measured values of uric acid clearance capacity (mg/g) of the test substance measured in each of the 12 times of simulated intestinal fluid (SIF) assay and the cumulative uric acid clearance capacity (mg/g) for the 12 times of assay according to Section 2.10 of Example 2.

Fig. 20 shows the amount of uric acid excreted via urine within 24 hours (mg/day) of each group of mice measured in the oteracil potassium and adenine induced hyperuricemia model mouse experiment according to Section 4.4.2 of Example 4.

Fig. 21 shows a curve of the change in serum uric acid levels ($\mu$M) over time in each group of mice measured in the uric acid-induced hyperuricemia model mouse experiment according to Section 4.4.3 of Example 4.

Fig. 22 shows the serum uric acid levels ($\mu$M) of each group of mice as measured in the oteracil potassium and hypoxanthine induced hyperuricemia model mouse experiment according to Section 4.4.4 of Example 4.

Fig. 23 shows the serum uric acid levels ($\mu$M) of each group of mice as measured in the oteracil potassium and uric acid induced hyperuricemia model mouse experiment according to Section 4.4.5 of Example 4.

## DETAILED DESCRIPTION OF THE INVENTION

Various exemplary embodiments or examples of the invention to which the present application relates are disclosed below. It should be understood that these embodiments or examples are merely illustrative and are not intended to limit the scope of protection of the invention involved in the present application. Many changes, modifications, and substitutions of these exemplary embodiments or examples will occur to those skilled in the art without departing from the spirit of the disclosure. It should be understood that these embodiments or examples obtained through changes, modifications or substitutions also fall within the scope of protection of the present application.

Where publications, patents, and patent applications are cited in the specification, all of the cited publications, patents, and patent applications are herein incorporated by reference in their entirety to the same extent as if each subj ect publication, patent, or patent application is specifically and individually indicated to be incorporated by reference in its entirety. To the extent that publications, patents, and patent applications incorporated by reference conflict with the disclosure contained in the specification, the interpretations and/or descriptions in the specification shall prevail.

Where the specification mentions a numerical range, it should be understood that all numerical ranges mentioned in the present disclosure are intended to include the two endpoints of the range (i.e. the upper limit and the lower limit), all numerical values within the range (especially integers) and sub-ranges formed by these numerical values (especially integers).

Definition

As used throughout this document, the singular forms "a", "an", "said", and "the" include plural referents as well, unless the context clearly dictates otherwise. In addition, unless it is specifically described whether a certain referent is singular or plural, the present application includes both the singular referent and the plural referents.

As used throughout this document, the term "multiple/a plurality of" refers to more than one, including but not limited to two, three, four, five, six, etc.

As used throughout this document, the terms "include", "comprise", "have", "contain" and variations thereof are intended to represent open-ended transitional phrases, terms, or words that do not exclude the possibility of additional steps, materials, or structures. When such terms are used to describe a certain drug, pharmaceutical composition, kit, use or method, etc. of the present disclosure, it also covers the situation that the drug, pharmaceutical composition, kit, use or method, etc. is composed of the listed factors or elements. In the context of the present disclosure, the term "consisting of" means that in addition to the listed factors or elements, unlisted factors or elements (e.g. steps, materials, structures, etc.) are not present in the subject matter or method.

As used throughout this document, the term "optionally" means that the subsequently described element or event may or may not exist/occur, and includes both the presence/occurrence and absence/occurrence of the element or event.

As used throughout this document, the term "about" means approximately, in the region of, roughly, or around and is used to indicate that the characteristic being described is within an acceptable error range for a person of ordinary skill in the art for a given value. When the term "about" is used in reference to a numerical value, it is used to indicate any value within the range of $\pm 20\%$ of the recited numerical value. In some embodiments, the term "about" can be used to encompass any value within a range of $\pm 10\%$, $\pm 5\%$, $\pm 1\%$, 0.5%, or 0.1% of a particular value.

As used throughout this document, the term "manganese-containing compound" refers to a chemical substance composed of manganese and any other one or more elements (usually in a fixed mass ratio) bonded together by chemical bonds (i.e. the forces binding the atoms or ions constituting the compound, including but not limited to covalent bonds and ionic bonds).

As used throughout this document, the term "insoluble manganese-containing compound" refers to a manganese-containing compound that is insoluble or has extremely low solubility in water and/or digestive tract fluids (including but not limited to gastric juice, small intestinal juice, large intestinal juice, pancreatic juice and/or bile) under physiological conditions. The insoluble manganese-containing compounds according to the present disclosure can be identified by measuring the mass of the manganese-containing compound dissolved in 1 mL of water or a simulated digestive tract fluid (including but not limited to simulated intestinal fluid (SIF), fasted state simulated intestinal fluid (FaSSIF) or fed state simulated intestinal fluid (FeSSIF)) when saturated at about 37°C and about 1 atmosphere. When the mass of the manganese-containing compound dissolved under the above conditions is less than about 100 $\mu$g, for example, less than about 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng or 1 ng, it can be regarded as the "insoluble manganese-containing compound" according to the present disclosure. On the other hand, "insoluble manganese-containing compounds" can also be characterized by their physiological inertness, that is, they are essentially unable to enter the blood circulation through the gastrointestinal tract (i.e. the digestive tract) and/or they do not substantially react chemically with surrounding substances or tissues in the body after administration. For example, an "insoluble manganese-containing compound" can be a manganese-containing compound that enters the blood circulation through absorption in the gastrointestinal tract in an amount less than about 20% of the dose, such as less than about 15%, 10%, 5%, 4%, 3%, 2%, 1%, 0.5%, 0.1%, 0.05%, 0.02% or 0.01% of the dose administered and/or a manganese-containing compound that does not chemically react with surrounding substances or tissues in the body after administration or chemically reacts with surrounding substances or tissues in the body after administration only in an amount less than about 20% of the dose, such as less than about 15%, 10%, 5%, 4%, 3%, 2%, 1%, 0.5%, 0.1%, 0.05%, 0.02% or 0.01% of the dose administered. Without being limited by theory, due to its physiological inertness and insolubility, at least 80%, 85%, 90%, 95%, 96%, 97%, 98% or 99% of the amount of the insoluble manganese-containing compound of the present application administered is excreted from the body. As non-limiting examples, the insoluble manganese-containing compound is selected from one or more of manganese oxide, manganese sulfide, manganese carbonate, manganese silicate, manganese borate, manganese ferrocyanate, manganese oxalate, manganese selenite, manganese iodate, manganese tungstate and manganese phosphate and their solvates (e.g. hydrates).

As used throughout this document, the terms "granularity" and "particle size" are used interchangeably and refer to a measurement of particle size (or dimensions) characterizing a population of particles (e.g. a population of particles of insoluble manganese-containing compounds in the context of the present disclosure). Methods for measuring particle size are known in the art. For example, dynamic image analysis (DIA) can be used to measure the particle size of a population of particles. For example, dynamic light scattering can be used to measure the particle size of a population of particles. For example, a laser particle size analyzer (also called laser diffraction particle size analyzer) may be used to measure the particle size of a population of particles. When referring to the particle size range of a certain population of particles in the context of the present disclosure, it should be understood that there may still be a small amount of particles that do not meet the particle size range in the population of particles (for example, the particles that do not meet the particle size range account for $\leq 5\%$, $\leq 4\%$, $\leq 3\%$, $\leq 2\%$, $\leq 1\%$, $\leq 0.9\%$, $\leq 0.8\%$, $\leq 0.7\%$, $\leq 0.6\%$, $\leq 0.5\%$, $\leq 0.4\%$, $\leq 0.5\%$, $\leq 0.4\%$, $\leq 0.3\%$, $\leq 0.2\%$ or $\leq 0.1\%$ of the total amount of the population of particles), but the presence of these particles will not substantially affect the overall properties of the population of particles.

As used throughout this document, the term "manganese oxide" refers to a chemical substance composed of at least one manganese (Mn) atom, at least one oxygen (O) atom, and optionally other metals (including atomic groups with metal-like properties, such as ammonia) or hydrogen (represented by M; M=H, K, Na, Li, Ba, Pb, Ca, Zn, etc.) atoms bonded together by chemical bonds (Note: the "atom" mentioned here also includes charged ions formed by losing or gaining one or more electrons, and for the purpose of the present disclosure, metal ions also include ammonium ions). Manganese oxide is usually a black or brown solid. Its basic structural unit is usually an octahedral unit [$MnO_6$] formed by one manganese atom coordinated with six oxygen atoms, which forms various crystal structures by stacking by sharing edges or angles. In the stacked structure, the atomic layers form tetrahedral and octahedral holes. In manganese oxide, manganese can assume oxidation states of +2, +3, +4, +6, +7, so that manganese oxide can exist in a variety of forms or mixtures of these forms. In addition, various cations can be incorporated into the holes between the atoms in the structure of manganese oxide; herein, such manganese oxide doped with impurity cations is referred to as "salt-type manganese oxide". In some embodiments of the present disclosure, the manganese oxide is: (1) manganese oxide consisting only of manganese atoms and oxygen atoms, such as manganese monoxide (MnO), manganese dioxide ($MnO_2$), monomanganese trioxide ($MnO_3$), diamanganese trioxide ($Mn_2O_3$), pentamanganese octoxide ($Mn_5O_8$), trimanganese tetraoxide ($Mn_3O_4$), dimanganese heptaoxide ($Mn_2O_7$), heptamanganese dodecoxide ($Mn_7O_{12}$), and heptamanganese tridecaoxide ($Mn_7O_{13}$); (2) manganese oxide consisting only of manganese atoms, hydrogen atoms and an oxygen atoms, such as manganese oxyhydroxide (MnOOH) and manganese hydroxide (Mn(OH)$_2$); and (3) manganese oxide consisting of manganese atoms, oxygen atoms, metal atoms and optionally hydrogen atoms, such as various types of salt-type manganese oxides, such as potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide and silver-type manganese oxide. In the above embodiment, the various atoms mentioned, including manganese, oxygen, hydrogen, metal atoms, may be present in their ionic form as desired. The manganese oxide can be generally obtained from one or more of natural minerals, such as hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixibyite, pyrochroite, and manganosite. Accordingly, in some embodiments, the manganese oxide is in the form of a natural mineral. Manganese oxide and their solvates (e.g. hydrates) may also be present in the form of polymorphs. In some embodiments, the manganese oxide is in a crystalline form such as $\alpha$, $\beta$, $\gamma$, $\delta$, $\lambda$, $\varepsilon$, or R form, or in an amorphous form. In some embodiments, during the preparation of polymorphs of manganese oxide, the formation of specific phases is driven by structure-oriented impurity ions. Depending on the type of ions in the solution used to prepare the polymorph of manganese oxide, various cations may be incorporated into the holes between the atoms in the structure of the formed polymorph of manganese oxide, thereby forming manganese oxide in the form of a "salt-type polymorph". In some embodiments, the cation is potassium ion, sodium ion, ammonium ion, calcium ion, magnesium ion, ferric ion, ferrous ion, zinc ion, lanthanum ion, bismuth ion, lithium ion or hydrogen ion; accordingly, the polymorphs of manganese oxide doped with potassium ions, sodium ions, ammonium ions, calcium ions, magnesium ions, ferric ions, ferrous ions, zinc ions, lanthanum ions, bismuth ions, lithium ions and hydrogen ions in these holes are respectively referred to as "potassium type", "sodium type", "ammonium type", "calcium type", "magnesium type", "ferrous type", "ferrous type", "zinc type", "lanthanum type", "bismuth type", "lithium type" or "hydrogen type" polymorphs of manganese oxide.

As used throughout this document, the terms "disease", "disorder", "condition", "(medical) condition" and the like are used interchangeably and refer to any deviation of a subject from a normal state. For example, these terms may refer to physical discomfort or distress in a subject, or to any change in the state of certain organs or tissues which prevents or disrupts the normal performance of their functions.

As used throughout this document, the term "adsorption" refers to the attachment of uric acid (also including urate salts such as monosodium urate) to the surface of a manganese-containing compound.

As used throughout this document, the term "uric acid" refers to 7,9-dihydro-1H-purine-2,6,8(3H)-trione. Uric acid is a heterocyclic compound containing carbon, nitrogen, oxygen and hydrogen, with the molecular formula $C_5H_4N_4O_3$. In humans and most other primates, uric acid is the oxidative end product of purine metabolism. Under the pH of body fluid, uric acid is mainly in the form of its ionic urate (radical). Thus, the term "uric acid" also includes urate salts (e.g. monosodium urate).

As used throughout this document, the term "increased uric acid level" or "higher uric acid level" refers to blood and/or serum uric acid levels greater than normal. For humans, the upper limit of normal levels is usually about 420 $\mu$mol/L for men and about 360 $\mu$mol/L for women. In some embodiments, a subject with "increased uric acid levels" or "higher uric acid levels" has blood and/or serum uric acid levels that are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or 100% higher than 240 $\mu$mol/L, 260 $\mu$mol/L, 280 $\mu$mol/L, 300 $\mu$mol/L, 320 $\mu$mol/L, 340 $\mu$mol/L, 360 $\mu$mol/L, 380 $\mu$mol/L, 400 $\mu$mol/L, or 420 $\mu$mol/L. An undue increase in blood and/or serum uric acid levels can be associated with a variety of diseases or disorders. In some embodiments, the disease or disorder may be at least one disease or disorder selected from the group consist of gout, gout complications, hyperuricemia, higher uric acid levels not reaching levels typically diagnosed as hyperuricemia, cardiovascular disease, diabetes, diabetes-related conditions, insulin resistance, metabolic syndrome, hypothyroidism, hyperparathyroidism, obesity, inflammation, muscle spasms, local swelling, joint pain, malignancies, tumor lysis syndrome, polycythemia vera, cognitive disorder, psoriasis, sarcoidosis, non-alcoholic fatty liver disease, stroke, hemolytic anemia, congenital metabolic errors, poisoning, epididymitis and orchitis, and transplantation of blood, bone marrow, or solid organs.

As used throughout this document, the term hyperuricemia (HUA) refers to abnormally high levels of uric acid in blood and/or serum. Under the pH of body fluid, uric acid is mainly in the form of its ionic urate. The urate content in the human body is determined by the balance between the amount of purine ingested in the diet, the amount of urate synthesized by the human body, and the amount of urate excreted through the urine and the digestive system. Hyperuricemia may result from excessive production of uric acid, decreased uric acid excretion, or both. Clinically, hyperuricemia can be divided into two major categories: primary hyperuricemia and secondary hyperuricemia. Primary hyperuricemia is usually caused by congenital abnormalities of purine metabolism. Secondary hyperuricemia may result from taking certain drugs that inhibit uric acid excretion (e.g. thiazide diuretics, aspirin, pyrazinamide, levodopa, ethambutol) or from certain other medical conditions (e.g. hematological malignancies, chronic kidney disease).

As used throughout this document, the term "higher uric acid levels not reaching levels typically diagnosed as hyperuricemia" refers to blood and/or serum uric acid levels that are greater than the appropriate blood and/or serum uric acid levels in a given population (e.g. those with a particular sex, age and/or disease or disorder), but do not reach the blood and/or serum uric acid levels required for the usual diagnosis of hyperuricemia. In the context of the present disclosure, for a certain given population, an appropriate blood and/or serum uric acid level may be less than or equal to 240 $\mu$mol/L, 260 $\mu$mol/L, 280 $\mu$mol/L, 300 $\mu$mol/L, 320 $\mu$mol/L, 340 $\mu$mol/L, 360 $\mu$mol/L, 380 $\mu$mol/L or 400 $\mu$mol/L.

As used throughout this document, the term "gout" refers to a recurrent inflammatory disease caused by elevated uric acid in the blood and deposition of urate crystals in joint synovium, bursa, cartilage, and other tissues resulting from increased purine biosynthesis metabolism, excessive uric acid production or poor uric acid excretion. In the context of the present disclosure, gout includes, but is not limited to, acute gout, chronic gout, and refractory gout. The term "acute gout" refers to gout present in a subject with at least one symptom of an acute attack of gout (e.g. podagra). The term "chronic gout" refers to gout that is present in subjects with recurrent or persistent acute gout attacks, tophus development, chronic inflammatory arthritis, or gout-associated joint degeneration and includes the stage following recovery from acute gout and the stage between attacks of acute gout (i.e. intercritical gout). The term "refractory gout" refers to gout in which there is no or poor response to one or more oral hypouricals (such as xanthine oxidase inhibitors) that make it difficult for a subject's blood uric acid levels to return to normal.

As used throughout this document, the term "gout complications" refers to diseases or disorders that result from secondary reactions during the development of gout. For example, gout can cause complications such as gouty arthritis due to the deposition of urate (e.g. monosodium urate) crystals in the joints. In the context of the present disclosure, the gout complication of interest may be at least one of gouty arthritis, gouty urinary calculus, and gouty nephropathy.

As used throughout this document, the term "effective amount" refers to an amount sufficient to achieve or at least partially achieve the desired effect. In the context of the present disclosure, a "therapeutically effective amount" of a drug, pharmaceutical composition, or therapeutic agent (e.g. the manganese-containing compound of the present disclosure, including an insoluble manganese-containing compound such as manganese oxide) means any amount that promotes regression of a disease or disorder, as evidenced by a reduction in the severity of the symptoms of the disease or disorder, an increase in the frequency and duration of the asymptomatic period of the disease or disorder, or prevention of damage or disability due to the disease or disorder. A "prophylactically effective amount" of a drug, pharmaceutical composition or therapeutic agent is any amount that inhibits the development or recurrence of a disease or disorder when administered to a subject at risk of the development or recurrence of the disease or disorder. An "effective amount" of a drug, pharmaceutical composition, or therapeutic agent can be assessed using a variety of methods known to those skilled in the art, such as by evaluating human subjects during clinical trials, by using animal model systems to predict efficacy in humans, or by measuring the activity of the drug, pharmaceutical composition, or therapeutic agent in the in vitro assays. In general, the exact amount of a drug, pharmaceutical composition or therapeutic agent required to achieve an effective amount will vary depend upon factors such as species, the age and general condition of a subject, the severity of a disease or disorder, the property of a substance administered and the mode of administration. In some embodiments, an effective amount is achieved by delivering the drug, pharmaceutical composition, or therapeutic agent according to the present disclosure once a day, twice a day, three times a day, once every other day, once every three days, once a week, once every two weeks, once every three weeks, or once every four weeks. In some embodiments, an effective amount is obtained by administering the manganese-containing compound of the present disclosure, including an insoluble manganese-containing compound such as manganese oxide, once, twice, or more times daily at a dosage level sufficient to deliver about 0.001 mg to about 10 g, such as about 0.005 mg to about 5000 mg, about 0.01 mg to about 2500 mg, about 0.05 mg to about 1000 mg, about 0.1 mg to about 750 mg, about 0.2 mg to about 500 mg, about 0.5 mg to about 250 mg, about 0.5 mg to about 100 mg, about 0.5 mg to about 75 mg, or about 1 mg to about 50 mg per kilogram of body weight of a subject per day. In some embodiments, an effective amount is obtained by administering the manganese-containing compound of the present disclosure, including an insoluble manganese-containing compound such as manganese oxide, once, twice or more times daily at a dosage level sufficient to deliver from about 3 mg to about 3000 mg of manganese oxide per day (e.g. from about 100 mg to about 3000 mg of manganese oxide per day, from about 200 mg to about 3000 mg of manganese oxide per day, or from about 500 mg to about 3000 mg of manganese oxide per day). In some embodiments, it is also possible to obtain an effective amount by administering the manganese-containing compound of the present disclosure, including an insoluble manganese-containing compound such as manganese oxide, once, twice or more times daily at a higher dosage level, that is, about 3 g to about 50 g of manganese oxide per day (e.g. about 3 g to about 5 g, 10 g, 15 g, 20 g, 25 g, 30 g, 35 g, 40 g, 45 g of manganese oxide per day), provided that administration of such high dosage levels does not cause unacceptable side effects to the subject. Determination of an effective amount of a drug, pharmaceutical composition, or therapeutic agent appropriate for a particular disease or disorder is within the skill of those skilled in the art.

As used throughout this document, the term "administration" refers to the process of introducing the drug, pharmaceutical composition, or therapeutic agent of the present disclosure into a subject's body by any route of introduction or delivery. Administration may be performed by any method known to those skilled in the art for contacting cells, tissues or organs with the drug, pharmaceutical composition or therapeutic agent. The route of administration may include, but is not limited to, parenteral, topical, or oral administration. In the context of the present disclosure, the term "parenteral administration" refers to a mode of administration other than transgastrointestinal and topical administration, typically by injection. Means of parenteral administration include, but are not limited to, intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion, and in vivo electroporation. In the context of the present disclosure, topical administration routes may include, but are not limited to, epidermal or mucosal administration routes, such as intranasal, vaginal, rectal or sublingual administration. A daily dose of a drug, pharmaceutical composition or therapeutic agent may be divided into one, two or more doses in an appropriate form for administration at one, two or more time points during a certain period of time.

As used throughout this document, the term "prevention" refers to preventing the appearance of a disease or disorder in a subject at risk of developing the disease or the recurrence of a disease or disorder that has disappeared, and "treatment" refers to controlling, reducing or alleviating the pathological progression of a disease or disorder and prolonging the survival of the affected subject. Therefore, in the context of the present disclosure, the term "prevention and/or treatment" covers preventing the onset or further increase in severity of a disease or disorder or one or more symptoms associated therewith; reducing and/or alleviating the severity and/or duration of a disease or disorder or one or more symptoms associated therewith; slowing or reversing the progression of a disease or disorder or one or more symptoms associated therewith; preventing, reducing or reversing any physiological damage caused by a disease or disorder or one or more symptoms associated therewith; any pharmacological effect that is generally beneficial to the subject being treated, etc.

As used throughout this document, the term "subject" refers to a human or non-human mammal in need of diagnosis, amelioration, prevention and/or treatment of a disease or disorder, such as humans (including neonates, infants, adolescents and adult subjects), non-human primates (such as apes (e.g. gibbons), orangutans (e.g. gorillas, chimpanzees), macaques, cynomolgus monkeys, rhesus monkeys), companion animals (e.g. dogs and cats), farm animals (e.g. poultry, such as chickens and ducks, horses, cows, goats, sheep, pigs), and experimental animals (e.g. mice, rats, rabbits, guinea pigs).

As used throughout this document, the term "pharmaceutically acceptable" refers to those substances that are suitable for use in contact with the tissues of subject mammals within the limits of reasonable medical judgment without causing excessive toxicity, irritation, allergic reactions or other problems, while having a reasonable benefit/risk ratio commensurate.

As used throughout this document, the term "pharmaceutically acceptable excipient" refers to an excipient (or carrier) that does not cause excessive irritation to the tissues or organs of a mammalian subject and does not eliminate the pharmacological activity and properties of the administered drug, pharmaceutical composition or therapeutic agent. In the context of the present disclosure, the term "pharmaceutically acceptable excipient" includes any and all solvents, dispersion media, coating agents, surfactants, antioxidants, preservatives (e.g. antibacterial agents, antifungal agents), isotonic agents, absorption retarders, salts, stabilizers, fillers, adhesives, disintegrants, lubricants, glidants, sweeteners, flavoring agents, dyes, etc., and combinations of one or more of them. As non-limiting examples, pharmaceutically acceptable excipients include, but are not limited to: ion exchangers; alumina; aluminum stearate; lecithin; serum proteins, such as human serum albumin; buffer substances, such as phosphates, glycine, sorbic acid, potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts; colloidal silicon dioxide; magnesium trisilicate; polyvinyl pyrrolidone; cellulosic substances; polyethylene glycol; sodium carboxymethyl cellulose; polyacrylates; waxes; polyethylene-polyoxypropylene block polymers; polyethylene glycol; and lanolin. Examples of suitable pharmaceutically acceptable excipients can be found, for example, in Remington's Pharmaceutical Sciences, version 18, Mack Printing Company, 1990, pp. 1289-1329, which is incorporated by reference herein. Except insofar as any conventional excipient is incompatible with the active ingredient, its use in the present disclosure is contemplated.

As used throughout this document, the term "drug" refers to a substance that can produce a beneficial biological effect in humans or other mammals. In the context of the present disclosure, "drug" may refer only to active pharmaceutical ingredients (bulk pharmaceutical chemicals), but also encompasses pharmaceutical compositions (including formulations thereof).

As used throughout this document, the term "pharmaceutical composition" refers to a substance or material having a specific medical use (e.g. for the prevention and/or treatment of increased uric acid levels in the blood and/or digestive tract and/or a disease or disorder associated with increased uric acid levels in the blood and/or digestive tract in a mammalian subject), which is formed by mixing an active pharmaceutical ingredient (e.g. a manganese-containing compound, optionally in combination with an additional therapeutic agent) and a pharmaceutically acceptable excipient in a certain ratio.

As used throughout this document, the terms "formulation" and "pharmaceutical formulation" or "dosage form" are used interchangeably and refer to a pharmaceutical composition in a form of administration that can be directly used by a subject in accordance with certain dosage form requirements to meet the needs of treatment or prevention. As non-limiting examples, the formulation may be one for parenteral administration, topical administration, or oral administration. As the oral formulation, there may be mentioned a solid oral formulation or a liquid oral formulation, wherein the solid oral formulation may be any one of powder, granule, tablet, capsule, pill, and lozenge.

As used throughout this document, the terms "crystalline form" and "crystal" can be used interchangeably to denote that the atoms, ions, or molecules that make up a substance, in accordance with a certain periodicity, form a solid with a regular geometric shape in a spatial arrangement during the crystallization process. Depending on the different periodic spatial arrangements of atoms, ions or molecules, a substance may exist in two or more different crystalline forms, which is also known as polymorphism.

As used throughout this document, the term "amorphous form" or "amorphous" refers to a solid in which the atoms, ions or molecules constituting the substance are not arranged periodically in a certain spatial order (i.e. they are in a disorderly distribution state), as opposed to a crystal.

As used herein throughout, the term "polymorph" refers to various different forms of a solid material that exists in more than one form. In the context of the present disclosure, the term "polymorph" encompasses both "crystalline form" and "amorphous form".

As used throughout this document, the term "solvate" refers to complex compounds formed by the combination of a solute molecule and one or more pharmaceutically acceptable solvent molecules. When the solvent is water, the "solvate" is specifically "hydrate".

As used throughout this document, the term "simulated intestinal fluid (SIF)" refers to a solution that simulates the main inorganic salt components and pH value of intestinal fluid in the human small intestine. Simulated intestinal fluid may be commercially available or prepared by methods well known to those skilled in the art. For example, in some embodiments, the simulated intestinal fluid is prepared as follows: taking 6.8 g of potassium dihydrogen phosphate and dissolving it with 500 mL of water; adjusting the pH value to 6.8 with 0.1 mol/L sodium hydroxide solution; taking 10 g of pancreatic enzyme and dissolving it with an appropriate amount of water; and mixing the two solutions and adding water to make up to 1000 mL, thereby obtaining the simulated intestinal fluid.

As used throughout this document, the term "fasted state simulated intestinal fluid (FaSSIF)" refers to a solution that simulates the intestinal fluid in the small intestine of humans in the hungry state before a meal. The components of FaSSIF and their respective concentrations are as follows: 3 mM taurocholate, 0.75 mM phospholipid, 148 mM sodium, 106 mM chloride and 29 mM phosphate. FaSSIF may be commercially available or prepared by methods well known to those skilled in the art. For example, in some embodiments, FaSSIF is prepared as follows: (1) preparation of buffer: mixing 37.48 g of FaSSIF buffer concentrate with 865.0 g of purified water; (2) adding FaSSIF powder to the buffer: adding 2.016 g of FaSSIF powder to the buffer; (3) stirring the resultant mixture until all solids dissolve; and (4) placing (balancing) the resulting solution for at least 2 h, thereby obtaining FaSSIF. The FaSSIF buffer concentrate and FaSSIF powder are commercially available, for example, from Biorelevant.com Ltd.

As used throughout this document, the term "fed state simulated intestinal fluid (FeSSIF)" refers to a solution that simulates the intestinal fluid in the small intestine of humans in the state of satiety after meals. The components of FeSSIF and their respective concentrations are as follows: 15 mM taurocholate, 3.75 mM phospholipid, 319 mM sodium, 203 mM chloride and 144 mM acetic acid FeSSIF may be commercially available or prepared by methods well known to those skilled in the art. For example, in some embodiments, FeSSIF is prepared as follows: (1) preparation of buffer: mixing 73.27 g of FeSSIF buffer concentrate with 827.5 g of purified water; (2) adding FeSSIF powder to the buffer: adding 10.08 g of FeSSIF powder to the buffer; (3) stirring the resulting mixture until all solids dissolve, thereby obtaining FeSSIF. The FeSSIF buffer concentrate and FeSSIF powder are commercially available, for example, from Biorelevant.com Ltd.

Detailed Description of Embodiments

To the best of the inventors' knowledge, no drug based on the mechanism of uric acid clearance in the digestive tract has been approved to reduce uric acid levels in patients with hyperuricemia. Although known adsorbents such as activated charcoal have large specific surface areas and have been shown to adsorb various substances, such adsorption often does not have good selectivity. Thus, in the complex physiological environment of the gastrointestinal tract, the adsorption effect of activated charcoal on uric acid will be interfered by a large number of other components in the intestinal tract, thereby failing to achieve efficient adsorption of uric acid. Therefore, how to achieve efficient adsorption of uric acid in a complex gastrointestinal environment is an important challenge for the development of drugs based on the mechanism of uric acid clearance in the digestive tract.

The present disclosure describes use of a manganese-containing compound in reducing uric acid levels in a mammalian subject, particularly to use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject, a pharmaceutical composition and a kit for such use, and a method for preventing and/or treating the related diseases or disorders.

The manganese-containing compounds of the present disclosure have particular utility and/or benefit in the prevention and/or treatment of increased uric acid levels in the blood and/or digestive tract or a disease or disorder associated with increased uric acid levels in the blood and/or digestive tract in mammalian subjects, the adsorption of uric acid in the digestive tract of mammals, and/or the maintenance of (normal or appropriate) uric acid levels in the blood of mammalian subjects. In some embodiments of the present disclosure, the manganese-containing compounds of the present disclosure exhibit excellent adsorption and/or clearance of uric acid (including its salts, such as monosodium urate) from the digestive tract in a mammalian subject. In some embodiments, the manganese-containing compounds of the present disclosure are superior to zeolite molecular sieves, Prussian blue, activated charcoal, montmorillonite, ion exchange resins (such as sevelamer hydrochloride), metallic organic framework materials, and the like known to have adsorption/or clearance properties on uric acid (including its salts, such as monosodium urate) in the digestive tract of mammalian subjects. In some embodiments, the adsorption/clearance of uric acid (including its salts, such as monosodium urate) from the digestive tract of a mammalian subject by the manganese-containing compounds of the present disclosure is unaffected, essentially unaffected or less affected by various substances present in the digestive tract of the mammal, including, but not limited to, digestive juices such as gastric juice, small intestinal juice and chyme. In some embodiments, the manganese-containing compound of the present disclosure has the ability to continuously adsorb uric acid (including its salts, such as monosodium urate) at a low concentration of uric acid (including its salts, such as monosodium urate), and can still maintain the ability to effectively adsorb uric acid (including its salts, such as monosodium urate) after multiple times of adsorption. In some embodiments, the manganese-containing compounds of the present disclosure can be administered at very high oral safe doses (e.g. about 50 g/day or even higher) without causing unacceptable side effects, thus having a desirable safety profile. In some embodiments, the manganese-containing compounds of the present disclosure have extremely low systemic absorption and in vivo accumulation effect after administration to mammals, and have no significant influence on blood biochemical indexes of mammal subjects.

In some embodiments, the manganese-containing compound is an insoluble manganese-containing compound. The insoluble manganese-containing compounds of the present disclosure are characterized by their insolubility or extremely low solubility in water and/or gastrointestinal fluids (including, but not limited to, gastric juice, small intestinal fluid, large intestinal fluid, pancreatic juice, and/or bile) under physiological conditions, and/or by their physiological inertness (i.e. they are substantially unable to enter the blood circulation through absorption in the gastrointestinal tract (i.e. the digestive tract) and/or they substantially do not chemically react with surrounding substances or tissues in the body after administration).

Thus, in a first aspect, the present disclosure provides use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject.

In some embodiments, the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of water and saturated as measured at about 37°C and about 1 atmospheric pressure.

In some embodiments, the insoluble manganese-containing compound is a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of simulated intestinal fluid (SIF) and saturated as measured at about 37°C and about 1 atmospheric pressure.

In some embodiments, the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg,

1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fasted state simulated intestinal fluid (FaSSIF) and saturated as measured at about 37°C and about 1 atmospheric pressure.

In some embodiments, the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fed state simulated intestinal fluid (FeSSIF) and saturated as measured at about 37°C and about 1 atmospheric pressure.

In some embodiments, the insoluble manganese-containing compound is a manganese-containing compound that enters the blood circulation by absorption in the gastrointestinal tract in an amount of less than about 20%, 15%, 10%, 5%, 4%, 3%, 2%, 1%, 0.5%, 0.1%, 0.05%, 0.02%, or 0.01% of the dose administered.

In some embodiments, the insoluble manganese-containing compound is a manganese-containing compound that does not chemically interact with a surrounding substance or tissue in the body after administration, or only less than about 20%, 15%, 10%, 5%, 4%, 3%, 2%, 1%, 0.5%, 0.1%, 0.05%, 0.02%, or 0.01% of the dose administered of the manganese-containing compound chemically interacts with a surrounding substance or tissue in the body.

In some embodiments, the particle size of the insoluble manganese-containing compound is ≥1 μm, e.g. 1 μm to 1 mm.

In some embodiments, the insoluble manganese-containing compound is selected from one or more of manganese oxide, manganese sulfide, manganese carbonate, manganese silicate, manganese borate, manganese ferricyanate, manganese oxalate, manganese selenite, manganese iodate, manganese tungstate and manganese phosphate, and solvates (e.g. hydrates) thereof.

In some embodiments, the insoluble manganese-containing compound is manganese oxide or a solvate (e.g. a hydrate) thereof. In some embodiments, the manganese oxide is selected from one or more of: (1) manganese oxide consisting only of manganese atoms and oxygen atoms; (2) manganese oxide consisting only of manganese atoms, hydrogen atoms and oxygen atoms; and (3) manganese oxide consisting only of manganese atoms, oxygen atoms, metal atoms and optionally hydrogen atoms. In some embodiments, the manganese oxide is manganese oxide consisting only of manganese atoms and oxygen atoms. In some embodiments, the manganese oxide is manganese oxide consisting only of manganese atoms, hydrogen atoms and oxygen atoms. In some embodiments, the manganese oxide is manganese oxide consisting of manganese atoms, oxygen atoms, metal atoms and optionally hydrogen atoms

In some embodiments, the manganese oxide is manganese oxide in the form of a natural mineral. In some embodiments, the manganese oxide in the form of a natural mineral is selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixbyite, pyrochroite, and manganosite. In some embodiments, the manganese oxide in the form of a natural mineral is hollandite. In some embodiments, the manganese oxide in the form of a natural mineral is cryptomelane. In some embodiments, the manganese oxide in the form of a natural mineral is pyrolusite. In some embodiments, the manganese oxide in the form of a natural mineral is nsutit. In some embodiments, the manganese oxide in the form of a natural mineral is birnessite. In some embodiments, the manganese oxide in the form of a natural mineral is buserite. In some embodiments, the manganese oxide in the form of a natural mineral is vernadite. In some embodiments, the manganese oxide in the form of a natural mineral is ramsdellit. In some embodiments, the manganese oxide in the form of a natural mineral is romanechite. In some embodiments, the manganese oxide in the form of a natural mineral is todorokite. In some embodiments, the manganese oxide in the form of a natural mineral is manganite. In some embodiments, the manganese oxide in the form of a natural mineral is groutite. In some embodiments, the manganese oxide in the form of a natural mineral is feitknechtitet. In some embodiments, the manganese oxide in the form of a natural mineral is hydrohausmannite. In some embodiments, the manganese oxide in the form of a natural mineral is lithiophorite. In some embodiments, the manganese oxide in the form of a natural mineral is chalcophanite. In some embodiments, the manganese oxide in the form of a natural mineral is hausmannite. In some embodiments, the manganese oxide in the form of a natural mineral is bixbyite. In some embodiments, the manganese oxide in the form of a natural mineral is pyrochroite. In some embodiments, the manganese oxide in the form of a natural mineral is manganosite.

In some embodiments, the manganese oxide is manganese oxide consisting only of manganese atoms and oxygen atoms. In some embodiments, the manganese oxide consisting only of manganese atoms and oxygen atoms is selected from one or more of manganese monoxide (MnO), manganese dioxide ($MnO_2$), monomanganese trioxide ($MnO_3$), diamanganese trioxide ($Mn_2O_3$), pentamanganese octoxide ($Mn_5O_8$), trimanganese tetraoxide ($Mn_3O_4$), di-manganese heptaoxide ($Mn_2O_7$), heptamanganese dodecoxide ($Mn_7O_{12}$), and heptamanganese tridecaoxide ($Mn_7O_{13}$). In some embodiments, the manganese oxide consisting only of manganese atoms and oxygen atoms is manganese monoxide (MnO). In some embodiments, the manganese oxide consisting only of manganese atoms and oxygen atoms is manganese dioxide ($MnO_2$). In some embodiments, the manganese oxide consisting only of manganese atoms and oxygen atoms is monomanganese trioxide ($MnO_3$). In some embodiments, the manganese oxide consisting only of manganese atoms and oxygen atoms is dimanganese trioxide ($Mn_2O_3$). In some embodiments, the manganese oxide consisting only of manganese atoms and oxygen atoms is pentamanganese octoxide ($Mn_5O_8$). In some embodiments, the manganese oxide consisting only of manganese atoms and oxygen atoms is trimanganese tetraoxide ($Mn_3O_4$). In some embodiments, the manganese oxide consisting only of manganese atoms and oxygen atoms is dimanganese heptaoxide ($Mn_2O_7$). In some embodiments, the manganese oxide consisting only of manganese atoms and oxygen atoms is heptamanganese dodecoxide ($Mn_7O_{12}$). In some embodiments, the manganese oxide consisting only of manganese atoms and oxygen atoms is heptamanganese tridecaoxide ($Mn_7O_{13}$).

In some embodiments, the manganese oxide is manganese oxide consisting only of manganese atoms, hydrogen atoms and oxygen atoms. In some embodiments, the manganese oxide composed of only manganese atoms, hydrogen atoms, and oxygen atoms is selected from one or both of manganese oxyhydroxide (MnOOH) and manganese hydroxide ($Mn(OH)_2$). In some embodiments, the manganese oxide consisting only of manganese atoms, hydrogen atoms and oxygen atoms is manganese oxyhydroxide. In some embodiments, the manganese oxyhydroxide is manganese oxyhydroxide in the form of a polymorph. In some embodiments, the manganese oxyhydroxide in the form of a polymorph is selected from one or more of $\alpha$-form crystalline manganese oxyhydroxide, $\beta$-form crystalline manganese oxyhydroxide, and $\gamma$-form crystalline manganese oxyhydroxide. In some embodiments, the manganese oxyhydroxide in the form of a polymorph is $\alpha$-form crystalline manganese oxyhydroxide. In some embodiments, the $\alpha$-form crystalline manganese oxyhydroxide is $\alpha$-form crystalline manganese oxyhydroxide in the form of groutite. In some embodiments, the manganese oxyhydroxide in the form of a polymorph is $\beta$-form crystalline manganese oxyhydroxide. In some embodiments, the $\beta$-form crystalline manganese oxyhydroxide is $\beta$-form crystalline manganese oxyhydroxide in the form of one or both of feitknechtite and hydrohausmannite. In some embodiments, the manganese oxyhydroxide in the form of a polymorph is $\gamma$-form crystalline manganese oxyhydroxide. In some embodiments, the $\gamma$-form crystalline manganese oxyhydroxide is $\gamma$-form crystalline manganese oxyhydroxide in the form of manganite. In some embodiments, the manganese oxide consisting only of manganese atoms, hydrogen atoms and oxygen atoms is manganese hydroxide. In some embodiments, the manganese hydroxide is manganese hydroxide in the form of a polymorph.

In some embodiments, the manganese oxide is manganese oxide consisting of manganese atoms, oxygen atoms, metal atoms and optionally hydrogen atoms In some embodiments, the manganese oxide consisting of manganese atoms, oxygen atoms, metal atoms and optionally hydrogen atoms is salt-form manganese oxide. In some embodiments, the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide. In some embodiments, the salt-type manganese oxide is potassium-type manganese oxide. In some embodiments, the salt-type manganese oxide is hydrogen-type manganese oxide. In some embodiments, the salt-type manganese oxide is ammonium-type manganese oxide. In some embodiments, the salt-type manganese oxide is sodium-type manganese oxide. In some embodiments, the salt-type manganese oxide is calcium-type manganese oxide. In some embodiments, the salt-type manganese oxide is magnesium-type manganese oxide. In some embodiments, the salt-type manganese oxide is iron-type manganese oxide. In some embodiments, the salt-type manganese oxide is ferrous-type manganese oxide. In some embodiments, the salt-type manganese oxide is zinc-type manganese oxide. In some embodiments, the salt-type manganese oxide is lanthanum-type manganese oxide. In some embodiments, the salt-type manganese oxide is bismuth-type manganese oxide. In some embodiments, the salt-type manganese oxide is lithium-type manganese oxide. In some embodiments, the salt-type manganese oxide is silver-type manganese oxide.

In some embodiments, the manganese oxide is manganese oxide in the form of a polymorph, such as manganese oxide in the form of a salt-type polymorph.

In some embodiments, the manganese oxide in the form of a polymorph is selected from one or more of $\alpha$-form crystalline manganese oxide (e.g. salt-type $\alpha$-form crystalline manganese oxide), $\beta$-form crystalline manganese oxide (e.g. salt-type $\beta$-form crystalline manganese oxide), $\gamma$-form crystalline manganese oxide (e.g. salt-type $\gamma$-form crystalline manganese oxide), $\delta$-form crystalline manganese oxide (e.g. salt-type $\delta$-form crystalline manganese oxide), $\lambda$-form crystalline manganese oxide (e.g. salt-type $\lambda$-form crystalline manganese oxide), $\varepsilon$-form crystalline manganese oxide (e.g. salt-type $\varepsilon$-form crystalline manganese oxide), R-form crystalline manganese oxide (e.g. salt-type R-form crystalline manganese oxide), amorphous manganese oxide (e.g. salt-type amorphous manganese oxide), $\alpha$-form crystalline manganese oxyhydroxide (e.g. salt-type $\alpha$-form crystalline manganese oxyhydroxide), $\beta$-form crystalline manganese oxyhydroxide (e.g. salt-type $\beta$-form crystalline manganese oxyhydroxide), and $\gamma$-form crystalline manganese oxyhydroxide (e.g. salt-type $\gamma$-form crystalline manganese oxyhydroxide).

In some embodiments, the manganese oxide in the form of a polymorph is $\alpha$-form crystalline manganese oxide. In some embodiments, the $\alpha$-form crystalline manganese oxide is $\alpha$-form crystalline manganese oxide in the form of hollandite or cryptomelane.

In some embodiments, the manganese oxide in the form of a polymorph is $\beta$-form crystalline manganese oxide. In some embodiments, the β-form crystalline manganese oxide is β-form crystalline manganese oxide in the form of pyrolusite.

In some embodiments, the manganese oxide in the form of a polymorph is γ-form crystalline manganese oxide. In some embodiments, the γ-form crystalline manganese oxide is γ-form crystalline manganese oxide in the form of nsutit.

In some embodiments, the manganese oxide in the form of a polymorph is δ-form crystalline manganese oxide. In some embodiments, the δ-form crystalline manganese oxide is δ-form crystalline manganese oxide in the form of one or more of birnessite, buserite, and vernadite.

In some embodiments, the manganese oxide in the form of a polymorph is λ-form crystalline manganese oxide.

In some embodiments, the manganese oxide in the form of a polymorph is ε-form crystalline manganese oxide.

In some embodiments, the manganese oxide in the form of a polymorph is R-form crystalline manganese oxide. In some embodiments, the R-form crystalline manganese oxide is R-form crystalline manganese oxide in the form of ramsdellite.

In some embodiments, the manganese oxide in the form of a polymorph is amorphous manganese oxide.

In some embodiments, the manganese oxide in the form of a polymorph is α-form crystalline manganese oxyhydroxide. In some embodiments, the α-form crystalline manganese oxyhydroxide is α-form crystalline manganese oxyhydroxide in the form of groutite.

In some embodiments, the manganese oxide in the form of a polymorph is β-form crystalline manganese oxyhydroxide. In some embodiments, the β-form crystalline manganese oxyhydroxide is β-form crystalline manganese oxyhydroxide in the form of one or both of feitknechtite and hydrohausmannite.

In some embodiments, the manganese oxide in the form of a polymorph is γ-form crystalline manganese oxyhydroxide. In some embodiments, the γ-form crystalline manganese oxyhydroxide is γ-form crystalline manganese oxyhydroxide in the form of manganite.

In some embodiments, the manganese oxide is manganese oxide in the form of a salt-type polymorph.

In some embodiments, the manganese oxide in the form of a salt-type polymorph is selected from one or more of a salt-type α-form crystalline manganese oxide, a salt-type β-form crystalline manganese oxide, a salt-type γ-form crystalline manganese oxide, a salt-type δ-form crystalline manganese oxide, a salt-type λ-form crystalline manganese oxide, a salt-type ε-form crystalline manganese oxide, a salt-type R-form crystalline manganese oxide, and a salt-type amorphous manganese oxide.

In some embodiments, the manganese oxide in the form of a salt-type polymorph is salt-type α-form crystalline manganese oxide. In some embodiments, the salt-type α-form crystalline manganese oxide is selected from one or both of potassium-type α-form crystalline manganese oxide and hydrogen-type α-form crystalline manganese oxide. In some embodiments, the salt-type α-form crystalline manganese oxide is potassium-type α-form crystalline manganese oxide. In some embodiments, the potassium-type α-form crystalline manganese oxide is characterized by its powder X-ray diffraction pattern showing characteristic X-ray diffraction peaks at the following 2θ angles: $12.8 \pm 10.2°$, $18.1 \pm 0.2°$, $25.5 \pm 0.2°$, $28.7 \pm 0.2°$, $39.0 \pm 0.2°$, $42.0 \pm 0.2°$, $49.9 \pm 0.2°$, $56.4 \pm 0.2°$, $60.2 \pm 0.2°$, $65.1 \pm 0.2°$, $70.0 \pm 0.2°$ and $72.5 \pm 0.2°$. In some embodiments, the potassium-type α-form crystalline manganese oxide is characterized by its powder X-ray diffraction pattern showing characteristic X-ray diffraction peaks at the following 2θ angles: $12.7 \pm 0.2°$, $18.0 \pm 0.2°$, $25.4 \pm 0.2°$, $28.7 \pm 0.2°$, $39.1 \pm 0.2°$, $42.0 \pm 0.2°$, $50.0 \pm 0.2°$, $56.5 \pm 0.2°$, $60.1 \pm 0.2°$, $65.2 \pm 0.2°$, $70.1 \pm 0.2°$, and $72.5 \pm 0.2°$. In some embodiments, the potassium-type α-form crystalline manganese oxide is characterized by its powder X-ray diffraction pattern showing characteristic X-ray diffraction peaks at the following 2θ angles: $12.7 \pm 0.2°$, $18.0 \pm 0.2°$, $25.4 \pm 0.2°$, $28.7 + 0.2°$, $39.2 \pm 0.2°$, $42.0 \pm 0.2°$, $50.0 \pm 0.2°$, $56.4 \pm 0.2°$, $60.1 \pm 0.2°$, $65.2 \pm 0.2°$, $70.1 \pm 0.2°$ and $72.5 \pm 0.2°$. In some embodiments, the salt-type α-form crystalline manganese oxide is hydrogen-type α-form crystalline manganese oxide. In some embodiments, the hydrogen-type α-form crystalline manganese oxide is characterized by its powder X-ray diffraction pattern showing characteristic X-ray diffraction peaks at the following 2θ angles: $12.8 \pm 0.2°$, $18.1 \pm 0.2°$, $25.7 + 0.2°$, $28.8 \pm 0.2°$, $36.7 \pm 0.2°$, $39.0 \pm 0.2°$, $41.9 \pm 0.2°$, $49.8 \pm 0.2°$, $56.4 \pm 0.2°$, $60.1 \pm 0.2°$, $65.2 \pm 0.2°$, $70.1 \pm 0.2°$ and $72.7 \pm 0.2°$. In some embodiments, the hydrogen-type α-form crystalline manganese oxide is characterized by its powder X-ray diffraction pattern showing characteristic X-ray diffraction peaks at the following 2θ angles: $12.7 \pm 0.2°$, $18.0 \pm 0.2°$, $25.5 \pm 0.2°$, $28.7 \pm 0.2°$, $39.1 \pm 0.2°$, $42.0 \pm 0.2°$, $50.0 \pm 0.2°$, $56.5 \pm 0.2°$, $60.1 \pm 0.2°$, $65.2 \pm 0.2°$, $70.1 \pm 0.2°$ and $72.5 \pm 0.2°$. In some embodiments, the hydrogen-type α-form crystalline manganese oxide is characterized by its powder X-ray diffraction pattern showing characteristic X-ray diffraction peaks at the following 2θ angles: $12.7 \pm 0.2°$, $18.1 \pm 0.2°$, $25.4 \pm 0.2°$, $28.8 \pm 0.2°$, $39.1 \pm 0.2°$, $42.0 \pm 0.2°$, $50.0 \pm 0.2°$, $56.5 \pm 0.2°$, $60.0 \pm 0.2°$, $65.2 \pm 0.2°$, $70.1 \pm 0.2°$ and $72.5 \pm 0.2°$.

In some embodiments, the manganese oxide in the form of a salt-type polymorph is salt-type β-form crystalline manganese oxide. In some embodiments, the salt-type β-form crystalline manganese oxide is selected from one or both of potassium-type β-form crystalline manganese oxide and hydrogen-type β-form crystalline manganese oxide. In some embodiments, the salt-type β-form crystalline manganese oxide is potassium-type β-form crystalline manganese oxide. In some embodiments, the potassium-type β-form crystalline manganese oxide is characterized by its powder X-ray diffraction pattern showing characteristic X-ray diffraction peaks at the following 2θ angles: $28.7 \pm 0.2°$, $37.4 \pm 0.2°$, $41.1 \pm 0.2°$, $42.9 \pm 0.2°$, $46.2 \pm 0.2°$, $56.8 \pm 0.2°$, $59.5 \pm 0.2°$, $65.0 \pm 0.2°$, $67.5 \pm 0.2°$ and $72.6 \pm 0.2°$. In some embodiments, the salt-type β-form crystalline manganese oxide is hydrogen-type β-form crystalline manganese oxide. In some embodiments, the hydrogen-type β-form crystalline manganese oxide is characterized by its powder X-ray diffraction pattern showing characteristic X-ray diffraction peaks at the following 2θ angles: 28.7+0.2°, 37.4±0.2°, 41.1±0.2°, 42.9 ±0.2°, 46.2±0.2°, 56.8±0.2°, 59.5±0.2°, 65.0±0.2°, 67.5±0.2°, and 72.6±0.2°. In some embodiments, the hydrogen-type β-form crystalline manganese oxide is characterized by its powder X-ray diffraction pattern showing characteristic X-ray diffraction peaks at the following 2θ angles: 28.7±0.2°, 37.4±0.2°, 41.1±0.2°, 42.9±0.2°, 46.2±0.2°, 56.8±0.2°, 59.5 ±0.2°, 65.0±0.2°, 67.5±0.2° and 72.6±0.2°.

In some embodiments, the manganese oxide in the form of a salt-type polymorph is salt-type γ-form crystalline manganese oxide. In some embodiments, the salt-type γ-form crystalline manganese oxide is selected from one or both of ammonium-type γ-form crystalline manganese oxide and hydrogen-type γ-form crystalline manganese oxide. In some embodiments, the salt-type γ-form crystalline manganese oxide is ammonium-type γ-form crystalline manganese oxide. In some embodiments, the ammonium-type γ-form crystalline manganese oxide is characterized by its powder X-ray diffraction pattern showing characteristic X-ray diffraction peaks at the following 2θ angles: 23.0±0.2°, 26.7±0.2°, 34.0 ±0.2°, 37.1+0.2°, 42.5±0.2°, 50.0±0.2°, 56.3±0.2°, 65.5±0.2° and 69.4±0.2°. In some embodiments, the salt-type γ-form crystalline manganese oxide is hydrogen-type γ-form crystalline manganese oxide. In some embodiments, the hydrogen-type γ-form crystalline manganese oxide is characterized by its powder X-ray diffraction pattern showing characteristic X-ray diffraction peaks at the following 2θ angles: 23.0±0.2°, 26.7±0.2°, 34.0±0.2°, 37.3±0.2°, 42.5±0.2°, 56.3±0.2°, and 65.5±0.2°. In some embodiments, the hydrogen-type γ-form crystalline manganese oxide is characterized by its powder X-ray diffraction pattern showing characteristic X-ray diffraction peaks at the following 2θ angles: 23.0 ±0.2°, 26.7±0.2°, 34.0±0.2°, 37.1±0.2°, 42.5±0.2°, 50.0±0.2°, 56.4±0.2°, 65.5±0.2° and 69.6±0.2°.

In some embodiments, the manganese oxide in the form of a salt-type polymorph is salt-type δ-form crystalline manganese oxide. In some embodiments, the salt-type δ-form crystalline manganese oxide is selected from one or more (e.g. 2, 3, 4, 5, 6, 7, 8, 9, 10, 11, 12, or 13) of potassium-type δ-form crystalline manganese oxide, hydrogen-type δ-form crystalline manganese oxide, sodium-type δ-form crystalline manganese oxide, ammonium-type δ-form crystalline manganese oxide, calcium-type δ-form crystalline manganese oxide, magnesium-type δ-form crystalline manganese oxide, iron-type δ-form crystalline manganese oxide, ferrous-type δ-form crystalline manganese oxide, zinc-type δ-form crystalline manganese oxide, lanthanum-type δ-form crystalline manganese oxide, bismuth-type δ-form crystalline manganese oxide, lithium-type δ-form crystalline manganese oxide, and silver-type δ-form crystalline manganese oxide. In some embodiments, the salt-type δ-form crystalline manganese oxide is potassium-type δ-form crystalline manganese oxide. In some embodiments, the potassium-type δ-form crystalline manganese oxide is characterized by its powder X-ray diffraction pattern showing characteristic X-ray diffraction peaks at the following 2θ angles: 12.2±0.2°, 24.9±0.2°, 37.0 ±0.2°, 56.4±0.2° and 65.8±0.2°. In some embodiments, the potassium-type δ-form crystalline manganese oxide is characterized by its powder X-ray diffraction pattern showing characteristic X-ray diffraction peaks at the following 2θ angles: 12.4±0.2°, 25.0±0.2°, 36.9±0.2°, 56.0±0.2° and 66.1±0.2°. In some embodiments, the potassium-type δ-form crystalline manganese oxide is characterized by its powder X-ray diffraction pattern showing characteristic X-ray diffraction peaks at the following 2θ angles: 12.7±0.2°, 37.2±0.2°, 56.9±0.2° and 66.4±0.2°. In some embodiments, the potassium-type δ-form crystalline manganese oxide is characterized by its powder X-ray diffraction pattern showing characteristic X-ray diffraction peaks at the following 2θ angles: 12.5±0.2°, 24.5±0.2°, 37.1±0.2° and 66.0±0.2°. In some embodiments, the salt-type δ-form crystalline manganese oxide is hydrogen-type δ-form crystalline manganese oxide. In some embodiments, the hydrogen-type δ-form crystalline manganese oxide is characterized by its powder X-ray diffraction pattern showing characteristic X-ray diffraction peaks at the following 2θ angles: 12.5±0.2°, 25.1±0.2°, 36.8±0.2°, 56.9±0.2° and 66.0±0.2°. In some embodiments, the hydrogen-type δ-form crystalline manganese oxide is characterized by its powder X-ray diffraction pattern showing characteristic X-ray diffraction peaks at the following 2θ angles: 12.6±0.2°, 24.8±0.2°, 36.5±0.2°, 55.7±0.2° and 66.3±0.2°. In some embodiments, the hydrogen-type δ-form crystalline manganese oxide is characterized by its powder X-ray diffraction pattern showing characteristic X-ray diffraction peaks at the following 2θ angles: 12.6±0.2°, 37.5±0.2°, 57.0±0.2° and 66.2±0.2°. In some embodiments, the hydrogen-type δ-form crystalline manganese oxide is characterized by its powder X-ray diffraction pattern showing characteristic X-ray diffraction peaks at the following 2θ angles: 12.8±0.2°, 37.3±0.2°, 56.7±0.2° and 66.8±0.2°. In some embodiments, the salt-type δ-form crystalline manganese oxide is sodium-type δ-form crystalline manganese oxide. In some embodiments, the sodium-type δ-form crystalline manganese oxide is characterized by its powder X-ray diffraction pattern showing characteristic X-ray diffraction peaks at the following 2θ angles: 12.7±0.2°, 37.1±0.2°, 57.2±0.2° and 66.3±0.2°. In some embodiments, the salt-type δ-form crystalline manganese oxide is ammonium-type δ-form crystalline manganese oxide. In some embodiments, the ammonium-type δ-form crystalline manganese oxide is characterized by its powder X-ray diffraction pattern showing characteristic X-ray diffraction peaks at the following 2θ angles: 12.6±0.2°, 24.9 ±0.2°, 36.9±0.2°, 56.9±0.2° and 66.1±0.2°. In some embodiments, the salt-type δ-form crystalline manganese oxide is calcium-type δ-form crystalline manganese oxide. In some embodiments, the calcium-type δ-form crystalline manganese oxide is characterized by its powder X-ray diffraction pattern showing characteristic X-ray diffraction peaks at the following 2θ angles: 8.9±0.2°, 12.6±0.2°, 18.5±0.2° and 37.0±0.2°. In some embodiments, the salt-type δ-form crystalline manganese oxide is magnesium-type δ-form crystalline manganese oxide. In some embodiments, the magnesium-type δ-form crystalline manganese oxide is characterized by its powder X-ray diffraction pattern showing characteristic X-ray diffraction peaks at the following 2θ angles: 9.0±0.2°, 18.5±0.2° and 28.1±0.2°. In some embodiments, the salt-type δ-form crystalline manganese oxide is iron-type δ-form crystalline manganese oxide. In some embodiments, the iron-type δ-form crystalline manganese oxide is characterized by its powder X-ray diffraction pattern showing characteristic X-ray diffraction peaks at the following 2θ angles: 12.3±0.2°, 25.0±0.2°, 36.8±0.2°, 56.7±0.2° and 66.2±0.2°. In some embodiments, the salt-type δ-form crystalline manganese oxide is ferrous-type δ-form crystalline manganese oxide. In some embodiments, the ferrous-type δ-form crystalline manganese oxide is characterized by its powder X-ray diffraction pattern showing characteristic X-ray diffraction peaks at the following 2θ angles: 12.6±0.2°, 24.9±0.2°, 36.84+0.2°, 56.5 ±0.2° and 66.7±0.2°. In some embodiments, the salt-type δ-form crystalline manganese oxide is zinc-type δ-form crystalline manganese oxide. In some embodiments, the zinc-type δ-form crystalline manganese oxide is characterized by its powder X-ray diffraction pattern showing characteristic X-ray diffraction peaks at the following 2θ angles: 12.5±0.2°, 25.2±0.2°, 36.9±0.2°, 56.7±0.2° and 66.9±0.2°. In some embodiments, the salt-type δ-form crystalline manganese oxide is lanthanum-type δ-form crystalline manganese oxide. In some embodiments, the lanthanum-type δ-form crystalline manganese oxide is characterized by its powder X-ray diffraction pattern showing characteristic X-ray diffraction peaks at the following 2θ angles: 9.2±0.2°, 12.5±0.2°, 18.3%0.2°, 25.0±0.2° and 37.0±0.2°. In some embodiments, the salt-type δ-form crystalline manganese oxide is bismuth-type δ-form crystalline manganese oxide. In some embodiments, the bismuth-type δ-form crystalline manganese oxide is characterized by its powder X-ray diffraction pattern showing characteristic X-ray diffraction peaks at the following 2θ angles: 12.6±0.2°, 25.2±0.2°, 37.1±0.2°, 56.7±0.2° and 67.0 ±0.2°. In some embodiments, the salt-type δ-form crystalline manganese oxide is lithium-type δ-form crystalline manganese oxide. In some embodiments, the lithium-type δ-form crystalline manganese oxide is characterized by its powder X-ray diffraction pattern showing characteristic X-ray diffraction peaks at the following 2θ angles: 12.6±0.2°, 25.0 ±0.2°, 36.9±0.2°, 56.5±0.2° and 66.8±0.2°. In some embodiments, the salt-type δ-form crystalline manganese oxide is silver-type δ-form crystalline manganese oxide. In some embodiments, the silver-type δ-form crystalline manganese oxide is characterized by its powder X-ray diffraction pattern showing characteristic X-ray diffraction peaks at the following 2θ angles: 12.5±0.2°, 24.9±0.2°, 36.8±0.2°, 56.7±0.2° and 66.9±0.2°.

In some embodiments, the manganese oxide in the form of a salt-type polymorph is salt-type λ-form crystalline manganese oxide. In some embodiments, the salt-type λ-form crystalline manganese oxide is selected from one or both of lithium-type λ-form crystalline manganese oxide and hydrogen-type λ-form crystalline manganese oxide. In some embodiments, the salt-type λ-form crystalline manganese oxide is lithium-type λ-form crystalline manganese oxide. In some embodiments, the salt-type λ-form crystalline manganese oxide is hydrogen-type λ-form crystalline manganese oxide. In some embodiments, the hydrogen-type λ-form crystalline manganese oxide is characterized by its powder X-ray diffraction pattern showing characteristic X-ray diffraction peaks at the following 2θ angles: 19.0±0.2°, 37.1±0.2°, 45.1 ±0.2°, 49.5±0.2°, 59.7±0.2°, 65.6±0.2° and 69.1±0.2°.

In some embodiments, the manganese oxide in the form of a salt-type polymorph is salt-type ε-form crystalline manganese oxide. In some embodiments, the salt-type ε-form crystalline manganese oxide is selected from one or both of potassium-type ε-form crystalline manganese oxide and hydrogen-type ε-form crystalline manganese oxide. In some embodiments, the salt-type ε-form crystalline manganese oxide is potassium-type ε-form crystalline manganese oxide. In some embodiments, the potassium-type ε-form crystalline manganese oxide is characterized by its powder X-ray diffraction pattern showing characteristic X-ray diffraction peaks at the following 2θ angles: 21.0±0.2°, 37.1±0.2°, 42.5±0.2°, 56.0±0.2° and 66.8±0.2°. In some embodiments, the salt-type ε-form crystalline manganese oxide is hydrogen-type ε-form crystalline manganese oxide. In some embodiments, the hydrogen-type ε-form crystalline manganese oxide is characterized by its powder X-ray diffraction pattern showing characteristic X-ray diffraction peaks at the following 2θ angles: 21.0±0.2°, 37.1+0.2°, 42.5±0.2°, 56.0±0.2° and 66.8±0.2°.

In some embodiments, the manganese oxide in the form of a salt-type polymorph is salt-type R-form crystalline manganese oxide. In some embodiments, the salt-type R-form crystalline manganese oxide is hydrogen-type R-form crystalline manganese oxide. In some embodiments, the hydrogen-type R-form crystalline manganese oxide is characterized by its powder X-ray diffraction pattern showing characteristic X-ray diffraction peaks at the following 2θ angles: 22.0±0.2°, 35.0±0.2°, 36.9±0.2°, 38.5±0.2°, 41.2±0.2°, 46.8±0.2° and 54.0±0.2°.

In some embodiments, the manganese oxide in the form of a salt-type polymorph is salt-type amorphous manganese oxide. In some embodiments, the salt-type amorphous manganese oxide is selected from one or both of potassium-type amorphous manganese oxide and hydrogen-type amorphous manganese oxide. In some embodiments, the salt-type amorphous manganese oxide is potassium-type amorphous manganese oxide. In some embodiments, the potassium-type amorphous manganese oxide is characterized in that its powder X ray diffraction pattern does not exhibit a distinctly characteristic X ray diffraction peak. In some embodiments, the salt-type amorphous manganese oxide is hydrogen-type amorphous manganese oxide. In some embodiments, the hydrogen-type amorphous manganese oxide is characterized in that its powder X ray diffraction pattern does not exhibit a distinctly characteristic X ray diffraction peak.

The insoluble manganese-containing compounds according to the present disclosure (e.g. manganese oxide) show a high uric acid clearance capacity in simulated intestinal fluid (SIF) assays. The simulated intestinal fluid (SIF) assay may be performed using methods known to those skilled in the art. For example, in some embodiments, the simulated intestinal fluid (SIF) assay is performed in SIF with a concentration of uric acid of about 8.4±0.2 mg/dL, such as 8.2 mg/dL, 8.3 mg/dL, 8.4 mg/dL, 8.5 mg/dL or 8.6 mg/dL. In some embodiments, the SIF has a pH of about 6.8±0.2, such as about 6.6, 6.7, 6.8, 6.9 or 7.0. In some embodiments, the SIF has a phosphate content of about 10 mM to 100 mM, such as about 10 mM to 100 mM, about 20 mM to 90 mM, about 30 mM to 80 mM, about 40 mM to 60 mM, or about 50 mM. In some embodiments, the simulated intestinal fluid (SIF) assay is performed in SIF with a concentration of uric acid of about 8.4±0.2 mg/dL, a pH of about 6.8±0.2, and a phosphate content of about 10 mM to 100 mM. In some embodiments, the simulated intestinal fluid (SIF) assay is performed at a temperature of about 25°C to 37°C, such as a temperature of about 25°C to 26°C, 25°C to 27°C, 25°C to 28°C, 25°C to 29°C, 25°C to 30°C, 25°C to 31°C, 25°C to 32°C, 25°C to 33°C, 25°C to 34°C, 25°C to 35°C, 25°C to 36°C, 26°C to 37°C, 27°C to 37°C, 28°C to 37°C, 29°C to 37°C, 30°C to 37°C, 31°C to 37°C, 32°C to 37°C, 33°C to 37°C, 34°C to 37°C, 35°C to 37°C, or 36°C to 37°C. In some embodiments, the simulated intestinal fluid (SIF) assay is performed at a concentration of an insoluble manganese-containing compound of about 0.1 g/L to 2 g/L, for example, about 0.1 g/L to 0.2 g/L, 0.1 g/L to 0.3 g/L, 0.1 g/L to 0.4 g/L, 0.1 g/L to 0.5 g/L, 0.1 g/L to 0.6 g/L, 0.1 g/L to 0.7 g/L, 0.1 g/L to 0.8 g/L, 0.1 g/L to 0.9 g/L, 0.1 g/L to 1.0 g/L, 0.1 g/L to 1.1 g/L, 0.1 g/L to 1.2 g/L, 0.1 g/L to 1.3 g/L, 0.1 g/L to 1.4 g/L, 0.1 g/L to 1.5 g/L, 0.1 g/L to 1.6 g/L, 0.1 g/L to 1.7 g/L, 0.1 g/L to 1.8 g/L, 0.1 g/L to 1.9 g/L, 0.2 g/L to 2 g/L, 0.3 g/L to 2 g/L, 0.4 g/L to 2 g/L, 0.5 g/L to 2 g/L, 0.6 g/L to 2 g/L, 0.7 g/L to 2 g/L, 0.8 g/L to 2 g/L, 0.9 g/L to 2 g/L, 1.0 g/L to 2 g/L, 1.1 g/L to 2 g/L, 1.2 g/L to 2 g/L, 1.3 g/L to 2 g/L, 1.4 g/L to 2 g/L, 1.5 g/L to 2 g/L, 1.6 g/L to 2 g/L, 1.7 g/L to 2 g/L, 1.8 g/L to 2 g/L or 1.9 g/L to 2.0 g/L. In some embodiments, the simulated intestinal fluid (SIF) assay is performed under oscillatory conditions. The oscillation frequency can be, for example, ≥50 rpm, ≥100 rpm, ≥150 rpm, ≥200 rpm, ≥250 rpm, ≥300 rpm, or higher. In some embodiments, the simulated intestinal fluid (SIF) assay is performed using simulated intestinal fluid (SIF) having a concentration of uric acid of about 8.4±0.2 mg/dL, at a temperature of about 25°C to 37°C, with a concentration of manganese oxide of about 0.1 g/L to 2 g/L, and under oscillatory conditions. In some embodiments, the duration of the simulated intestinal fluid (SIF) assay is ≥5min, such as ≥10min, ≥15min, ≥20min, ≥25min, ≥30min, ≥35min, ≥40min, ≥45min, ≥50min, ≥55min, ≥60min, ≥90min, ≥120min, ≥180min, ≥240min, ≥300min, ≥360min, ≥420min, ≥480min, ≥520min or ≥600min.

In some embodiments, the insoluble manganese-containing compound (e.g. manganese oxide) has a uric acid clearance capacity of about 50 mg/g to about 1000 mg/g as measured in simulated intestinal fluid (SIF) assays.

In some embodiments, the insoluble manganese-containing compound (e.g. manganese oxide) has a uric acid clearance capacity of about 100 mg/g to about 900 mg/g as measured in simulated intestinal fluid (SIF) assays.

In some embodiments, the insoluble manganese-containing compound (e.g. manganese oxide) has a uric acid clearance capacity of about 200 mg/g to about 800 mg/g as measured in simulated intestinal fluid (SIF) assays.

For example, in some embodiments, the insoluble manganese-containing compound (e.g. manganese oxide) has a uric acid clearance capacity measured in simulated intestinal fluid (SIF) assays of about 100 mg/g to about 1000 mg/g, about 150 mg/g to about 1000 mg/g, about 200 mg/g to about 1000 mg/g, about 250 mg/g to about 1000 mg/g, about 300 mg/g to about 1000 mg/g, about 350 mg/g to about 1000 mg/g, about 400 mg/g to about 1000 mg/g, about 450 mg/g to about 1000 mg/g, about 500 mg/g to about 1000 mg/g, about 550 mg/g to about 1000 mg/g, about 600 mg/g to about 1000 mg/g, about 650 mg/g to about 1000 mg/g, about 700 mg/g to about 1000 mg/g, about 750 mg/g to about 1000 mg/g, about 800 mg/g to about 1000 mg/g, about 850 mg/g to about 1000 mg/g, about 900 mg/g to about 1000 mg/g, about 950 mg/g to about 1000 mg/g, about 100 mg/g to about 900 mg/g, about 150 mg/g to about 900 mg/g, about 200 mg/g to about 900 mg/g, about 250 mg/g to about 900 mg/g, about 300 mg/g to about 900 mg/g, about 350 mg/g to about 900 mg/g, about 400 mg/g to about 900 mg/g, about 450 mg/g to about 900 mg/g, about 500 mg/g to about 900 mg/g, about 550 mg/g to about 900 mg/g, about 600 mg/g to about 900 mg/g, about 650 mg/g to about 900 mg/g, about 700 mg/g to about 900 mg/g, about 750 mg/g to about 900 mg/g, about 800 mg/g to about 900 mg/g, about 850 mg/g to about 900 mg/g, about 100 mg/g to about 800 mg/g, about 150 mg/g to about 800 mg/g, about 200 mg/g to about 800 mg/g, about 250 mg/g to about 800 mg/g, about 300 mg/g to about 800 mg/g, about 350 mg/g to about 800 mg/g, about 400 mg/g to about 800 mg/g, about 450 mg/g to about 800 mg/g, about 500 mg/g to about 800 mg/g, about 550 mg/g to about 800 mg/g, about 600 mg/g to about 800 mg/g, about 650 mg/g to about 800 mg/g, about 700 mg/g to about 800 mg/g, about 750 mg/g to about 800 mg/g, about 100 mg/g to about 700 mg/g, about 150 mg/g to about 700 mg/g, about 200 mg/g to about 700 mg/g, about 250 mg/g to about 700 mg/g, about 300 mg/g to about 700 mg/g, about 350 mg/g to about 700 mg/g, about 400 mg/g to about 700 mg/g, about 450 mg/g to about 700 mg/g, about 500 mg/g to about 700 mg/g, about 550 mg/g to about 700 mg/g, about 600 mg/g to about 700 mg/g, about 650 mg/g to about 700 mg/g, about 100 mg/g to about 600 mg/g, about 150 mg/g to about 600 mg/g, about 200 mg/g to about 600 mg/g, about 250 mg/g to about 600 mg/g, about 300 mg/g to about 600 mg/g, about 350 mg/g to about 600 mg/g, about 400 mg/g to about 600 mg/g, about 450 mg/g to about 600 mg/g, about 500 mg/g to about 600 mg/g, about 550 mg/g to about 600 mg/g, about 100 mg/g to about 500 mg/g, about 150 mg/g to about 500 mg/g, about 200 mg/g to about 500 mg/g, about 250 mg/g to about 500 mg/g, about 300 mg/g to about 500 mg/g, about 350 mg/g to about 500mg/g, about 400 mg/g to about 500 mg/g, about 450 mg/g to about 500 mg/g, about 100 mg/g to about 400 mg/g, about 150 mg/g to about 400 mg/g, about 200 mg/g to about 400 mg/g, about 250 mg/g to about 400 mg/g, about 300 mg/g to about 400 mg/g, about 350 mg/g to about 400 mg/g, about 100 mg/g to about 300 mg/g, about 150 mg/g to about 300 mg/g, about 200 mg/g to about 300 mg/g, about 250 mg/g to about 300 mg/g, about 100 mg/g to about 200 mg/g, or about 150 mg/g to about 200 mg/g.

The insoluble manganese-containing compounds according to the present disclosure (e.g. manganese oxide) also show a high uric acid clearance capacity in fasted sate simulated intestinal fluid (FaSSIF) and/or fed sate simulated intestinal fluid (FeSSIF) assays. The fasted sate simulated intestinal fluid (FaSSIF) or fed sate simulated intestinal fluid (FeSSIF) assay can be performed using methods known to those skilled in the art. In some embodiments, the fasted sate simulated intestinal fluid (FaSSIF) or fed sate simulated intestinal fluid (FeSSIF) assay is performed in FaSSIF or FeSSIF with a concentration of uric acid of about $8.4 \pm 0.2$ mg/dL, such as 8.2 mg/dL, 8.3 mg/dL, 8.4 mg/dL, 8.5 mg/dL or 8.6 mg/dL. In some embodiments, the fasted sate simulated intestinal fluid (FaSSIF) or fed sate simulated intestinal fluid (FeSSIF) assay is performed at a temperature of about 25°C to 37°C, such as a temperature of about 25°C to 26°C, 25°C to 27°C, 25°C to 28°C, 25°C to 29°C, 25°C to 30°C, 25°C to 31°C, 25°C to 32°C, 25°C to 33°C, 25°C to 34°C, 25°C to 35°C, 25°C to 36°C, 26°C to 37°C, 27°C to 37°C, 28°C to 37°C, 29°C to 37°C, 30°C to 37°C, 31°C to 37°C, 32°C to 37°C, 33°C to 37°C, 34°C to 37°C, 35°C to 37°C, or 36°C to 37°C. In some embodiments, the fasted sate simulated intestinal fluid (FaSSIF) or fed sate simulated intestinal fluid (FeSSIF) assay is performed at a concentration of an insoluble manganese-containing compound of about 0.1 g/L to 2 g/L, for example, about 0.1 g/L to 0.2 g/L, 0.1 g/L to 0.3 g/L, 0.1 g/L to 0.4 g/L, 0.1 g/L to 0.5 g/L, 0.1 g/L to 0.6 g/L, 0.1 g/L to 0.7 g/L, 0.1 g/L to 0.8 g/L, 0.1 g/L to 0.9 g/L, 0.1 g/L to 1.0 g/L, 0.1 g/L to 1.1 g/L, 0.1 g/L to 1.2 g/L, 0.1 g/L to 1.3 g/L, 0.1 g/L to 1.4 g/L, 0.1 g/L to 1.5 g/L, 0.1 g/L to 1.6 g/L, 0.1 g/L to 1.7 g/L, 0.1 g/L to 1.8 g/L, 0.1 g/L to 1.9 g/L, 0.2 g/L to 2 g/L, 0.3 g/L to 2 g/L, 0.4 g/L to 2 g/L, 0.5 g/L to 2 g/L, 0.6 g/L to 2 g/L, 0.7 g/L to 2 g/L, 0.8 g/L to 2 g/L, 0.9 g/L to 2 g/L, 1.0 g/L to 2 g/L, 1.1 g/L to 2 g/L, 1.2 g/L to 2 g/L, 1.3 g/L to 2 g/L, 1.4 g/L to 2 g/L, 1.5 g/L to 2 g/L, 1.6 g/L to 2 g/L, 1.7 g/L to 2 g/L, 1.8 g/L to 2 g/L or 1.9 g/L to 2.0 g/L. In some embodiments, the fasted sate simulated intestinal fluid (FaSSIF) or fed sate simulated intestinal fluid (FeSSIF) assay is performed under oscillatory conditions. The oscillation frequency can be, for example, $\geq$50 rpm, $\geq$100 rpm, $\geq$150 rpm, $\geq$200 rpm, $\geq$250 rpm, $\geq$300 rpm, or higher. In some embodiments, the fasted sate simulated intestinal fluid (FaSSIF) or fed sate simulated intestinal fluid (FeSSIF) assay is performed using fasted sate simulated intestinal fluid (FaSSIF) or fed sate simulated intestinal fluid (FeSSIF) having a concentration of uric acid of about $8.4 \pm 0.2$ mg/dL, at a temperature of about 25°C to 37°C, with a concentration of manganese oxide of about 0.1 g/L to 2 g/L, and under oscillatory conditions. In some embodiments, the duration of the fasted sate simulated intestinal fluid (FaSSIF) or fed sate simulated intestinal fluid (FeSSIF) assay is $\geq$5min, such as $\geq$10min, $\geq$15min, $\geq$20min, $\geq$25min, $\geq$30min, $\geq$35min, $\geq$40min, $\geq$45min, $\geq$50min, $\geq$55min, $\geq$60min, $\geq$90min, $\geq$120min, $\geq$180min, $\geq$240min, $\geq$300min, $\geq$360min, $\geq$420min, $\geq$480min, $\geq$520min or >600min.

In some embodiments, the insoluble manganese-containing compound (e.g. manganese oxide) has a uric acid clearance capacity of about 200 mg/g to about 800 mg/g as measured in fasted sate simulated intestinal fluid (FaSSIF) assays.

In some embodiments, the insoluble manganese-containing compound (e.g. manganese oxide) has a uric acid clearance capacity of about 200 mg/g to about 800 mg/g as measured in fed sate simulated intestinal fluid (FeSSIF) assays.

For example, in some embodiments, the insoluble manganese-containing compound (e.g. manganese oxide) has a uric acid clearance capacity measured in fasted state simulated intestinal fluid (FaSSIF) assay of about 250 mg/g to about 800 mg/g, about 300 mg/g to about 800 mg/g, about 350 mg/g to about 800 mg/g, about 400 mg/g to about 800 mg/g, about 450 mg/g to about 800 mg/g, about 500 mg/g to about 800 mg/g, about 550 mg/g to about 800 mg/g, about 600 mg/g to about 800 mg/g, about 650 mg/g to about 800 mg/g, about 700 mg/g to about 800 mg/g, about 750 mg/g to about 800 mg/g, about 200 mg/g to about 700 mg/g, about 250 mg/g to about 700 mg/g, about 300 mg/g to about 700 mg/g, about 350 mg/g to about 700 mg/g, about 400 mg/g to about 700 mg/g, about 450 mg/g to about 700 mg/g, about 500 mg/g to about 700 mg/g, about 550 mg/g to about 700 mg/g, about 600 mg/g to about 700 mg/g, about 650 mg/g to about 700 mg/g, about 200 mg/g to about 600 mg/g, about 250 mg/g to about 600 mg/g, about 300 mg/g to about 600 mg/g, about 350 mg/g to about 600 mg/g, about 400 mg/g to about 600 mg/g, about 450 mg/g to about 600 mg/g, about 500 mg/g to about 600 mg/g, about 550 mg/g to about 600 mg/g, about 200 mg/g to about 500 mg/g, about 250 mg/g to about 500 mg/g, about 300 mg/g to about 500 mg/g, about 350 mg/g to about 500 mg/g, about 400 mg/g to about 500 mg/g, about 450 mg/g to about 500 mg/g, about 200 mg/g to about 400 mg/g, about 250 mg/g to about 400 mg/g, about 300 mg/g to about 400 mg/g, about 350 mg/g to about 400 mg/g, about 200 mg/g to about 300 mg/g or about 250 mg/g to about 300 mg/g. In some embodiments, the insoluble manganese-containing compound has a uric acid clearance capacity measured in fed state simulated intestinal fluid (FeSSIF) assay of about 250 mg/g to about 800 mg/g, about 300 mg/g to about 800 mg/g, about 350 mg/g to about 800 mg/g, about 400 mg/g to about 800 mg/g, about 450 mg/g to about 800 mg/g, about 500 mg/g to about 800 mg/g, about 550 mg/g to about 800 mg/g, about 600 mg/g to about 800 mg/g, about 650 mg/g to about 800 mg/g, about 700 mg/g to about 800 mg/g, about 750 mg/g to about 800 mg/g, about 200 mg/g to about 700 mg/g, about 250 mg/g to about 700 mg/g, about 300 mg/g to about 700 mg/g, about 350 mg/g to about 700 mg/g, about 400 mg/g to about 700 mg/g, about 450 mg/g to about 700 mg/g, about 500 mg/g to about 700 mg/g, about 550 mg/g to about 700 mg/g, about 600 mg/g to about 700 mg/g, about 650 mg/g to about 700 mg/g, about 200 mg/g to about 600, about 250 mg/g to about 600 mg/g, about 300 mg/g to about 600 mg/g, about 350 mg/g, to about 600 mg/g, about 400 mg/g to about 600 mg/g, about 450 mg/g to about 600 mg/g, about 500 mg/g to about 600 mg/g, about 550 mg/g to about 600 mg/g, about 200 mg/g to about 500 mg/g, about 250 mg/g to about 500 mg/g, about 300 mg/g to about 500 mg/g, about 350 mg/g to about 500 mg/g, about 400 mg/g to about 500 mg/g, about 450 mg/g to about 500 mg/g, about 200 mg/g to about 400 mg/g, about 250 mg/g to about 400 mg/g, about 300 mg/g to about 400 mg/g, about 350 mg/g to about 400 mg/g, about 200 mg/g to about 300 mg/g or about 250 mg/g to about 300 mg/g.

In some embodiments, the drug is an oral formulation.

In some embodiments, the oral formulation is a solid oral formulation. In some embodiments, the solid oral formulation is powder, granule, tablet, capsule, pill, or lozenge. In some embodiments, the oral formulation is a liquid oral formulation.

In some embodiments, the drug does not contain an additional therapeutic agent.

In some embodiments, the drug comprises an effective amount of an insoluble manganese-containing compound (e.g. manganese oxide). In some embodiments, the drug comprises about 3mg to about 3000mg, about 3mg to about 1500mg, about 5mg to about 500mg, or about 10mg to about 200mg of an insoluble manganese-containing compound. For example, in some embodiments, the drug comprises about 3mg to about 3000mg, about 3mg to about 2500mg, about 3mg to about 2000mg, about 3mg to about 1500mg, about 3mg to about 1000mg, about 3mg to about 900mg, about 3mg to about 800mg, about 3mg to about 700mg, about 3mg to about 600mg, about 3mg to about 500mg, about 3mg to about 400mg, about 3mg to about 300mg, about 3mg to about 200mg, about 5mg to about 3000mg, about 5mg to about 2500mg, about 5mg to about 2000mg, about 5mg to about 1500mg, about 5mg to about 1000mg, about 5mg to about 900mg, about 5mg to about 800mg, about 5mg to about 700mg, about 5mg to about 600mg, about 5mg to about 500mg, about 5mg to about 400mg, about 5mg to about 300mg, about 5mg to about 200mg, about 10mg to about 3000mg, about 10mg to about 2500mg, about 10mg to about 2000mg, about 10mg to about 1500mg, about 10mg to about 1000mg, about 10mg to about 900mg, about 10mg to about 800mg, about 10mg to about 700mg, about 10mg to about 600mg, about 10mg to about 500mg, about 10mg to about 400mg, about 10mg to about 300mg, or about 10mg to about 200mg of an insoluble manganese-containing compound. In some embodiments, the drug comprises about 15mg, about 20mg, about 25mg, about 30mg, about 35mg, about 40mg, about 45mg, about 50mg, about 55mg, about 60mg, about 65mg, about 70mg, about 75mg, about 80mg, about 85mg, about 90mg, about 100mg, about 110mg, about 120mg, about 130mg, about 140mg, about 150mg, about 160mg, about 170mg, about 180mg, about 190mg, about 200mg, about 300mg, about 400mg, about 500mg, about 600mg, about 700mg, about 800mg, about 900mg, about 1000mg, about 1500mg, about 2000mg, about 2500mg, or about 3000 mg of an insoluble manganese-containing compound.

In some embodiments, the mammal is a human. In some embodiments, the mammal is an adult man aged ≥18 years. In some embodiments, the mammal is an adult man aged ≥18 years and <60 years. In some embodiments, the mammal is an adult man aged ≥60 years. In some embodiments, the mammal is an adult woman aged ≥18 years. In some embodiments, the mammal is an adult woman aged ≥18 years and <49 years. In some embodiments, the mammal is an adult woman aged ≥49 years.

In some embodiments, the mammal is a mammal with reduced ability to clear uric acid via the kidneys. For example, in some embodiments, the mammal is a mammal having one or more of gouty urinary calculus, gouty nephropathy, and diabetes. In some embodiments, the mammal is a mammal with gouty urinary calculus. In some embodiments, the mammal is a mammal having gouty nephropathy, such as at least one of nephritis, pyelonephritis, hydronephrosis, uric acid nephrolithiasis, renal dysfunction, renal failure, and uremia. In some embodiments, the mammal is a mammal having diabetes.

In some embodiments, the digestive tract is the intestinal tract. For example, in some embodiments, the digestive tract is the small intestine. In some embodiments, the digestive tract is the duodenum. In some embodiments, the digestive tract is the jejunum. In some embodiments, the digestive tract is the ileum. In some embodiments, the digestive tract is the large intestine. In some embodiments, the digestive tract is the cecum. In some embodiments, the digestive tract is the colon (e.g. one or more of the ascending colon, transverse colon, descending colon, and sigmoid colon). In some embodiments, the digestive tract is the rectum. In some embodiments, the digestive tract is the small intestine and/or large intestine. In some embodiments, the digestive tract is one or more of the duodenum, jejunum, and ileum. In some embodiments, the digestive tract is one or more of the cecum, colon and rectum. In some embodiments, the digestive tract is one or more of the duodenum, jejunum, ileum, cecum, colon and rectum.

In some embodiments, the disease or disorder associated with increased uric acid levels in the blood and/or digestive tract is at least one selected from the group consisting of: gout, gout complications, hyperuricemia, higher uric acid levels not reaching levels typically diagnosed as hyperuricemia, cardiovascular disease, diabetes, diabetes-related disorders, insulin resistance, metabolic syndrome, hypothyroidism, hyperparathyroidism, obesity, inflammation, muscle spasms, local swelling, joint pain, malignancies, tumor lysis syndrome, polycythemia vera, cognitive disorder, psoriasis, sarcoidosis, non-alcoholic fatty liver disease, stroke, hemolytic anemia, congenital metabolic genetic errors, poisoning, epididymitis and orchitis, and transplantation of blood, bone marrow, or solid organs.

In some embodiments, the gout is acute gout, chronic gout, or refractory gout.

In some embodiments, the gout complication is at least one of gouty arthritis, gouty urinary calculus, and gouty nephropathy.

In some embodiments, the gouty nephropathy is at least one of nephritis, pyelonephritis, hydronephrosis, uric acid nephrolithiasis, renal dysfunction, renal failure, and uremia.

In some embodiments, the cardiovascular disease is at least one of hypertension, coronary heart disease, heart failure, congenital heart disease, deep vein thrombosis, pulmonary embolism, aortic aneurysm, aortic dissection, hyperlipidemia, myocardial infarction and atherosclerosis.

In some embodiments, the hyperuricemia is primary hyperuricemia or secondary hyperuricemia.

In some embodiments, the secondary hyperuricemia is at least one of drug-related hyperuricemia and hyperuricemia associated with other medical conditions.

In some embodiments, the diabetes-related condition is at least one of diabetic nephropathy, diabetic peripheral neuropathy, diabetic retinopathy, diabetic macroangiopathy, diabetic microangiopathy, diabetic foot, and diabetic ketoacidosis.

In some embodiments, the malignant tumor is a hematological malignancy.

In some embodiments, the hematological malignancy is at least one of leukemia and multiple myeloma. In some embodiments, the leukemia is acute lymphocytic leukemia, chronic lymphocytic leukemia, acute myeloid leukemia, or chronic myeloid leukemia.

In some embodiments, the non-alcoholic fatty liver disease is nonalcoholic steatohepatitis.

In some embodiments, the poisoning is at least one of chloroform poisoning, carbon tetrachloride poisoning, and lead poisoning.

In some embodiments, the congenital metabolic genetic error is Lesch-Nyhan syndrome.

In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240μmol/L, 260μmol/L, 280μmol/L, 300μmol/L, 320μmol/L, 340μmol/L, 360μmol/L, 380μmol/L, 400μmol/L or 420μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 260μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 280μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 300μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 320μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 340μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 360μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 380μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 400μmol/L. In some embodiments, prior to administration of the drug to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 420μmol/L.

In some embodiments, the subject's blood and/or serum uric acid levels after administration of the drug are reduced compared to those prior to administration of the drug.

In some embodiments, the subject 'blood and/or serum uric acid levels after administration of the drug are reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% compared to those prior to administration of the drug.

Still further, in some embodiments, the subject's blood and/or serum uric acid levels after administration of the drug are 80μmol/L to 460μmol/L, 100μmol/L to 450μmol/L, 150μmol/L to 440μmol/L, 200μmol/L to 430μmol/L, 210μmol/L to 420μmol/L, 80 μmol/L to 380 μmol/L, 100μmol/L to 370 μmol/L, or 150μmol/L to 360μmol/L.

In some embodiments, 4h, 5h, 6h, 7h, 8h, 9h, 10h, 11h, 12h, 14h, 16h, 18h, 20h, 22h, 24h, 36h, 48h, 3 days, 4 days, 5 days, 6 days, or 7 days after administration of the drug at an effective amount, the subject's blood and/or serum uric acid levels are reduced compared to those prior to administration of the drug.

In a second aspect, the present disclosure provides use of the manganese-containing compound as described herein in the preparation of a drug for adsorbing uric acid in the digestive tract of a mammal.

In some embodiments, the manganese-containing compound is an insoluble manganese-containing compound.

Other embodiments of this aspect may be referred to those detailed in the first aspect above.

In a third aspect, the present disclosure provides a pharmaceutical composition for preventing and/or treating increased uric acid levels in the blood and/or digestive tract or a disease or disorder associated with increased uric acid levels in the blood and/or digestive tract in a mammalian subject, wherein the pharmaceutical composition comprises an insoluble manganese-containing compound and a pharmaceutically acceptable excipient.

In some embodiments, the pharmaceutical composition is an oral formulation.

In some embodiments, the oral formulation is a solid oral formulation. In some embodiments, the solid oral formulation is powder, granule, tablet, capsule, pill, or lozenge. In some embodiments, the oral formulation is a liquid oral formulation.

In some embodiments, the pharmaceutical composition does not contain additional therapeutic agents.

In some embodiments, the pharmaceutical composition comprises an effective amount of an insoluble manganese-containing compound (e.g. manganese oxide). In some embodiments, the pharmaceutical composition comprises about 3mg to about 3000mg, about 3mg to about 1500mg, about 5mg to about 500mg, or about 10mg to about 200mg of an insoluble manganese-containing compound. For example, in some embodiments, the pharmaceutical composition comprises about 3mg to about 3000mg, about 3mg to about 2500mg, about 3mg to about 2000mg, about 3mg to about 1500mg, about 3mg to about 1000mg, about 3mg to about 900mg, about 3mg to about 800mg, about 3mg to about 700mg, about 3mg to about 600mg, about 3mg to about 500mg, about 3mg to about 400mg, about 3mg to about 300mg, about 3mg to about 200mg, about 5mg to about 3000mg, about 5mg to about 2500mg, about 5mg to about 2000mg, about 5mg to about 1500mg, about 5mg to about 1000mg, about 5mg to about 900mg, about 5mg to about 800mg, about 5mg to about 700mg, about 5mg to about 600mg, about 5mg to about 500mg, about 5mg to about 400mg, about 5mg to about 300mg, about 5mg to about 200mg, about 10mg to about 3000mg, about 10mg to about 2500mg, about 10mg to about 2000mg, about 10mg to about 1500mg, about 10mg to about 1000mg, about 10mg to about 900mg, about 10mg to about 800mg, about 10mg to about 700mg, about 10mg to about 600mg, about 10mg to about 500mg, about 10mg to about 400mg, about 10mg to about 300mg or about 10mg to about 200mg of an insoluble manganese-containing compound. In some embodiments, the pharmaceutical composition comprises about 15mg, about 20mg, about 25mg, about 30mg, about 35mg, about 40mg, about 45mg, about 50mg, about 55mg, about 60mg, about 65mg, about 70mg, about 75mg, about 80mg, about 85mg, about 90mg, about 100mg, about 110mg, about 120mg, about 130mg, about 140mg, about 150mg, about 160mg, about 170mg, about 180mg, about 190mg, about 200mg, about 300mg, about 400mg, about 500mg, about 600mg, about 700mg, about 800mg, about 900mg, about 1000mg, about 1500mg, about 2000mg, about 2500mg or about 3000 mg of an insoluble manganese-containing compound.

In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240μmol/L, 260μmol/L, 280μmol/L, 300μmol/L, 320μmol/L, 340μmol/L, 360μmol/L, 380μmol/L, 400μmol/L or 420μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 260μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 280μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 300μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 320μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 340μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 360μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 380μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 400μmol/L. In some embodiments, prior to administration of the pharmaceutical composition to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 420μmol/L.

In some embodiments, the subject's blood and/or serum uric acid levels after administration of the pharmaceutical composition are reduced compared to those prior to administration of the pharmaceutical composition.

In some embodiments, the subject 'blood and/or serum uric acid levels after administration of the pharmaceutical composition are reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% compared to those prior to administration of the pharmaceutical composition.

Still further, in some embodiments, the subject's blood and/or serum uric acid levels after administration of the pharmaceutical composition are 80μmol/L to 460μmol/L, 100μmol/L to 450μmol/L, 150μmol/L to 440μmol/L, 200μmol/L to 430μmol/L, 210 μmol/L to 420μmol/L, 80 μmol/L to 380 μmol/L, 100μmol/L to 370 μmol/L, or 150μmol/L to 360μmol/L.

In some embodiments, 4h, 5h, 6h, 7h, 8h, 9h, 10h, 11h, 12h, 14h, 16h, 18h, 20h, 22h, 24h, 36h, 48h, 3 days, 4 days, 5 days, 6 days, or 7 days after administration of the pharmaceutical composition at an effective amount, the subject's blood and/or serum uric acid levels are reduced compared to those prior to administration of the pharmaceutical composition.

Other embodiments of this aspect may be referred to those detailed in the first aspect above.

In a fourth aspect, the present disclosure provides a kit comprising: (i) any of the pharmaceutical compositions according to the present disclosure; and (ii) drug instructions and/or drug labels.

As used throughout this article, the term "kit", alternatively referred to as "drug box", refers to a package for use in the commercially available form of a pharmaceutical composition, optionally in the form of a pharmaceutically acceptable formulation such as a unit dosage form. The package may be made from a material including, but not limited to, one or more of paper, glass, metal, and plastic. In the kit according to the present disclosure, the pharmaceutical composition may be placed in at least one container in the package. As a non-limiting example, the container may be, for example, an ampoule, a bottle, a test tube, a bag, a blister pack, a syringe, and/or a dispenser. In addition to the container for placing the pharmaceutical composition, the kit may optionally include one or more additional containers containing pharmaceutically acceptable excipients for diluting or suspending the pharmaceutical composition. In addition, the kit according to the present disclosure also comprises drug instructions and/or drug labels. In the context of the present disclosure, "drug instructions" should be understood as a paper specification placed inside the kit, which is intended to provide information about the ingredients, administration routes, side effects, etc. of the pharmaceutical composition. In addition, "drug label" should be understood as a label containing explanatory text related to the pharmaceutical composition, which is attached to the outer surface of the container and/or package or which itself constitutes a part of the outer surface of the container and/or packaging.

In a fifth aspect, the present disclosure provides a method for preventing and/or treating increased uric acid levels in the blood and/or digestive tract or a disease or disorder associated with increased uric acid levels in the blood and/or digestive tract in a mammalian subject, which comprises administering an effective amount of an insoluble manganese-containing compound to a subject in need thereof.

In a sixth aspect, the present disclosure provides a method for preventing and/or treating increased uric acid levels in the blood and/or digestive tract or a disease or disorder associated with increased uric acid levels in the blood and/or digestive tract in a mammalian subject, which comprises administering an effective amount of an insoluble manganese-containing compound to a subject in need thereof over an extended period of time.

In a seventh aspect, the present disclosure provides a method for maintaining uric acid levels in the blood in a mammalian subject, comprising administering to a subject in need thereof an effective amount of an insoluble manganese-containing compound over an extended period of time.

In some embodiments, the method maintains blood and/or serum uric acid levels in the subject at blood and/or serum uric acid levels that do not cause or are less likely to cause gout or gout complications. In some embodiment, the method maintains blood and/or serum uric acid levels in the subject at 80μmol/L to 460μmol/L, 100μmol/L to 450μmol/L, 150μmol/L to 440μmol/L, 200μmol/L to 430μmol/L, 210 μmol/L to 420μmol/L, 80 μmol/L to 380 μmol/L, 100μmol/L to 370 μmol/L, or 150μmol/L to 360μmol/L.

In some embodiments, the effective amount is about 3mg to about 3000mg of an insoluble manganese-containing compound (e.g. manganese oxide) per day. In some embodiments, the effective amount is about 3mg to about 1500mg of an insoluble manganese-containing compound per day. In some embodiments, the effective amount is about 5mg to about 500mg of an insoluble manganese-containing compound per day. In some embodiments, the effective amount is about 10mg to about 200mg of an insoluble manganese-containing compound per day. For example, in some embodiments, the effective amount is about 3mg to about 3000mg of an insoluble manganese-containing compound per day, about 3mg to about 1500mg of an insoluble manganese-containing compound per day, about 3mg to about 1000mg of an insoluble manganese-containing compound per day, about 3mg to about 900mg of an insoluble manganese-containing compound per day, about 3mg to about 800mg of an insoluble manganese-containing compound per day, about 3mg to about 700mg of an insoluble manganese-containing compound per day, about 3mg to about 600mg of an insoluble manganese-containing compound per day, about 3mg to about 500mg of an insoluble manganese-containing compound per day, about 3mg to about 400mg of an insoluble manganese-containing compound per day, about 3mg to about 300mg of an insoluble manganese-containing compound per day, about 3mg to about 200mg of an insoluble manganese-containing compound per day, about 5mg to about 3000mg of an insoluble manganese-containing compound per day, about 5mg to about 1500mg of an insoluble manganese-containing compound per day, about 5mg to about 1000mg of an insoluble manganese-containing compound per day, about 5mg to about 900mg of an insoluble manganese-containing compound per day, about 5mg to about 800mg of an insoluble manganese-containing compound per day, about 5mg to about 700mg of an insoluble manganese-containing compound per day, about 5mg to about 600mg of an insoluble manganese-containing compound per day, about 5mg to about 500mg of an insoluble manganese-containing compound per day, about 5mg to about 400mg of an insoluble manganese-containing compound per day, about 5mg to about 300mg of an insoluble manganese-containing compound per day, about 5mg to about 200mg of an insoluble manganese-containing compound per day, about 10mg to about 3000mg of an insoluble manganese-containing compound per day, about 10mg to about 1500mg of an insoluble manganese-containing compound per day, about 10mg to about 1000mg of an insoluble manganese-containing compound per day, about 10mg to about 900mg of an insoluble manganese-containing compound per day, about 10mg to about 800mg of an insoluble manganese-containing compound per day, about 10mg to about 700mg of an insoluble manganese-containing compound per day, about 10mg to about 600mg of an insoluble manganese-containing compound per day, about 10mg to about 500mg of an insoluble manganese-containing compound per day, about 10mg to about 400mg of an insoluble manganese-containing compound per day, about 10mg to about 300mg of an insoluble manganese-containing compound per day, or about 10mg to about 200mg of an insoluble manganese-containing compound per day. In some embodiments, the pharmaceutical composition comprises about 15mg, about 20mg, about 25mg, about 30mg, about 35mg, about 40mg, about 45mg, about 50mg, about 55mg, about 60mg, about 65mg, about 70mg, about 75mg, about 80mg, about 85mg, about 90mg, about 100mg, about 110mg, about 120mg, about 130mg, about 140mg, about 150mg, about 160mg, about 170mg, about 180mg, about 190mg, about 200mg, about 300mg, about 400mg, about 500mg, about 600mg, about 700mg, about 800mg, about 900mg, about 1000mg, about 1500mg, about 2000mg, about 2500mg or about 3000 mg of an insoluble manganese-containing compound per day.

In some embodiments, the insoluble manganese-containing compound (e.g. manganese oxide) is administered orally.

In some embodiments, the insoluble manganese-containing compound (e.g. manganese oxide) is prepared into an oral formulation.

In some embodiments, the oral formulation is a solid oral formulation. In some embodiments, the solid oral formulation is powder, granule, tablet, capsule, pill, or lozenge. In some embodiments, the oral formulation is a liquid oral formulation.

In some embodiments, the oral formulation comprises an effective amount of an insoluble manganese-containing compound (e.g. manganese oxide). In some embodiments, the oral formulation comprises about 3mg to about 3000mg, about 3mg to about 1500mg, about 5mg to about 500mg, or about 10mg to about 200mg of an insoluble manganese-containing compound. For example, in some embodiments, the oral formulation comprises about 3mg to about 3000mg, about 3mg to about 2500mg, about 3mg to about 2000mg, about 3mg to about 1500mg, about 3mg to about 1000mg, about 3mg to about 900mg, about 3mg to about 800mg, about 3mg to about 700mg, about 3mg to about 600mg, about 3mg to about 500mg, about 3mg to about 400mg, about 3mg to about 300mg, about 3mg to about 200mg, about 5mg to about 3000mg, about 5mg to about 2500mg, about 5mg to about 2000mg, about 5mg to about 1500mg, about 5mg to about 1000mg, about 5mg to about 900mg, about 5mg to about 800mg, about 5mg to about 700mg, about 5mg to about 600mg, about 5mg to about 500mg, about 5mg to about 400mg, about 5mg to about 300mg, about 5mg to about 200mg, about 10mg to about 3000mg, about 10mg to about 2500mg, about 10mg to about 2000mg, about 10mg to about 1500mg, about 10mg to about 1000mg, about 10mg to about 900mg, about 10mg to about 800mg, about 10mg to about 700mg, about 10mg to about 600mg, about 10mg to about 500mg, about 10mg to about 400mg, about 10mg to about 300mg or about 10mg to about 200mg of an insoluble manganese-containing compound. In some embodiments, the oral formulation comprises about 15mg, about 20mg, about 25mg, about 30mg, about 35mg, about 40mg, about 45mg, about 50mg, about 55mg, about 60mg, about 65mg, about 70mg, about 75mg, about 80mg, about 85mg, about 90mg, about 100mg, about 110mg, about 120mg, about 130mg, about 140mg, about 150mg, about 160mg, about 170mg, about 180mg, about 190mg, about 200mg, about 300mg, about 400mg, about 500mg, about 600mg, about 700mg, about 800mg, about 900mg, about 1000mg, about 1500mg, about 2000mg, about 2500mg or about 3000 mg of an insoluble manganese-containing compound.

In some embodiment, the insoluble manganese-containing compound (e.g. manganese oxide) is administered to the subject once, twice, or more times daily. In some embodiments, the insoluble manganese-containing compound is administered to the subject once daily. In some embodiments, the insoluble manganese-containing compound is administered to the subject twice daily. In some embodiments, the insoluble manganese-containing compound is administered to the subject three times daily. In some embodiments, the insoluble manganese-containing compound is administered to the subject four times or more daily.

In some embodiments, an effective amount of an insoluble manganese-containing compound (e.g. manganese oxide) is administered to a subject in need thereof over an extended period of time. In view of its advantageous biological properties (including but not limited to low solubility and/or physiological inertness), the insoluble manganese-containing compound according to the present disclosure (e.g. manganese oxide) may be administered to a subject in need thereof over a prolonged period of time (i.e. an extended period of time) to prevent and/or treat increased uric acid levels in the blood and/or digestive tract or diseases or disorders associated with increased uric acid levels in the blood and/or digestive tract in a mammalian subject, to adsorb uric acid in the digestive tract of the mammal, and/or to maintain (normal or appropriate) uric acid levels in the blood in the mammalian subject. For example, in some embodiments, the administration lasts 1 day, 2 days, 3 days, 4 days, 5 days, 6 days, 1 week, 2 weeks, 3 weeks, 1 month, 2 months, 3 months, 4 months, 5 months, 6 months, 8 months, 10 months, 1 year, 1.5 years, 2 years, 2.5 years, 3 years, 4 years, 5 years, 10 years or longer (e.g. the entire duration of the life of the subject). In some embodiments, the extended period of time is 1 day, 2 days, 3 days, 4 days, 5 days, 6 days, 1 week, 2 weeks, 3 weeks, 1 month, 2 months, 3 months, 4 months, 5 months, 6 months, 8 months, 10 months, 1 year, 1.5 years, 2 years, 2.5 years, 3 years, 4 years, 5 years, 10 years or longer (e.g. the entire duration of the life of the subject).

The insoluble manganese-containing compounds according to the present disclosure (e.g. manganese oxide) have an adsorption effect on uric acid that is not, substantially, or less affected by eating, so that the insoluble manganese-containing compounds and foods may be taken simultaneously or sequentially at certain time intervals. Thus, in some embodiments, the insoluble manganese-containing compounds and foods are taken simultaneously or at intervals of less than 1min, 2min, 3min, 4min, 5min, 10min, 15min, 20min, 25min, or 30min. In some embodiments, the insoluble manganese-containing compound is administered 30min, 40min, 50min, 1 h, 1.5h, 2 h or more aft food intake. The food may be, but is not limited to, one or more of meats (e.g. pork, beef, chicken, lamb, fish, shrimp), vegetables, fruits, cereals (e.g. rice and/or millet).

In some embodiments, prior to administration of insoluble manganese-containing compound (e.g. manganese oxide) to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240$\mu$mol/L, 260$\mu$mol/L, 280$\mu$mol/L, 300$\mu$mol/L, 320$\mu$mol/L, 340$\mu$mol/L, 360$\mu$mol/L, 380$\mu$mol/L, 400$\mu$mol/L or 420$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 240$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 260$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 280$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 300$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 320$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 340$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 360$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 380$\mu$mol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 400µmol/L. In some embodiments, prior to administration of the insoluble manganese-containing compound to the subject, the subject's blood and/or serum uric acid levels are at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% higher than 420µmol/L.

In some embodiments, the subject's blood and/or serum uric acid levels after administration of the insoluble manganese-containing compound (e.g. manganese oxide) are reduced compared to those prior to administration of the insoluble manganese-containing compound.

In some embodiments, the subject's blood and/or serum uric acid levels after administration of the insoluble manganese-containing compound (e.g. manganese oxide) are reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% compared to those prior to administration of the insoluble manganese-containing compound.

Still further, in some embodiments, the subject's blood and/or serum uric acid levels after administration of the insoluble manganese-containing compound (e.g. manganese oxide) are 80µmol/L to 460µmol/L, 100µmol/L to 450µmol/L, 150µmol/L to 440µmol/L, 200µmol/L to 430µmol/L, 210 µmol/L to 420µmol/L, 80 µmol/L to 380 µmol/L, 100µmol/L to 370 µmol/L, or 150µmol/L to 360µmol/L.

In some embodiments, 4h, 5h, 6h, 7h, 8h, 9h, 10h, 11h, 12h, 14h, 16h, 18h, 20h, 22h, 24h, 36h, 48h, 3 days, 4 days, 5 days, 6 days, or 7 days after administration of the insoluble manganese-containing compound (e.g. manganese oxide) at an effective amount, the subject's blood and/or serum uric acid levels are reduced compared to those prior to administration of the insoluble manganese-containing compound.

Other embodiments of this aspect may be referred to those detailed in the first aspect above.

In an eighth aspect, the present disclosure provides a pharmaceutical composition, wherein the amount of manganese oxide is sufficient to reduce blood and/or serum uric acid levels by at least about 5% in a mammalian subject whose blood and/or serum uric acid levels are at least 10% higher than 360µmol/L or 420µmol/L within 24 hours after administration of the pharmaceutical composition.

In some embodiments, the particle size of the manganese oxide is ≥1 µm, e.g. 1 µm to 1 mm.

In some embodiments, the pharmaceutical composition is an oral formulation.

In some embodiments, the oral formulation is a solid oral formulation. In some embodiments, the solid oral formulation is powder, granule, tablet, capsule, pill or lozenge. In some embodiments, the oral formulation is a liquid oral formulation.

In some embodiments, the pharmaceutical composition does not contain additional therapeutic agents.

In some embodiments, the pharmaceutical composition comprises an effective amount of manganese oxide. In some embodiments, the pharmaceutical composition comprises about 3mg to about 3000mg, about 3mg to about 1500mg, about 5mg to about 500mg, or about 10mg to about 200mg of manganese oxide. For example, in some embodiments, the pharmaceutical composition comprises about 3mg to about 3000mg, about 3mg to about 2500mg, about 3mg to about 2000mg, about 3mg to about 1500mg, about 3mg to about 1000mg, about 3mg to about 900mg, about 3mg to about 800mg, about 3mg to about 700mg, about 3mg to about 600mg, about 3mg to about 500mg, about 3mg to about 400mg, about 3mg to about 300mg, about 3mg to about 200mg, about 5mg to about 3000mg, about 5mg to about 2500mg, about 5mg to about 2000mg, about 5mg to about 1500mg, about 5mg to about 1000mg, about 5mg to about 900mg, about 5mg to about 800mg, about 5mg to about 700mg, about 5mg to about 600mg, about 5mg to about 500mg, about 5mg to about 400mg, about 5mg to about 300mg, about 5mg to about 200mg, about 10mg to about 3000mg, about 10mg to about 2500mg, about 10mg to about 2000mg, about 10mg to about 1500mg, about 10mg to about 1000mg, about 10mg to about 900mg, about 10mg to about 800mg, about 10mg to about 700mg, about 10mg to about 600mg, about 10mg to about 500mg, about 10mg to about 400mg, about 10mg to about 300mg or about 10mg to about 200mg of manganese oxide. **In** some embodiments, the pharmaceutical composition comprises about 15mg, about 20mg, about 25mg, about 30mg, about 35mg, about 40mg, about 45mg, about 50mg, about 55mg, about 60mg, about 65mg, about 70mg, about 75mg, about 80mg, about 85mg, about 90mg, about 100mg, about 110mg, about 120mg, about 130mg, about 140mg, about 150mg, about 160mg, about 170mg, about 180mg, about 190mg, about 200mg, about 300mg, about 400mg, about 500mg, about 600mg, about 700mg, about 800mg, about 900mg, about 1000mg, about 1500mg, about 2000mg, about 2500mg or about 3000 mg of manganese oxide.

**In** some embodiments, the blood and/or serum uric acid levels in the mammalian subject are higher than 360µMol/L or 420µMol/L by at least about 15%, 20%, 25%, 30%, 35%, 40%, 45%, 50%, 55%, 60‰, 65%, 70%, 75%, 80%, 85%, 90%, 95% or 100%. **In** some embodiments, the blood and/or serum uric acid levels in the mammalian subject are higher than 360µMol/L by at least about 15%, 20%, 25%, 30%, 35%, 40%, 45%, 50%, 55%, 60‰, 65%, 70%, 75%, 80%, 85%, 90%, 95% or 100%. In some embodiments, blood and/or serum uric acid levels in the mammalian subject are higher than 420µMol/L by at least about 15%, 20%, 25%, 30%, 35%, 40%, 45%, 50%, 55%, 60‰, 65%, 70%, 75%, 80%, 85%, 90%, 95% or 100%.

In some embodiments, the blood and/or serum uric acid levels in the mammalian subject are reduced by at least about 10%, 15%, 20%, 25%, 30%, 35%, 40%, 45%, 50%, 55%, 60%, 65%, 70%, 75%, 80%, 85%, 90%, 95% or 100% 24 hours after administration of the pharmaceutical composition.

Other embodiments of this aspect may be referred to those detailed in the first aspect above.

In a ninth aspect, the present disclosure provides a pharmaceutical composition formulated as at least one solid dose unit for oral administration to a mammalian subject, wherein the solid dose unit comprises manganese oxide, and wherein the manganese oxide has a uric acid clearance capacity measured in a fasted state simulated intestinal fluid (FaSSIF) or fed state simulated intestinal fluid (FeSSIF) assay that is at least about 100 mg/g higher than the uric acid clearance capacity of activated charcoal measured under the same conditions.

In some embodiments, the uric acid clearance capacity of manganese oxide measured in a fasted state simulated intestinal fluid (FaSSIF) or fed state simulated intestinal fluid (FeSSIF) assay is at least about 200mg/g, 300mg/g, 400mg/g, 500mg/g, or 600mg/g higher than the uric acid clearance capacity of activated charcoal measured under the same conditions.

The fasted sate simulated intestinal fluid (FaSSIF) or fed sate simulated intestinal fluid (FeSSIF) assay can be performed using methods known to those skilled in the art. In some embodiments, the fasted sate simulated intestinal fluid (FaSSIF) or fed sate simulated intestinal fluid (FeSSIF) assay is performed in FaSSIF or FeSSIF with a concentration of uric acid of about $8.4 \pm 0.2$mg/dL, such as 8.2 mg/dL, 8.3 mg/dL, 8.4 mg/dL, 8.5 mg/dL or 8.6 mg/dL. In some embodiments, the fasted sate simulated intestinal fluid (FaSSIF) or fed sate simulated intestinal fluid (FeSSIF) assay is performed at a temperature of about 25°C to 37°C, such as a temperature of about 25°C to 26°C, 25°C to 27°C, 25°C to 28°C, 25°C to 29°C, 25°C to 30°C, 25°C to 31°C, 25°C to 32°C, 25°C to 33°C, 25°C to 34°C, 25°C to 35°C, 25°C to 36°C, 26°C to 37°C, 27°C to 37°C, 28°C to 37°C, 29°C to 37°C, 30°C to 37°C, 31°C to 37°C, 32°C to 37°C, 33°C to 37°C, 34°C to 37°C, 35°C to 37°C, or 36°C to 37°C. In some embodiments, the fasted state simulated intestinal fluid (FaSSIF) or simulated ingestion small intestine fluid (FeSSIF) assay is performed at a concentration of manganese oxide of about 0.1 g/L to 2 g/L, for example, about 0.1 g/L to 0.2 g/L, 0.1 g/L to 0.3 g/L, 0.1 g/L to 0.4 g/L, 0.1 g/L to 0.5 g/L, 0.1 g/L to 0.6 g/L, 0.1 g/L to 0.7 g/L, 0.1 g/L to 0.8 g/L, 0.1 g/L to 0.9 g/L, 0.1 g/L to 1.0 g/L, 0.1 g/L to 1.1 g/L, 0.1 g/L to 1.2 g/L, 0.1 g/L to 1.3 g/L, 0.1 g/L to 1.4 g/L, 0.1 g/L to 1.5 g/L, 0.1 g/L to 1.6 g/L, 0.1 g/L to 1.7 g/L, 0.1 g/L to 1.8 g/L, 0.1 g/L to 1.9 g/L, 0.2 g/L to 2 g/L, 0.3 g/L to 2 g/L, 0.4 g/L to 2 g/L, 0.5 g/L to 2 g/L, 0.6 g/L to 2 g/L, 0.7 g/L to 2 g/L, 0.8 g/L to 2 g/L, 0.9 g/L to 2 g/L, 1.0 g/L to 2 g/L, 1.1 g/L to 2 g/L, 1.2 g/L to 2 g/L, 1.3 g/L to 2 g/L, 1.4 g/L to 2 g/L, 1.5 g/L to 2 g/L, 1.6 g/L to 2 g/L, 1.7 g/L to 2 g/L, 1.8 g/L to 2 g/L or 1.9 g/L to 2.0 g/L. In some embodiments, the fasted sate simulated intestinal fluid (FaSSIF) or fed sate simulated intestinal fluid (FeSSIF) assay is performed under oscillatory conditions. The oscillation frequency can be, for example, $\geq$50 rpm, $\geq$100 rpm, $\geq$150 rpm, $\geq$200 rpm, $\geq$250 rpm, $\geq$300 rpm, or higher. In some embodiments, the fasted sate simulated intestinal fluid (FaSSIF) or fed sate simulated intestinal fluid (FeSSIF) assay is performed using fasted sate simulated intestinal fluid (FaSSIF) or fed sate simulated intestinal fluid (FeSSIF) having a concentration of uric acid of about $8.4 \pm 0.2$ mg/dL, at a temperature of about 25°C to 37°C, with a concentration of manganese oxide of about 0.1 g/L to 2 g/L, and under oscillatory conditions. In some embodiments, the duration of the fasted sate simulated intestinal fluid (FaSSIF) or fed sate simulated intestinal fluid (FeSSIF) assay is $\geq$5min, such as $\geq$10min, $\geq$15min, $\geq$20min, $\geq$25min, $\geq$30min, $\geq$35min, $\geq$40min, $\geq$45min, $\geq$50min, $\geq$55min, $\geq$60min, $\geq$90min, $\geq$120min, $\geq$180min, $\geq$240min, $\geq$300min, $\geq$360min, $\geq$420min, $\geq$480min, $\geq$520min or $\geq$600min.

Other embodiments of this aspect may be referred to those detailed in the first and eighth aspects above.

Without causing conflict, the features of the various embodiments described in the above aspects of the present disclosure may be combined to obtain one or more additional embodiments within the spirit and/or scope of the present disclosure.

**Examples**

The present disclosure is further described in detail below using examples. Obviously, the described examples are only a part of the invention disclosed in the present disclosure, rather than all of them. These examples are for illustrative purposes only and should not be construed as limiting the scope of protection of the present invention. Based on the disclosed examples, all equivalent or similar technical solutions obtained by those skilled in the art without paying any creative work shall fall within the scope of protection of the present invention.

**Example 1 Preparation of test substances**

This example describes the sources or preparation methods of various test substances used in subsequent assays (when not commercially available).

**1.1 Preparation of control substances**

In the examples of the present application, the "control substance" refers to a substance that does not belong to the category of manganese-containing compounds. Unless otherwise indicated, these materials are commercially available.

The following is a non-limiting list of commercially available control substances:

   (1) zeolite molecular sieves, including TS-1, NaY, 10X, ZSM-5, 4A, HY, 13X, zeolite, 5A, 3A and USY;
   (2) Prussian blue;
   (3) activated charcoal, i.e. medicinal activated charcoal AST-120 (or "AST120", "KREMEZIN®") and Medicinal Charcoal Capsules, and commercial activated charcoal with specific surface area of 600 m$^2$/g, 720 m$^2$/g, 1045 m$^2$/g, 1400 m$^2$/g, 1800 m$^2$/g and 3500 m$^2$/g, respectively;
   (4) ion exchange resins, including IRA900 (Amberlite IRA-900 (Cl) ion exchange resin, LOT: 10227274, CAS: 9050-97-9, Alfa Aesar), cholestyramine, sevelamer hydrochloride, CER-1 (Amberlyst 15(H) ion exchange resin, LOT: 5007R10N, CAS: 39389-20-3, Alfa Aesar), DOWEX (DOWEX (R) 1X8, LOT: 10224593, CAS: 12627-85-9, Alfa Aesar), NAER-1 (ion exchange resin, LOT: 10228055, CAS: 9017-79-2, Alfa Aesar), NAER-2 (macroporous ion exchange resin, LOT: 10229336, CAS:39339-85-0, Alfa Aesar);
   (5) metal organic frameworks (MOFs), including ZIF-8, UIO66 and UIO66NH2; and
   (6) montmorillonite.

In addition, some reference substances can be prepared by artificial synthesis. The preparation methods of different types of hydrotalcite (Note: the following "2-1", "3-1" and "4-1" refer to magnesium-aluminum ratio) used as control substances in the embodiments of the present application are listed below:

Preparation of 2-1 hydrotalcite

570 mg of magnesium chloride, 723 mg of aluminum chloride hexahydrate, and 1.26 g of urea were weighed and dissolved in 10 mL of deionized water. The solution obtained above was transferred to a hydrothermal reactor to react at 160 °C for 6 h. After natural cooling, the solid was collected by centrifugation at 1000 rpm, washed with water until neutral, and dried at 70°C for 24 h to obtain 2-1 hydrotalcite.

Preparation of 3-1 hydrotalcite

570 mg of magnesium chloride, 482 mg of aluminum chloride hexahydrate, and 1.12 g of urea were weighed and dissolved in 10 mL of deionized water. The solution obtained above was transferred to a hydrothermal reactor to react at 160 °C for 6 h. After natural cooling, the solid was collected by centrifugation at 1000 rpm, washed with water until neutral, and dried at 70°C for 24 h to obtain 3-1 hydrotalcite.

Preparation of 4-1 hydrotalcite

570 mg of magnesium chloride, 362 mg of aluminum chloride hexahydrate, and 1.048 g of urea were weighed and dissolved in 10 mL of deionized water. The solution obtained above was transferred to a hydrothermal reactor to react at 160 °C for 6 h. After natural cooling, the solid was collected by centrifugation at 1000 rpm, washed with water until neutral, and dried at 70°C for 24 h to obtain 4-1 hydrotalcite.

Preparation of CTAB (cetyltrimethyl ammonium bromide) hydrotalcite

82 mg of magnesium chloride, 104 mg of aluminum chloride hexahydrate, and 180 g of urea were weighed and dissolved in 15 mL of deionized water to prepare solution 1. 131 mg of CTAB was weighed and dissolve it in 9 mL of deionized water to prepare solution 2. Solutions 1 and 2 were mixed uniformly, transferred to a hydrothermal reactor to react at 160°C for 6 h. After natural cooling, the system was centrifuged at 1000 rpm, the resultant solid was separated, washed with water until neutral, and dried at 70°C for 24 h to obtain CTAB hydrotalcite.

**1.2 Preparation of insoluble manganese-containing compounds as test samples**

As test samples, the insoluble manganese-containing compounds used in the examples of the present application include commercially available insoluble manganese-containing compounds, insoluble manganese-containing compounds in the form of natural minerals, and insoluble manganese-containing compounds that can be prepared by artificial synthesis methods.

The following is a non-limiting list of commercially available insoluble manganese-containing compounds: $\beta M$ ($\beta$-form crystalline manganese oxide, LOT: A0431349, 99%, 10 mesh, Acros), $\gamma M$ ($\gamma$-form crystalline manganese oxide, LOT: G1804053, >90%, Aladdin) and $\lambda MLi$ (lithium-type $\lambda$-form crystalline manganese oxide, Spinel $LiMn_2O_4$).

The following is a non-limiting list of insoluble manganese-containing compounds in the form of natural minerals: romanechite, todorokite, manganite (composed primarily of $\gamma$-form crystalline manganese oxyhydroxide), groutite (composed primarily of $\alpha$-form crystalline manganese oxyhydroxide), feitknechtite (composed primarily of $\beta$-form crystalline manganese oxyhydroxide), hydrohausmannite (composed primarily of $\beta$-form crystalline manganese oxyhydroxide), lithiophorite, chalcophanite, hausmannite, bixbyite, pyrochroite, manganosite.

For the purpose of use in the embodiments of the present application, all raw materials of insoluble manganese-containing compounds in the form of natural minerals were ground and sieved to obtain test samples with a particle size ranging from 1 $\mu M$ to 1 mm.

In addition, some test samples need to be prepared through artificial synthesis methods. The following lists the preparation methods for different forms of insoluble manganese-containing compounds used as test samples in the embodiments of the present application. After preparation was completed, the polymorphic forms of the products obtained by these methods were identified by powder X-ray diffraction using a multifunctional X-ray diffractometer D8 ADVANCE Da Vinci. The test conditions of the powder X-ray diffraction method are as follows: the X-ray generator adopts a Cu target, the X-ray tube is a ceramic tube, the voltage is $\leq$40 kV, the current is $\leq$40 mA, the scanning range is 5° to 90°, the step size is 0.020435°, and the scanning speed is 4°/S.

Preparation of potassium-type $\delta$-form crystalline manganese oxide-1 ($\delta M1K$)

0.948 g of potassium permanganate was weighed, dissolved in an appropriate amount of deionized water, placed on a magnetic stirrer for stirring at room temperature to obtain a potassium permanganate solution. 0.169 g of manganese sulfate monohydrate was weighed, dissolved in an appropriate amount of deionized water, and added dropwise to the potassium permanganate solution (total water volume, 35 mL). After the dropwise addition was completed, the mixture was stirred at room temperature for 30 min to form a homogeneous solution. The solution was transferred to a hydrothermal reactor to react at 160°C for 12 h. After natural cooling, the solid was collected by filtration, washed several times with water, washed several times with ethanol, and dried at 60°C for 12 h to obtain $\delta M1K$.

The powder X ray diffraction pattern of the $\delta M1K$ exhibits characteristic X ray diffraction peaks at about the following $2\theta$ angles: 12.2, 24.9, 37.0, 56.4, and 65.8. This indicates that the characteristic X-ray diffraction peaks in the powder X-ray diffraction pattern of $\delta M1K$ are substantially consistent with the characteristic X-ray diffraction peaks in the powder X-ray diffraction pattern of manganese oxide in the form of birnessite in the $\delta$-form crystalline form.

Preparation of hydrogen-type $\delta$-form crystalline manganese oxide-1 ($\delta M1KH$)

$\delta M1K$ was weighed and placed in a beaker. Dilute nitric acid solution was added in a ratio of 1 g of $\delta M1K$ to 100 mL of 0.7 M dilute nitric acid solution. The beaker was heated and stirred in a water bath at 60°C for 90 min and then naturally cooled to room temperature. The system was filtered to obtain a solid, the solid was washed with water, and dried at 60°C in air for 12 h to obtain $\delta M1KH$.

The powder X ray diffraction pattern of the $\delta M1KH$ exhibits characteristic X ray diffraction peaks at about the following $2\theta$ angles: 12.5, 25.1, 36.8, 56.9 and 66.0. This indicates that the characteristic X-ray diffraction peaks in the powder X-ray diffraction pattern of $\delta M1KH$ are substantially consistent with the characteristic X-ray diffraction peaks in the powder X-ray diffraction pattern of manganese oxide in the form of birnessite in the $\delta$-form crystalline form.

Preparation of ammonium-type $\delta$-form crystalline manganese oxide-1 ($\delta M1KNH4$)

$\delta M1K$ was weighed and placed in a beaker. $\delta M1K$ was dispersed in 1 M ammonium chloride solution in a ratio of 1g/100 mL. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain $\delta M1KNH4$.

The powder X ray diffraction pattern of the $\delta M1KNH4$ exhibits characteristic X ray diffraction peaks at about the following $2\theta$ angles: 12.6, 24.9, 36.9, 56.9 and 66.1. This indicates that the characteristic X-ray diffraction peaks in the powder X-ray diffraction pattern of $\delta M1KNH4$ are substantially consistent with the characteristic X-ray diffraction peaks in the powder X-ray diffraction pattern of manganese oxide in the form of birnessite in the $\delta$-form crystalline form.

Preparation of calcium-type δ-form crystalline manganese oxide-1 (δM1KCa)

δM1K was weighed and placed in a beaker. δM1K was dispersed in 1 M calcium chloride solution in a ratio of 1g/100 mL. The beaker was stirred at room temperature for 4 h, then placed in a hydrothermal reactor 155°C to allow the system to react for 24 h, and then naturally cooled to room temperature. The system was filtered to obtain a solid, the solid was washed with water, and dried at 60°C under vacuum overnight to obtain δM1KCa.

The powder X ray diffraction pattern of the δM1KCa exhibits characteristic X ray diffraction peaks at about the following 2θ angles: 8.9, 12.6, 18.5 and 37.0. This indicates that the characteristic X-ray diffraction peaks in the powder X-ray diffraction pattern of δM1KCa are substantially consistent with the characteristic X-ray diffraction peaks in the powder X-ray diffraction pattern of manganese oxide in the form of birnessite and buserite in the δ-form crystalline form.

Preparation of magnesium-type δ-form crystalline manganese oxide-1 (δM1KMg)

δM1K was weighed and placed in a beaker. δM1K was dispersed in 1 M magnesium chloride solution in a ratio of 1g/100 mL. The beaker was stirred at room temperature for 4 h, then placed in a hydrothermal reactor 155°C to allow the system to react for 24 h, and then naturally cooled to room temperature. The system was filtered to obtain a solid, the solid was washed with water, and dried at 60°C under vacuum overnight to obtain δM1KMg.

The powder X ray diffraction pattern of the δM1KMg exhibits characteristic X ray diffraction peaks at about the following 2θ angles: 9.0, 18.5 and 28.1. This indicates that the characteristic X-ray diffraction peaks in the powder X-ray diffraction pattern of δM1KMg are substantially consistent with the characteristic X-ray diffraction peaks in the powder X-ray diffraction pattern of manganese oxide in the form of buserite in the δ-form crystalline form.

Preparation of iron-type δ-form crystalline manganese oxide-1 (δM1KFe3)

δM1K was weighed and placed in a beaker. δM1K was dispersed in 1 M ferric chloride solution in a ratio of 1g/100 mL. The beaker was stirred at room temperature for 4 h, then placed in a hydrothermal reactor 155°C to allow the system to react for 24 h, and then naturally cooled to room temperature. The system was filtered to obtain a solid, the solid was washed with water, and dried at 60°C under vacuum overnight to obtain δM1KFe3.

The powder X ray diffraction pattern of the δM1KFe3 exhibits characteristic X ray diffraction peaks at about the following 2θ angles: 12.3, 25.0, 36.8, 56.7 and 66.2. This indicates that the characteristic X-ray diffraction peaks in the powder X-ray diffraction pattern of δM1KFe3 are substantially consistent with the characteristic X-ray diffraction peaks in the powder X-ray diffraction pattern of manganese oxide in the form of birnessite in the δ-form crystalline form.

Preparation of ferrous-type δ-form crystalline manganese oxide-1 (δM1KFe2)

δM1K was weighed and placed in a beaker. δM1K was dispersed in 1 M ferrous chloride solution in a ratio of 1g/100 mL. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM1KFe2.

The powder X ray diffraction pattern of the δM1KFe2 exhibits characteristic X ray diffraction peaks at about the following 2θ angles: 12.6, 24.9, 36.8, 56.5 and 66.7. This indicates that the characteristic X-ray diffraction peaks in the powder X-ray diffraction pattern of δM1KFe2 are substantially consistent with the characteristic X-ray diffraction peaks in the powder X-ray diffraction pattern of manganese oxide in the form of birnessite in the δ-form crystalline form.

Preparation of zinc-type δ-form crystalline manganese oxide-1 (δM1KZn)

δM1K was weighed and placed in a beaker. δM1K was dispersed in 1 M zinc chloride solution in a ratio of 1g/100 mL. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM1KZn.

The powder X ray diffraction pattern of the δM1KZn exhibits characteristic X ray diffraction peaks at about the following 2θ angles: 12.5, 25.2, 36.9, 56.7 and 66.9. This indicates that the characteristic X-ray diffraction peaks in the powder X-ray diffraction pattern of δM1KZn are substantially consistent with the characteristic X-ray diffraction peaks in the powder X-ray diffraction pattern of manganese oxide in the form of birnessite in the δ-form crystalline form.

Preparation of lanthanum-type δ-form crystalline manganese oxide-1 (δM1KLa)

δM1K was weighed and placed in a beaker. δM1K was dispersed in 1 M lanthanum chloride solution in a ratio of 1g/100 mL. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM1KLa.

The powder X ray diffraction pattern of the δM1KLa exhibits characteristic X ray diffraction peaks at about the following 2θ angles: 9.2, 12.5, 18.3, 25.0 and 37.0. This indicates that the characteristic X-ray diffraction peaks in the powder X-ray diffraction pattern of δM1KLa are substantially consistent with the characteristic X-ray diffraction peaks in the powder X-ray diffraction pattern of manganese oxide in the form of birnessite and buserite in the δ-form crystalline form.

Preparation of bismuth-type δ-form crystalline manganese oxide-1 (δM1KBi)

δM1K was weighed and placed in a beaker. A solution of bismuth nitrate in 0.7 M dilute nitric acid was added in a ratio of 1 g of δM1K/100 mL of solution of bismuth nitrate in 0.7 M dilute nitric acid. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM1KBi.

The powder X ray diffraction pattern of the δM1KBi exhibits characteristic X ray diffraction peaks at about the following 2θ angles: 12.6, 25.2, 37.1, 56.7 and 67.0. This indicates that the characteristic X-ray diffraction peaks in the powder X-ray diffraction pattern of δM1KBi are substantially consistent with the characteristic X-ray diffraction peaks in the powder X-ray diffraction pattern of manganese oxide in the form of birnessite in the δ-form crystalline form.

Preparation of lithium-type δ-form crystalline manganese oxide-1 (δM1KLi)

δM1K was weighed and placed in a beaker. δM1K was dispersed in 1 M lithium chloride solution in a ratio of 1g/100 mL. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM1KLi.

The powder X ray diffraction pattern of the δM1KLi exhibits characteristic X ray diffraction peaks at about the following 2θ angles: 12.6, 25.0, 36.9, 56.5 and 66.8. This indicates that the characteristic X-ray diffraction peaks in the powder X-ray diffraction pattern of δM1KLi are substantially consistent with the characteristic X-ray diffraction peaks in the powder X-ray diffraction pattern of manganese oxide in the form of birnessite in the δ-form crystalline form.

Preparation of silver-type δ-form crystalline manganese oxide-1 (δM1KAg)

δM1K was weighed and placed in a beaker. δM1K was dispersed in 1 M silver nitrate solution in a ratio of 1g/100 mL. The beaker was stirred in the dark at room temperature for 4 h, and then the solid was filtered out, washed with water, and dried under vacuum at room temperature overnight to obtain δM1KAg.

The powder X ray diffraction pattern of the δM1KAg exhibits characteristic X ray diffraction peaks at about the following 2θ angles: 12.5, 24.9, 36.8, 56.7 and 66.9. This indicates that the characteristic X-ray diffraction peaks in the powder X-ray diffraction pattern of δM1KAg are substantially consistent with the characteristic X-ray diffraction peaks in the powder X-ray diffraction pattern of manganese oxide in the form of birnessite in the δ-form crystalline form.

Preparation of potassium-type δ-form crystalline manganese oxide-2 (δM2K) and hydrogen-type δ-form crystalline manganese oxide-2.1 (δM2KH)

9.65 g of potassium hydroxide was weighed and dissolved in 180 mL of deionized water. 20 mL of 30% hydrogen peroxide solution was measured, added to the above solution and mixed. Then the mixture was added dropwise to 100 mL of aqueous manganese nitrate solution (containing 0.03 M manganese) with vigorous stirring. After the dropwise addition was completed, the system was further stirred at room temperature for aging for 1 h and then let stand for 10 min. The supernatant was decanted, and the lower solid was centrifuged, washed with water and ethanol, and dried overnight under vacuum at 80°C to obtain δM2K. The δM2K obtained above was taken and put into a beaker. Dilute nitric acid solution was added in a ratio of 1 g of δM2K/100 mL of 0.7 M dilute nitric acid solution. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM2KH.

The powder X ray diffraction pattern of the δM2K exhibits characteristic X ray diffraction peaks at about the following 2θ angles: 12.4, 25.0, 36.9, 56.0 and 66.1. This indicates that the characteristic X-ray diffraction peaks in the powder X-ray diffraction pattern of δM2K are substantially consistent with the characteristic X-ray diffraction peaks in the powder X-ray diffraction pattern of manganese oxide in the form of birnessite in the δ-form crystalline form.

The powder X ray diffraction pattern of the δM2KH exhibits characteristic X ray diffraction peaks at about the following 2θ angles: 12.6, 24.8, 36.5, 55.7 and 66.3. This indicates that the characteristic X-ray diffraction peaks in the powder X-ray diffraction pattern of δM2KH are substantially consistent with the characteristic X-ray diffraction peaks in the powder X-ray diffraction pattern of manganese oxide in the form of birnessite in the δ-form crystalline form.

Preparation of sodium-type δ-form crystalline manganese oxide-2 (δM2Na) and hydrogen-type δ-form crystalline manganese oxide-2.2 (δM2NaH)

3.44 g of sodium hydroxide was weighed and dissolved in 90 mL of deionized water. 10 mL of 30% hydrogen peroxide solution was measured, added to the above solution and mixed. Then the mixture was added dropwise to 50 mL of aqueous manganese nitrate solution (containing 0.03 M manganese) with vigorous stirring. After the dropwise addition was completed, the mixture was further stirred at room temperature for aging for 1 h and then allowed to stand for 10 min. The supernatant was decanted and the lower solid was centrifuged, washed with water and dried under vacuum at room temperature overnight to obtain δM2Na. The δM2Na obtained above was taken and put into a beaker. Dilute nitric acid solution was added in a ratio of 1 g of δM2Na/100 mL of 0.7 M dilute nitric acid solution. The beaker was heated and stirred in a water bath at 60°C for 1.5 h and then naturally cooled to room temperature. The system was filtered to obtain a solid, the solid was washed with water, and dried at 60°C under vacuum overnight to obtain δM2NaH.

The powder X ray diffraction pattern of the δM2Na exhibits characteristic X ray diffraction peaks at about the following 2θ angles: 12.7, 37.1, 57.2 and 66.3. This indicates that the characteristic X-ray diffraction peaks in the powder X-ray diffraction pattern of δM2Na are substantially consistent with the characteristic X-ray diffraction peaks in the powder X-ray diffraction pattern of manganese oxide in the form of birnessite in the δ-form crystalline form.

The powder X ray diffraction pattern of the δM2NaH exhibits characteristic X ray diffraction peaks at about the following 2θ angles: 12.6, 37.5, 57.0 and 66.2. This indicates that the characteristic X-ray diffraction peaks in the powder X-ray diffraction pattern of δM2NaH are substantially consistent with the characteristic X-ray diffraction peaks in the powder X-ray diffraction pattern of manganese oxide in the form of birnessite in the δ-form crystalline form.

Preparation of potassium-type δ-form crystalline manganese oxide-3 (δM3K) and hydrogen-type δ-form crystalline manganese oxide-3 (δM3KH)

2 g of potassium permanganate was weighed and dissolved in 500 mL of deionized water. Under vigorous stirring, 10 mL of anhydrous ethanol was added dropwise and the system was stirred at room temperature to react for 6 h. After standing for 30 min, the upper turbid solution was decanted, and the lower solid was centrifuged, washed with water and ethanol, and dried overnight under vacuum at 80°C to obtain δM3K. The δM3K obtained above was taken and put into a beaker. Dilute nitric acid solution was added in a ratio of 1 g of δM3K/100 mL of 0.7 M dilute nitric acid solution. The beaker was stirred at room temperature for 4 h, then heated in a 60°C water bath for 2 h, and then naturally cooled to room temperature. The system was filtered to obtain a solid, the solid was washed with water, and dried at 60°C under vacuum overnight to obtain δM3KH.

The powder X ray diffraction pattern of the δM3K exhibits characteristic X ray diffraction peaks at about the following 2θ angles: 12.7, 37.2, 56.9 and 66.4. This indicates that the characteristic X-ray diffraction peaks in the powder X-ray diffraction pattern of δM3K are substantially consistent with the characteristic X-ray diffraction peaks in the powder X-ray diffraction pattern of manganese oxide in the form of birnessite in the δ-form crystalline form.

The powder X ray diffraction pattern of the δM3KH exhibits characteristic X ray diffraction peaks at about the following 2θ angles: 12.8, 37.3, 56.7 and 66.8. This indicates that the characteristic X-ray diffraction peaks in the powder X-ray diffraction pattern of δM3KH are substantially consistent with the characteristic X-ray diffraction peaks in the powder X-ray diffraction pattern of manganese oxide in the form of birnessite in the δ-form crystalline form.

Preparation of potassium-type δ-form crystalline manganese oxide-4 (δM4K)

0.75 g of potassium permanganate and 0.14 g of manganese sulfate monohydrate were weighed, dissolved in 40 mL of deionized water, placed on a magnetic stirrer and stirred at room temperature for about 30 min to obtain a homogeneous solution. The solution was transferred to a 100 mL hydrothermal reactor to react at 140°C for 24 h. After natural cooling, the solid was collected by filtration using a sand core funnel, washed several times with deionized water, and dried using a freeze dryer for about 12 h to obtain δM4K.

The powder X ray diffraction pattern of the δM4K exhibits characteristic X ray diffraction peaks at about the following 2θ angles: 12.5, 24.5, 37.1 and 66.0. This indicates that the characteristic X-ray diffraction peaks in the powder X-ray diffraction pattern of δM1K are substantially consistent with the characteristic X-ray diffraction peaks in the powder X-ray diffraction pattern of manganese oxide in the form of birnessite in the δ-form crystalline form.

Preparation of potassium-type $\alpha$-form crystalline manganese oxide-1 ($\alpha$M1K) and hydrogen-type $\alpha$-form crystalline manganese oxide-1 ($\alpha$M1KH)

1.264 g of potassium permanganate was weighed and dissolved in 30 mL of deionized water. 0.507 g of manganese sulfate monohydrate was weighed and dissolved in 10 mL of deionized water. The manganese sulfate solution was added dropwise to a vigorously stirred potassium permanganate solution. After the dropwise addition was completed, the mixture was stirred at room temperature for 30 min to form a homogeneous solution. The solution was transferred to a hydrothermal reactor to react at 160°C for 12 h. After natural cooling, the solid was collected by filtration, washed several times with water, washed several times with ethanol, and dried under vacuum at 80°C overnight to obtain $\alpha$M1K. The $\alpha$M1K obtained above was weighed and put into a beaker. Dilute nitric acid solution was added in a ratio of 1 g of $\alpha$M1K/100 mL of 0.7 M dilute nitric acid solution. The beaker was heated and stirred in a water bath at 60°C for 2 h and then naturally cooled to room temperature. The system was filtered to obtain a solid, the solid was washed with water, and dried at 60°C under vacuum for 24 h to obtain $\alpha$M1KH.

The powder X ray diffraction pattern of the $\alpha$M1K exhibits characteristic X ray diffraction peaks at about the following $2\theta$ angles: 12.8, 18.1, 25.5, 28.7, 39.0, 42.0, 49.9, 56.4, 60.2, 65.1, 70.0 and 72.5. This indicates that the characteristic X-ray diffraction peaks in the powder X-ray diffraction pattern of $\alpha$M1K are substantially consistent with the characteristic X-ray diffraction peaks in the powder X-ray diffraction pattern of manganese oxide in the $\alpha$-form crystalline form.

The powder X ray diffraction pattern of the $\alpha$M1KH exhibits characteristic X ray diffraction peaks at about the following $2\theta$ angles: 12.8, 18.1, 25.7, 28.8, 36.7, 39.0, 41.9, 49.8, 56.4, 60.1, 65.2, 70.1 and 72.7. This indicates that the characteristic X-ray diffraction peaks in the powder X-ray diffraction pattern of $\alpha$M1KH are substantially consistent with the characteristic X-ray diffraction peaks in the powder X-ray diffraction pattern of manganese oxide in the $\alpha$-form crystalline form.

Preparation of potassium-type $\alpha$-form crystalline manganese oxide-2 ($\alpha$M2K) and hydrogen-type $\alpha$-form crystalline manganese oxide-2 ($\alpha$M2KH)

24 mmol of potassium permanganate was weighed and dissolved in 60 mL of deionized water. 8 mmol of manganese sulfate monohydrate was weighed and dissolved in 60 mL of deionized water. The manganese sulfate solution was added dropwise to a vigorously stirred potassium permanganate solution. After the dropwise addition was completed, the mixture was stirred at room temperature for 30 min to form a homogeneous solution. The solution was transferred to a hydrothermal reactor to react at 120°C for 12 h. After natural cooling, the solid was collected by filtration, washed several times with water, washed several times with ethanol, and dried under vacuum at 80°C overnight to obtain $\alpha$M2K. The $\alpha$M2K obtained above was weighed and put into a beaker. Dilute nitric acid solution was added in a ratio of 1 g of $\alpha$M2K/100 mL of 0.7 M dilute nitric acid solution. The beaker was heated and stirred in a water bath at 60°C for 1.5 h and then naturally cooled to room temperature. The system was filtered to obtain a solid, the solid was washed with water, and dried at 60°C under vacuum overnight to obtain $\alpha$M2KH.

The powder X ray diffraction pattern of the $\alpha$M2K exhibits characteristic X ray diffraction peaks at about the following $2\theta$ angles: 12.7, 18.0, 25.4, 28.7, 39.1, 42.0, 50.0, 56.5, 60.1, 65.2, 70.1 and 72.5. This indicates that the characteristic X-ray diffraction peaks in the powder X-ray diffraction pattern of $\alpha$M2K are substantially consistent with the characteristic X-ray diffraction peaks in the powder X-ray diffraction pattern of manganese oxide in the $\alpha$-form crystalline form.

The powder X ray diffraction pattern of the $\alpha$M2KH exhibits characteristic X ray diffraction peaks at about the following $2\theta$ angles: 12.7, 18.0, 25.5, 28.7, 39.1, 42.0, 50.0, 56.5, 60.1, 65.2, 70.1 and 72.5. This indicates that the characteristic X-ray diffraction peaks in the powder X-ray diffraction pattern of $\alpha$M2KH are substantially consistent with the characteristic X-ray diffraction peaks in the powder X-ray diffraction pattern of manganese oxide in the $\alpha$-form crystalline form.

Preparation of potassium-type $\alpha$-form crystalline manganese oxide-3 ($\alpha$M3K) and hydrogen-type $\alpha$-form crystalline manganese oxide-3 ($\alpha$M3KH)

10 mmol of potassium permanganate was weighed and dissolved in 30 mL of 1.5 M sulfuric acid solution. 30 mmol of manganese sulfate monohydrate was weighed and dissolved in 30 mL of 1.5M sulfuric acid solution. The manganese sulfate solution was added dropwise to a vigorously stirred potassium permanganate solution. After the dropwise addition was completed, the mixture was stirred at room temperature for 30 min to form a homogeneous solution. The solution was transferred to a hydrothermal reactor to react at 120°C for 12 h. After natural cooling, the solid was collected by filtration, washed several times with water, washed several times with ethanol, and dried under vacuum at 80°C overnight to obtain $\alpha$M3K. The $\alpha$M3K obtained above was weighed and put into a beaker. Dilute nitric acid solution was added in a ratio of 1 g of αM3K/100 mL of 0.7M dilute nitric acid solution. The beaker was heated and stirred in a water bath at 60°C for 1.5 h and then naturally cooled to room temperature. The system was filtered to obtain a solid, the solid was washed with water, and dried at 60°C under vacuum overnight to obtain αM3KH.

The powder X ray diffraction pattern of the αM3K exhibits characteristic X ray diffraction peaks at about the following 2θ angles: 12.7, 18.0, 25.4, 28.7, 39.2, 42.0, 50.0, 56.4, 60.1, 65.2, 70.1 and 72.5. This indicates that the characteristic X-ray diffraction peaks in the powder X-ray diffraction pattern of αM3K are substantially consistent with the characteristic X-ray diffraction peaks in the powder X-ray diffraction pattern of manganese oxide in the α-form crystalline form.

The powder X ray diffraction pattern of the αM3KH exhibits characteristic X ray diffraction peaks at about the following 2θ angles: 12.7, 18.1, 25.4, 28.8, 39.1, 42.0, 50.0, 56.5, 60.0, 65.2, 70.1 and 72.5. This indicates that the characteristic X-ray diffraction peaks in the powder X-ray diffraction pattern of αM3KH are substantially consistent with the characteristic X-ray diffraction peaks in the powder X-ray diffraction pattern of manganese oxide in the α-form crystalline form.

Preparation of potassium-type β-form crystalline manganese oxide (βMK) and hydrogen-type β-form crystalline manganese oxide-1 (βMKH)

1 mmol of potassium permanganate was weighed and dissolved in an appropriate amount of deionized water. 1.5 mmol of manganese sulfate monohydrate was weighed and dissolved in appropriate amount of deionized water. The manganese sulfate solution was added dropwise to a vigorously stirred potassium permanganate solution. After the dropwise addition was completed, the mixture was stirred at room temperature for 30 min to form a homogeneous solution (total volume 40 mL). The solution was transferred to a hydrothermal reactor to react at 160°C for 12 h. After natural cooling, the solid was collected by filtration, washed several times with water, washed several times with ethanol, and dried under vacuum at 80°C overnight to obtain βMK. The βMK obtained above was weighed and put into a beaker. Dilute nitric acid solution was added in a ratio of 1 g of βMK/100 mL of 0.7 M dilute nitric acid solution. The beaker was heated and stirred in a water bath at 60°C for 2 h and then naturally cooled to room temperature. The system was filtered to obtain a solid, the solid was washed with water, and dried at 60°C under vacuum for 24 h to obtain βMKH.

The powder X ray diffraction pattern of the βMK exhibits characteristic X ray diffraction peaks at about the following 2θ angles: 28.7, 37.4, 41.1, 42.9, 46.2, 56.8, 59.5, 65.0, 67.5 and 72.6. This indicates that the characteristic X-ray diffraction peaks in the powder X-ray diffraction pattern of βMK are substantially consistent with the characteristic X-ray diffraction peaks in the powder X-ray diffraction pattern of manganese oxide in the β-form crystalline form.

The powder X ray diffraction pattern of the βMKH exhibits characteristic X ray diffraction peaks at about the following 2θ angles: 28.7, 37.4, 41.1, 42.9, 46.2, 56.8, 59.5, 65.0, 67.5 and 72.6. This indicates that the characteristic X-ray diffraction peaks in the powder X-ray diffraction pattern of βMKH are substantially consistent with the characteristic X-ray diffraction peaks in the powder X-ray diffraction pattern of manganese oxide in the β-form crystalline form.

Preparation of hydrogen-type β-form crystalline manganese oxide-2 (βMH)

βM manganese oxide was weighed and placed in a beaker. Dilute nitric acid solution was added in a ratio of 1 g of βM manganese oxide/100 mL of 0.7 M dilute nitric acid solution. The beaker was heated and stirred in a water bath at 60°C for 2 h and then naturally cooled to room temperature. The system was filtered to obtain a solid, the solid was washed with water, and dried at 60°C under vacuum for 12 h to obtain βMH.

The powder X ray diffraction pattern of the βMH exhibits characteristic X ray diffraction peaks at about the following 2θ angles: 28.7, 37.4, 41.1, 42.9, 46.2, 56.8, 59.5, 65.0, 67.5 and 72.6. This indicates that the characteristic X-ray diffraction peaks in the powder X-ray diffraction pattern of βMH are substantially consistent with the characteristic X-ray diffraction peaks in the powder X-ray diffraction pattern of manganese oxide in the β-form crystalline form.

Preparation of ammonium-type γ-form crystalline manganese oxide (γMN) and hydrogen-type γ-form crystalline manganese oxide-1 (γMNH)

1.69 g of manganese sulfate monohydrate and 2.28 g of ammonium persulfate were weighed and dissolved in 40 mL of deionized water. A colorless and transparent homogeneous solution was formed after stirring for 30 min at room temperature. The solution was transferred to a hydrothermal reactor to react at 80°C for 24 h. After natural cooling, the solid was collected by filtration, washed several times with water, washed several times with ethanol, and dried under vacuum at 80°C overnight to obtain γMN. The γMN obtained above was weighed and put into a beaker. Dilute nitric acid solution was added in a ratio of 1 g of γMN/100 mL of 0.7 M dilute nitric acid solution. The beaker was heated and stirred in a water bath at 60°C for 2 h and then naturally cooled to room temperature. The system was filtered to obtain a solid, the solid was washed with water, and dried at 60°C under vacuum for 24 h to obtain γMNH.

The powder X ray diffraction pattern of the γMN exhibits characteristic X ray diffraction peaks at about the following 2θ angles: 23.0, 26.7, 34.0, 37.1, 42.5, 50.0, 56.3, 65.5 and 69.4. This indicates that the characteristic X-ray diffraction peaks in the powder X-ray diffraction pattern of γMN are substantially consistent with the characteristic X-ray diffraction peaks in the powder X-ray diffraction pattern of manganese oxide in the γ-form crystalline form.

The powder X ray diffraction pattern of the γMNH exhibits characteristic X ray diffraction peaks at about the following 2θ angles: 23.0, 26.7, 34.0, 37.3, 42.5, 56.3 and 65.5. This indicates that the characteristic X-ray diffraction peaks in the powder X-ray diffraction pattern of γMNH are substantially consistent with the characteristic X-ray diffraction peaks in the powder X-ray diffraction pattern of manganese oxide in the γ-form crystalline form.

Preparation of hydrogen-type γ-form crystalline manganese oxide-2 (γMH)

γM manganese oxide was weighed and placed in a beaker. Dilute nitric acid solution was added in a ratio of 1 g of γM manganese oxide/100 mL of 0.7M dilute nitric acid solution. The beaker was heated and stirred in a water bath at 60°C for 2 h and then naturally cooled to room temperature. The system was filtered to obtain a solid, the solid was washed with water, and dried at 60°C under vacuum for 12 h to obtain γMH.

The powder X ray diffraction pattern of the γMH exhibits characteristic X ray diffraction peaks at about the following 2θ angles: 23.0, 26.7, 34.0, 37.1, 42.5, 50.0, 56.4, 65.5 and 69.6. This indicates that the characteristic X-ray diffraction peaks in the powder X-ray diffraction pattern of γMH are substantially consistent with the characteristic X-ray diffraction peaks in the powder X-ray diffraction pattern of manganese oxide in the γ-form crystalline form.

Preparation of hydrogen-type λ-form crystalline manganese oxide (λMLiH)

2 g of λMLi (lithium-type λ-form crystalline manganese oxide, Spinel $LiMn_2O_4$) was weighed, dispersed in 0.5 M aqueous sulfuric acid solution, and stirred at room temperature for 1 h. The resulting mixture was filtered, solids were collected, washed 5 times with deionized water, dried overnight in a drying oven at 80°C to obtain λMLiH.

The powder X ray diffraction pattern of the λMLiH exhibits characteristic X ray diffraction peaks at about the following 2θ angles: 19.0, 37.1, 45.1, 49.5, 59.7, 65.6 and 69.1. This indicates that the characteristic X-ray diffraction peaks in the powder X-ray diffraction pattern of λMLiH are substantially consistent with the characteristic X-ray diffraction peaks in the powder X-ray diffraction pattern of manganese oxide in the λ-form crystalline form.

Preparation of potassium-type ε-form crystalline manganese oxide (εMK) and hydrogen-type ε-form crystalline manganese oxide (εMKH)

4 mmol of potassium permanganate was weighed and dissolved in 20mL of deionized water to prepare 0.2 M potassium permanganate solution. In addition, 6 mmol of manganese sulfate monohydrate was weighed and dissolved in 20 mL of deionized water to prepare 0.3 M manganese sulfate solution. The manganese sulfate solution and potassium permanganate solution were mixed, stirred at room temperature for 3 h, and solids were collected through filtration. The resulting solids were washed with water for 5 times and dried in an oven at 60°C for 12 h to give εMK. The εMK obtained above was weighed and put into a beaker. Dilute nitric acid solution was added in a ratio of 1 g of εMK to 100 mL of 0.7 M dilute nitric acid solution. The beaker was heated and stirred in a water bath at 60°C for 2 h, and then naturally cooled to room temperature. Solids were obtained through filtration, washed with water for 5 times and dried in an oven at 60°C for 12 h to give εMKH.

The powder X ray diffraction pattern of the εMK exhibits characteristic X ray diffraction peaks at about the following 2θ angles: 21.0, 37.1, 42.5, 56.0 and 66.8. This indicates that the characteristic X-ray diffraction peaks in the powder X-ray diffraction pattern of εMK are substantially consistent with the characteristic X-ray diffraction peaks in the powder X-ray diffraction pattern of manganese oxide in the ε-form crystalline form.

The powder X ray diffraction pattern of the εMKH exhibits characteristic X ray diffraction peaks at about the following 2θ angles: 21.0, 37.1, 42.5, 56.0 and 66.8. This indicates that the characteristic X-ray diffraction peaks in the powder X-ray diffraction pattern of εMKH are substantially consistent with the characteristic X-ray diffraction peaks in the powder X-ray diffraction pattern of manganese oxide in the ε-form crystalline form.

Preparation of potassium-type amorphous manganese oxide (AMK) and hydrogen-type amorphous manganese oxide (AMKH)

1.58 g of potassium permanganate was weighed and dissolved in 60 mL of deionized water to prepare potassium permanganate solution. In addition, 2.28 g of oxalic acid was weigh and dissolved in 100 mL of deionized water to prepare oxalic acid solution. The potassium permanganate solution was added dropwise to the oxalic acid solution under magnetic stirring. After the dropwise addition was completed, the mixture was stirred at room temperature for 2 h and solids were collected by filtration. The resulting solids were washed with water 5 times, ethanol 3 times, and dried overnight in an oven at 60°C to obtain AMK. The AMK obtained above was weighed and put into a beaker. Dilute nitric acid solution was added in a ratio of 1 g of AMK/100 mL of 0.7 M dilute nitric acid solution. The beaker was heated and stirred in a water bath at 60°C for 2 h, and then naturally cooled to room temperature. Solids were obtained through filtration, washed with water for 5 times and dried in an oven at 60°C for 12 h to give AMKH.

The powder X ray diffraction patterns of the AMK and AMKH do not show distinct characteristic X ray diffraction peaks. This indicates that the powder X-ray diffraction patterns of AMK and AMKH are basically consistent with the powder X-ray diffraction pattern of amorphous manganese oxide.

Preparation of hydrogen-type R-form crystalline manganese oxide (RMH)

3 g of λMLi (lithium-type λ-form crystalline manganese oxide, Spinel $LiMn_2O_4$) was weighed, dispersed in 15 mL of 2.5 M aqueous sulfuric acid solution, and stirred at 80°C for 2.5 h. The precipitate was obtained by centrifugation, cleaned 4 times with ultrapure water, and dried overnight at 80°C under vacuum to obtain RMH.

The powder X ray diffraction pattern of the RMH exhibits characteristic X ray diffraction peaks at about the following 2θ angles: 22.0, 35.0, 36.9, 38.5, 41.2, 46.8 and 54.0. This indicates that the characteristic X-ray diffraction peaks in the powder X-ray diffraction pattern of RMH are substantially consistent with the characteristic X-ray diffraction peaks in the powder X-ray diffraction pattern of manganese oxide in the R-form crystalline form.

**Example 2 In vitro performance evaluation**

In this example, the clearance of uric acid by the test substances (i.e. a uric acid adsorbents) was tested under different in vitro simulated conditions to compare the performance of insoluble manganese-containing compounds according to the disclosure with control substances.

The composition of the body's gastrointestinal fluid is very complex and varies widely under different conditions (for example, in different parts of the stomach, small intestine, and large intestine), before and after meals (fasted, fed). Therefore, it is necessary to choose different simulated gastrointestinal fluids to simulate the gastrointestinal environment under different conditions. In this example, the uric acid clearance capacity of each test substance in simulated intestinal fluid (SIF), fasted state simulated intestinal fluid (FaSSIF), fed state simulated intestinal fluid (FeSSIF), and food enzymatic hydrolysate was determined, respectively. SIF only simulates the common pH and main inorganic salt components of intestinal fluid, FaSSIF and FeSSIF simulate the small intestinal fluid components (including inorganic and organic components) before and after meals, while the food enzymatic hydrolysate better simulates gastrointestinal chyme after taking different types of food. The complexity of the components of the above simulated liquids increases, and their proximity to the real human gastrointestinal environment increases accordingly. The higher the complexity of the components of the simulated liquid, the greater the clearance effect of the substance to be tested on uric acid by other components interference, so that the clearance effect becomes weaker.

**2.1 Comparison between control substances and test substances in terms of uric acid clearance capacity and clearance rate as measured in SIF assay**

The experimental method is as follows: 500 μM solution of uric acid in SIF was freshly formulated (concentration 8.4 mg/dL) with a pH of 6.8 and containing about 50 mM phosphate. 8 mg of each of the adsorbents was weighed, 4 mL of solution of uric acid in SIF (adsorbent concentration: 2 g/L) was added. The mixture was placed on a shaker and oscillated at 200 rpm for 4 h at 25°C. Hereafter, 1 mL of each sample was pipetted, centrifuged at 10000 rpm for 5 min, the supernatant was taken and diluted 10 times (90 μL of SIF+10 μL of supernatant), placed on a 96-well plate, and the absorbance was measured at 291 nm with a microplate reader. Standard curves were made freshly, including 0, 12.5, 25, 50, 100, and 500 μM point values. The uric acid concentration C (μM) in the supernatant was calculated according to the standard curve, and the uric acid clearance capacity Q (mg/g) was calculated according to formula 1 and the uric acid clearance rate (%) was calculated according to formula 2.

$$\text{Formula 1: } Q = 0.168 * (500\text{-}C) * 4/8$$

$$\text{Formula 2: Uric acid clearance rate} = (500\text{-}C) / 500 * 100\%$$

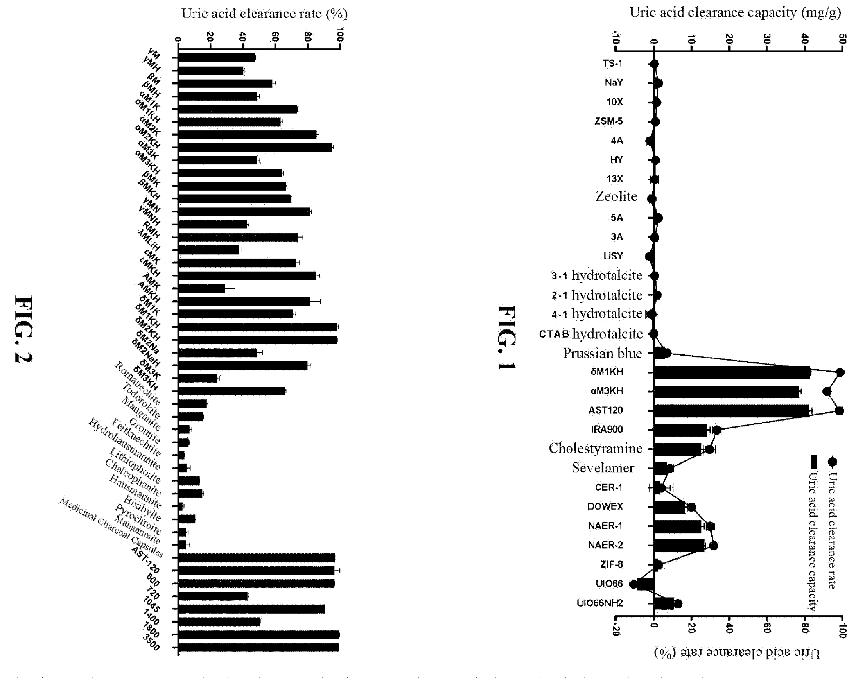

The result is shown in Fig. 1. Fig. 1 shows that among various types of uric acid adsorbents, manganese oxide (δM1KH and αM3KH) and activated charcoal (AST-120) have the best adsorption effect on uric acid under the conditions of SIF assay.

**2.2 Comparison between different types of insoluble manganese-containing compounds and activated charcoal adsorbents in terms of uric acid clearance rate as measured in SIF** assay

The experimental method is as follows: 500 $\mu$M solution of uric acid in SIF was freshly formulated (concentration 8.4 mg/dL) with a pH of 6.8 and containing about 50 mM phosphate. Different types of insoluble manganese-containing compound adsorbents ($\gamma$M, $\gamma$MH, $\beta$M, $\beta$MH, $\alpha$M1K, $\alpha$M1KH, $\alpha$M2K, $\alpha$M2KH, $\alpha$M3K, $\alpha$M3KH, $\beta$MK, $\beta$MKH, $\gamma$MN, $\gamma$MNH, RMH, $\lambda$MLiH, $\varepsilon$MK, $\varepsilon$MKH, AMK, AMKH, $\delta$M1K, $\delta$M1KH, $\delta$M2KH, $\delta$M2Na, $\delta$M2NaH, $\delta$M3K, $\delta$M3KH, romanechite, todorokite, manganite, groutite, feitknechtite, hydrohausmannite, lithiophorite, chalcophanite, hausmannite, bixibyite, pyrochroite, and manganosite) and different types of activated charcoal adsorbents (two medicinal activated charcoals: Medicinal Charcoal Capsules, AST-120, and commercial activated charcoals with a specific surface area of 600 m$^2$/g, 720 m$^2$/g, 1045 m$^2$/g, 1400 m$^2$/g, 1800 m$^2$/g and 3500 m$^2$/g) were weighed, each 20 mg, 10 mL of solution of uric acid in SIF (adsorbent concentration: 2 g/L) was added, and the mixture was placed on a shaker and oscillated at 200 rpm for 1 h at 25°C. Thereafter, 1 mL of each sample was pipetted and centrifuged at 10000 rpm for 5 min. The supernatant was diluted 10 times (90 $\mu$L of SIF + 10 $\mu$L of supernatant) and placed on a 96-well plate. The absorbance was measured at 291 nm using a microplate reader. Standard curves were made freshly, including 0, 12.5, 25, 50, 100, and 500 $\mu$M point values. The uric acid concentration C ($\mu$M) in the supernatant was calculated according to the standard curve, and the uric acid clearance rate (%) was calculated according to formula 2.

The result is shown in Fig. 2. Fig. 2 shows that different types of insoluble manganese-containing compounds and different types of activated charcoals have certain clearance effect on uric acid under the conditions of SIF assay. Among them, the various commercially available insoluble manganese-containing compounds, the insoluble manganese-containing compounds prepared according to the method of Example 1, and the various activated charcoal adsorbents could achieve high uric acid clearance rate. In addition, it was also found that the uric acid clearance rate of potassium-type, sodium-type or ammonium-type manganese oxides changed after they were converted into corresponding hydrogen-type manganese oxides.

**2.3 Comparison between different types of insoluble manganese-containing compounds and activated charcoal adsorbents in uric acid clearance rate as measured in SIF assay**

The experimental method is as follows: 500 $\mu$M solution of uric acid in SIF was freshly formulated (concentration 8.4 mg/dL) with a pH of 6.8 and containing about 50 mM phosphate. Different types of adsorbents ($\delta$M1KH, $\alpha$M3KH, $\delta$M2KH, $\beta$MKH, RMH, $\gamma$MNH, $\lambda$MLiH, AMKH and AST-120) were weighed, 4 mg each, and 2 mL of solution of uric acid in SIF (concentration of adsorbent: 2 g/L) was added. At 25°C, the mixture was put on a shaker for oscillation at 200 rpm for 10 min, 30 min, 60 min, 120 min, 240 min, and shaken manually for 5 min. Thereafter, the mixture was filtered immediately, and the filtrate was diluted 10 times (90 $\mu$L of SIF + 10 $\mu$L of filtrate), placed on a 96-well plate, and the absorbance was measured at 291 nm using a microplate reader. Standard curves were made freshly, including 0, 12.5, 25, 50, 100, and 500 $\mu$M point values. The uric acid concentration C($\mu$M) in the supernatant was calculated according to the standard curve, the uric acid clearance rate (%) after mixing for different periods of time was calculated according to formula 2, and a curve of uric acid clearance rate versus mixing time was plotted.

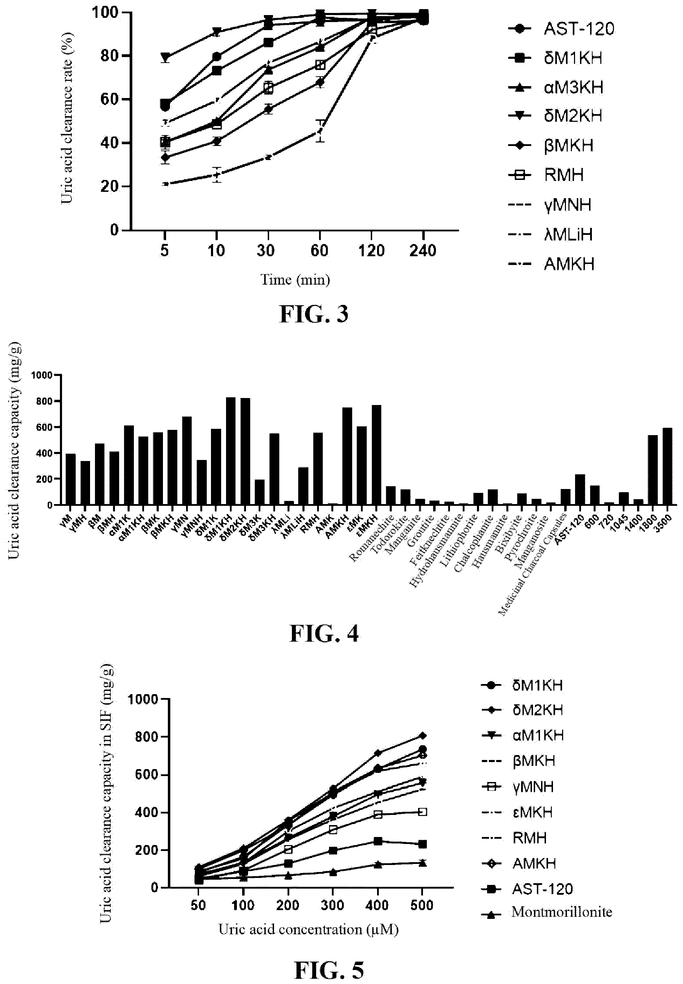

The result is shown in Fig. 3. Fig. 3 shows that the various insoluble manganese-containing compounds and activated charcoals prepared according to the method of Example 1 had a relatively fast uric acid clearance rate, and both could effectively clear uric acid within 30-60 min, and cleared the majority ($\geq$80%) of the uric acid in the solution system within 120 min.

**2.4 Comparison between different types of insoluble manganese-containing compounds and activated charcoal adsorbents in terms of uric acid clearance capacity measured in SIF assay**

The experimental method is as follows: 500 $\mu$M solution of uric acid in SIF was freshly formulated (concentration 8.4 mg/dL) with a pH of 6.8 and containing about 50 mM phosphate. Different types of adsorbents ($\gamma$M, $\gamma$MH, $\beta$M, $\beta$MH, $\alpha$M1K, $\alpha$M1KH, $\beta$MK, $\beta$MKH, $\gamma$MN, $\gamma$MNH, $\delta$M1K, $\delta$M1KH, $\delta$M2KH, $\delta$M3K, $\delta$M3KH, $\lambda$MLi, $\lambda$MLiH, RMH, AMK, AMKH, $\varepsilon$MK, $\varepsilon$MKH, romanechite, todorokite, manganite, groutite, feitknechtite, hydrohausmannite, lithiophorite, chalcophanite, hausmannite, bixibyite, pyrochroite, manganosite, Medicinal Charcoal Capsules, AST-120, and commercial activated charcoals with a specific surface area of 600 m$^2$/g, 720 m$^2$/g, 1045 m$^2$/g, 1400 m$^2$/g, 1800 m$^2$/g and 3500 m$^2$/g) were weighed, each 4 mg, 40 mL of solution of uric acid in SIF (adsorbent concentration: 0.1 g/L) was added, the mixture was placed on a shaker and oscillated at 200 rpm for 4 h at 37°C. Thereafter, 1 mL of each sample was pipetted and centrifuged at 10,000 rpm for 5 min. 100 $\mu$L of the supernatant was taken and placed on a 96-well plate. The absorbance was measured at 291 nm using a microplate reader. Standard curves were made freshly, including 0, 100, 200, 300, 400 and 500 $\mu$M point values. The uric acid concentration C ($\mu$M) in the supernatant was calculated according to the standard curve, and the uric acid clearance capacity Q(mg/g) was calculated according to formula 3.

$$\text{Formula 3: } Q = 0.168 * (500 - C) * 40/4$$

The result is shown in Fig. 4. Fig. 4 shows that different types of insoluble manganese-containing compounds and different types of activated charcoals have certain clearance effect on uric acid under the conditions of SIF assay. Among all the types of insoluble manganese-containing compounds, the minimum and maximum clearance capacities are about 10 mg/g and about 800 mg/g, respectively, and among all the types of activated charcoal, the minimum and maximum clearance capacities are about 20 mg/g and about 600 mg/g, respectively. In general, insoluble manganese-containing compounds and activated charcoal are similar in uric acid clearance capacity under the SIF assay conditions.

**2.5 Comparison of uric acid clearance capacity and clearance rate between insoluble manganese-containing compounds and activated charcoal adsorbents, montmorillonite, and ion exchange resins in simple simulated intestinal fluid (SIF) and complex simulated intestinal fluid FaSSIF and FeSSIF assays**

Compared with SIF, FaSSIF and FeSSIF have more complex components that are more close to the real situation in the body. Under such complex conditions, the clearance effect of the adsorbent on uric acid is easily affected by the complex environmental components. Therefore, this example compares the uric acid clearance capacity of various insoluble manganese-containing compounds with that of activated charcoal adsorbent, montmorillonite and ion exchange resin as measured in SIF, FaSSIF and FeSSIF assays to examine the effect of complex environment on the uric acid adsorption effect of the adsorbent.

In particular, the example is divided into two separate parts. In the first part, comparison of uric acid clearance capacity was made between various insoluble manganese-containing compounds and activated charcoal adsorbent (AST-120) and montmorillonite as measured in SIF, FaSSIF or FeSSIF assays. In the second part, an exemplary insoluble manganese-containing compound, $\delta$M4K was compared with an ion exchange resin (sevelamer hydrochloride) in terms of uric acid clearance capacity and clearance rate as measured in SIF, FaSSIF, or FeSSIF assays.

The specific experimental method in the first part is as follows: fresh 500 $\mu$M solutions of uric acid in SIF, FaSSIF or FeSSIF (concentration 8.4 mg/dL) were freshly formulated. The 500 $\mu$M uric acid solutions obtained above were diluted with SIF, FaSSIF or FeSSIF solutions to obtain SIF, FaSSIF or FeSSIF solutions with uric acid concentrations of 400, 300, 200, 100 and 50 $\mu$M, respectively. The uric acid concentration was recorded as $C0$. The adsorbents ($\delta$M1KH, $\delta$M2KH, $\alpha$M1KH, $\beta$MKH, $\gamma$MNH, $\epsilon$MKH, RMH, AMKH, AST-120 and montmorillonite) were weighed, 4 mg each, and 40 mL of solution with different uric acid concentrations (adsorbent concentration: 0.1 g/L) were added. At 37°C, the mixture was placed on a shaker and oscillated at 200 rpm for 4 h. Thereafter, 1 mL of each sample was pipetted and centrifuged at 10,000 rpm for 5 min. 100 $\mu$L of the supernatant was taken and placed on a 96-well plate. The absorbance was measured at 291 nm using a microplate reader. Standard curves were made freshly using SIF, FaSSIF or FeSSIF, including 0, 100, 200, 300, 400 and 500 $\mu$M point values. The uric acid concentration Ct($\mu$M) in the supernatant was calculated according to the standard curve, and the uric acid clearance capacity Q(mg/g) was calculated according to formula 4.

$$\text{Formula 4: } Q = 0.168 * (C0 - Ct) * 40/4$$

The specific experimental method in the second part is as follows: fresh 500 $\mu$M solutions of uric acid in SIF, FaSSIF or FeSSIF (concentration 8.4 mg/dL) was freshly formulated. The 500 $\mu$M uric acid solutions obtained above were diluted with SIF, FaSSIF or FeSSIF solutions to obtain SIF, FaSSIF or FeSSIF solutions with uric acid concentrations of 500, 400, 300, 200 and 100 $\mu$M, respectively. The uric acid concentration was recorded as $C0$. The adsorbents ($\delta$M4K and sevelamer Hydrochloride) were weighed, 4 mg each, and 40 mL of solutions with different uric acid concentrations (adsorbent concentration: 0.1 g/L) were added. At 37°C, the mixture was placed on a shaker and oscillated at 200 rpm for 4 h. Thereafter, 1 mL of each sample was pipetted and centrifuged at 10,000 rpm for 5 min. 100 $\mu$L of the supernatant was taken and placed on a 96-well plate. The absorbance was measured at 291 nm using a microplate reader. Standard curves were made freshly using SIF, FaSSIF or FeSSIF, including 0, 100, 200, 300, 400, 500, 750 and 1000 $\mu$M point values. The uric acid concentration $Ct$($\mu$M) in the supernatant was calculated according to the standard curve, and the uric acid clearance capacity Q (mg/g) was calculated according to formula 4 and the uric acid clearance rate (%) was calculated according to formula 5.

$$\text{Formula 5: Uric acid clearance rate} = (C0 - Ct)/C0 * 100\%$$

The results of the first part are shown in Figs. 5, 6 and 7. Figs. 5, 6 and 7 show that under the conditions of SIF assay, the equilibrium uric acid clearance capacity reached 400-800 mg/g for the various insoluble manganese-containing compounds, about 250 mg/g for activated charcoal, and about 100mg/g for montmorillonite. The equilibrium uric acid clearance capacity of $\delta$-form manganese oxide was about 800 mg/g under FaSSIF assay conditions, while that of activated charcoal and montmorillonite was about 0 mg/g. Under FeSSIF assay conditions, the equilibrium uric acid clearance capacity of δ-form manganese oxide was reduced slightly but stilled reached about 600 mg/g, while that of activated charcoal and montmorillonite was substantially 0 mg/g. This shows that under different complex simulated intestinal fluid conditions, δ-form manganese oxide retains a high uric acid adsorption effect, while the adsorption effect of activated charcoal and montmorillonite on uric acid basically disappears.

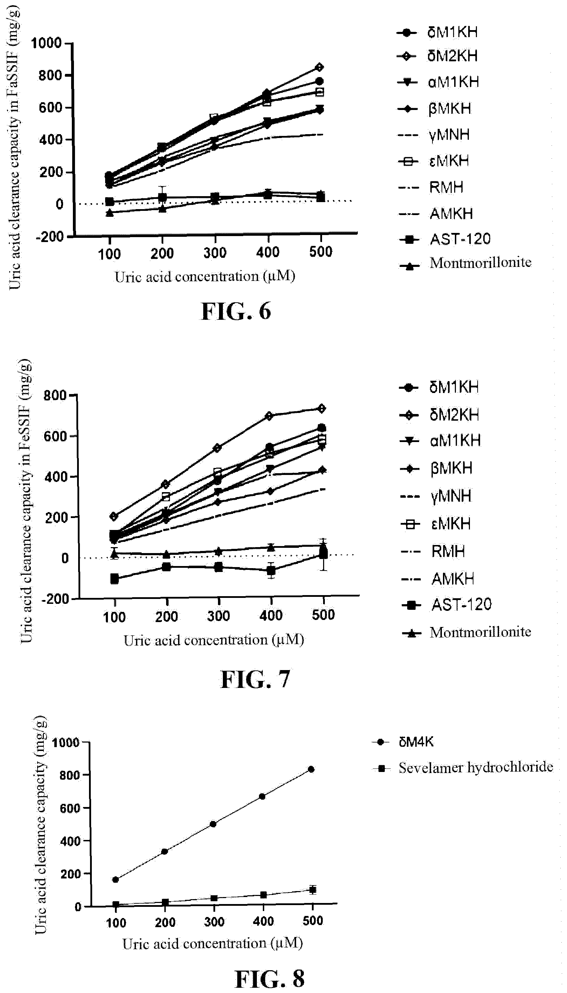

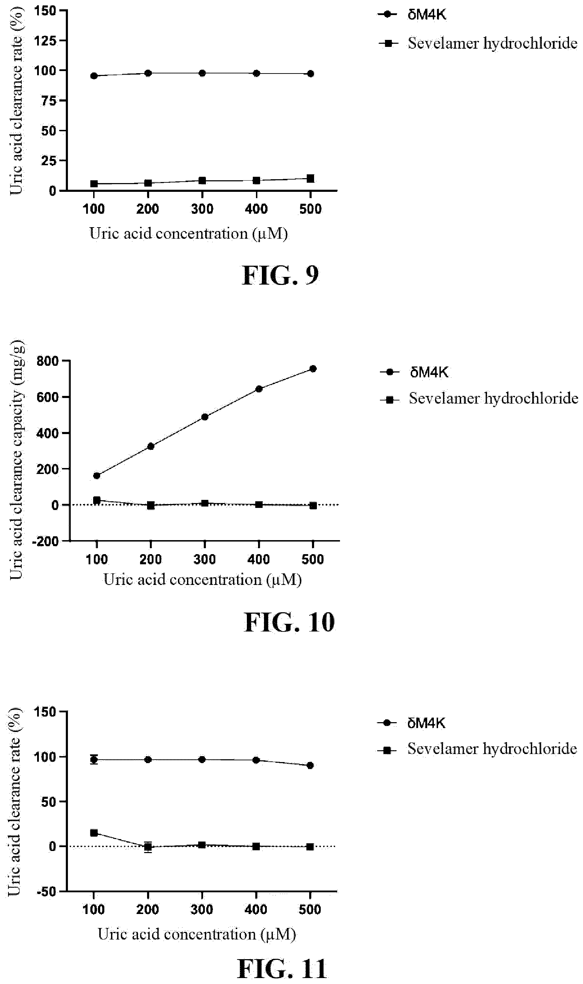

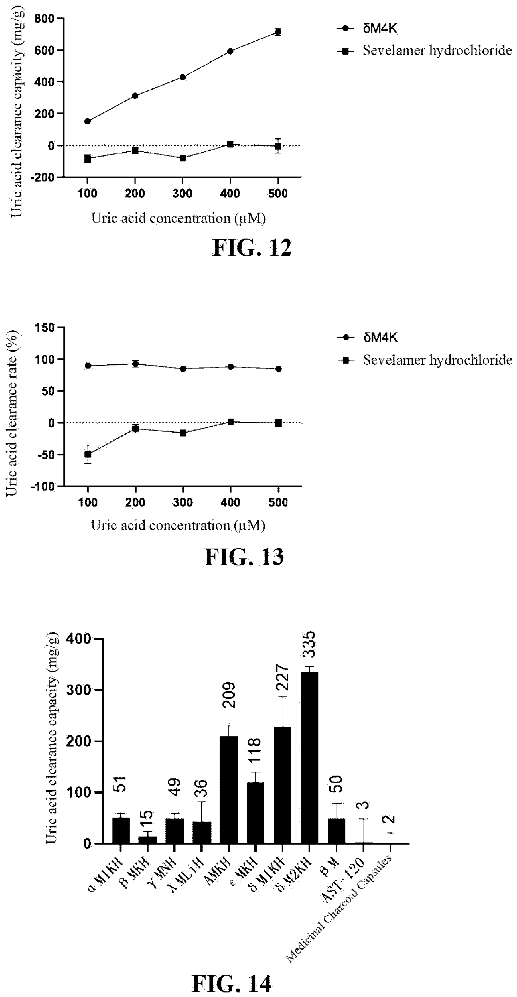

The results of the second part are shown in Figs. 8 to 13. Figs. 8 and 9 show that the uric acid clearance capacity of δM4K increases with the concentration of uric acid under the SIF assay conditions. Under the condition of 500 μM uric acid concentration, the uric acid clearance capacity and uric acid clearance rate of δM4K were about 800 mg/g and about 100%, respectively, while the uric acid clearance capacity and uric acid clearance rate of ion exchange resin were only about 84 mg/g and about 10%, respectively. Figs. 10 and 11 show that the clearance effect of δM4K on uric acid under the FaSSIF assay conditions is similar to that under the SIF assay conditions. Specifically, under the condition of 500 μM uric acid concentration, the uric acid clearance capacity and uric acid clearance rate of δM4K still could reach about 750 mg/g and about 90%, respectively, while the uric acid clearance capacity and uric acid clearance rate of ion exchange resin were substantially 0 mg/g and 0%, respectively. Figs. 12 and 13 show that the clearance effect of δM4K on uric acid under the FeSSIF assay conditions is similar to that under the SIF assay conditions. Specifically, under the condition of 500 μM uric acid concentration, the uric acid clearance capacity and uric acid clearance rate of δM4K were reduced slightly, but still reached about 700 mg/g and about 80%, respectively, while the uric acid clearance capacity and uric acid clearance rate of ion exchange resin were also substantially 0 mg/g and about 0%, respectively. This also shows that δ-form manganese oxide can retain high uric acid adsorption effect under different complex simulated intestinal fluid conditions, while the uric acid adsorption effect of ion exchange resin basically disappears under the same conditions.

### 2.6 Comparison of uric acid adsorption effect between different types of insoluble manganese-containing compounds and activated charcoal adsorbents in pork enzymatic hydrolysate assay

Pork enzymatic hydrolysate is rich in various organic molecules, such as peptides, amino acids, lipid molecules, carbohydrates and various vitamins, which have a strong interference effect on the uric acid clearance effect of uric acid adsorbents. Therefore, this example compares the uric acid clearance capacity of various insoluble manganese-containing compounds with that of activated charcoal adsorbents in a pork enzymatic hydrolysate assay to further examine the effect of complex environment on the uric acid adsorption effect of the adsorbent.

The specific experimental method is as follows: pork was heated and cooked, then broken in a stirrer, a mixed enzymatic hydrolysate of α-amylase, amyloglucosidase, nuclease, pancreatic lipase and trypsin was added, the system was enzymolyzed at 37°C overnight to obtain the pork enzymatic hydrolysate. Uric acid (final concentration 500 μM) was added into the pork enzymatic hydrolysate, and different types of adsorbents (final concentration 0.1 g/L) were added. At 37°C, the system was put on a shaker and oscillated at 200 rpm for 4 h. The supernatant was obtained by filtration. The uric acid concentration C (μM) in the supernatant was tested and the clearance capacity Q (mg/g) was calculated according to formula 3.

The result is shown in Fig. 14. Fig. 14 shows that under the pork enzymatic hydrolysate assay conditions, amorphous manganese oxide (AMKH) and δ-form manganese oxides (δM1KH and δM2KH) had the best adsorption effect on uric acid, and still retained a uric acid clearance capacity of more than 200 mg/g, while the uric acid clearance effect of activated charcoal (AST-120 and Medicinal Charcoal Capsules) completely disappeared.

### 2.7 Comparison of uric acid adsorption effect between different types of insoluble manganese-containing compound adsorbents in food enzymatic hydrolysate assay

Different food enzymatic hydrolysates simulate the human gastrointestinal chyme and have a strong interference effect on the uric acid clearance effect of uric acid adsorbents. Therefore, this example compares the uric acid clearance capacity of various insoluble manganese-containing compounds in a food enzymatic hydrolysate assay to further examine the effect of complex environment on the uric acid adsorption effect of the adsorbent.

The specific experimental method is as follows: pork, rice and a vegetable were separately heated and cooked, then broken in a stirrer, a fruit was directly broken in a stirrer, a mixed enzymatic hydrolysate of α-amylase, amyloglucosidase, nuclease, pancreatic lipase and trypsin was added, the system was enzymolyzed at 37°C overnight to obtain 4 food enzymatic hydrolysate. Uric acid (final concentration of 500 μM) and different types of insoluble manganese-containing compounds (manganese oxide) (final concentration of 0.1 g/L) were added to the 4 food enzymatic hydrolysates, respectively, and the samples were placed on a shaker and oscillated at 200 rpm for 4 h at 37°C. The supernatant was obtained by filtration. The uric acid concentration C (μM) in the supernatant was tested and the clearance capacity Q (mg/g) was calculated according to formula 3.

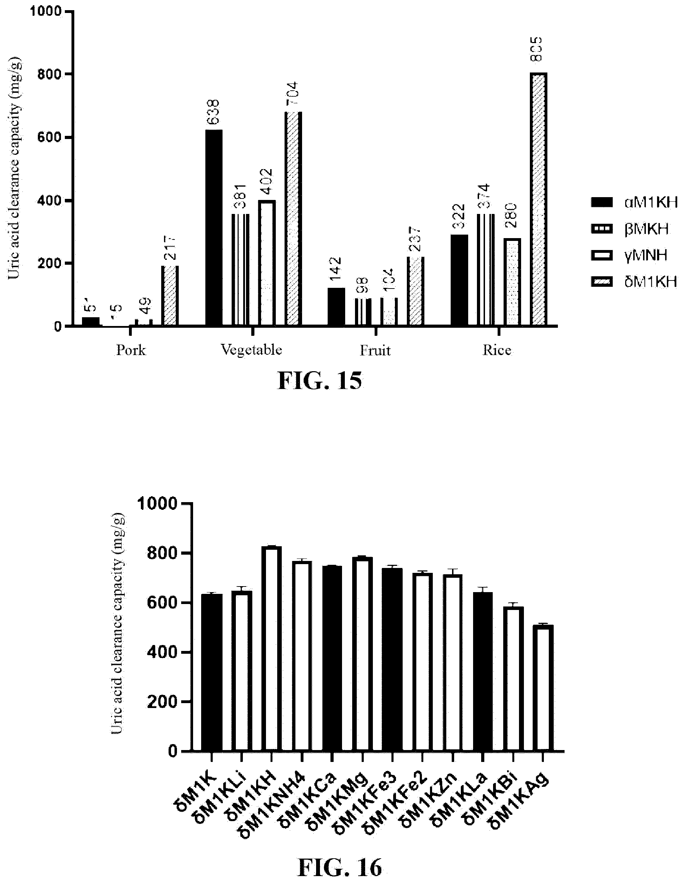

The result is shown in Fig. 15. Fig. 15 shows that under the assay conditions of the 4 food enzymatic hydrolysates, different types of manganese oxide all retained a high adsorption effect on uric acid, among which δ-form manganese oxide had the best effect, retaining a uric acid clearance capacity of more than 200 mg/g in the enzymatic hydrolysates of different foods.

**2.8 Comparison of uric acid clearance capacity of manganese oxide adsorbents of different salt types in SIF assay**

The purpose of this example is to investigate the effect of the type of cations incorporated into the holes between atoms in the manganese oxide structure on the uric acid adsorption effect of the corresponding salt-type manganese oxide adsorbent.

The experimental method is as follows: 500 $\mu$M solution of uric acid in SIF was freshly formulated (concentration 8.4 mg/dL) with a pH of 6.8 and containing about 50 mM phosphate. Different salt types of manganese oxide absorbents ($\delta$M1K, $\delta$M1KLi, $\delta$M1KH, $\delta$M1KNH4, $\delta$M1KCa, $\delta$M1KMg, $\delta$M1KFe2, $\delta$M1KFe3, $\delta$M1KZn, $\delta$M1KLa, $\delta$M1KBi, $\delta$M1KAg) were weighed, 4 mg each. 40 mL of uric acid solution (absorbent concentration: 0.1 g/L) in SIF was added, and the mixture was placed on a shaker and oscillated at 37°C for 4 h at 200 rpm. Thereafter, 1 mL of each sample was pipetted and centrifuged at 10,000 rpm for 5 min. 100 $\mu$L of the supernatant was taken and placed on a 96-well plate. The absorbance was measured at 291 nm using a microplate reader. Standard curves were made freshly, including 0, 100, 200, 300, 400 and 500 $\mu$M point values. The uric acid concentration C ($\mu$M) in the supernatant was calculated according to the standard curve, and the uric acid clearance capacity Q(mg/g) was calculated according to formula 3.

The result is shown in Fig. 16. Fig. 16 shows that under the SIF assay conditions, different salt types of manganese oxide adsorbents had a certain clearance effect on uric acid. Among all types of manganese oxide absorbents, the minimum clearance capacity was about 500 mg/g and the maximum clearance capacity was about 800 mg/g. In general, under the SIF assay conditions, different salt types of manganese oxide absorbents had similar capacity to clear uric acid.

**2.9 Comparison of uric acid clearance capacity and uric acid clearance rate as measured in SIF assay for different concentrations of insoluble manganese-containing compounds**

The experimental method is as follows: 500 $\mu$M solution of uric acid in SIF was freshly formulated (concentration 8.4 mg/dL) with a pH of 6.8 and containing about 50 mM phosphate. 2, 2.4, 2.8, 3, 3.2, 3.6, 4, 6 or 8 mg of insoluble manganese-containing compound ($\delta$M4K) was weighed separately, and 40 mL of uric acid solution in SIF was added to make the concentration of the adsorbent (Cx) to be 0.06, 0.07, 0.075, 0.08, 0.09, 0.1, 0.15 or 0.2 g/L, respectively. The mixture was placed on a shaker and oscillated at 200 rpm for 4 h at 37°C. Thereafter, 1 mL of each sample was pipetted and centrifuged at 10,000 rpm for 5 min. 100 $\mu$L of the supernatant was taken and placed on a 96-well plate. The absorbance was measured at 291 nm using a microplate reader. Standard curves were made freshly, including 0, 100, 200, 300, 400 and 500 $\mu$M point values. The uric acid concentration C ($\mu$M) in the supernatant was calculated according to the standard curve, and the uric acid clearance capacity Q (mg/g) was calculated according to formula 6 and the uric acid clearance rate (%) was calculated according to formula 2.

$$\text{Formula 6: } Q = 0.168 * (500 - C) / Cx$$

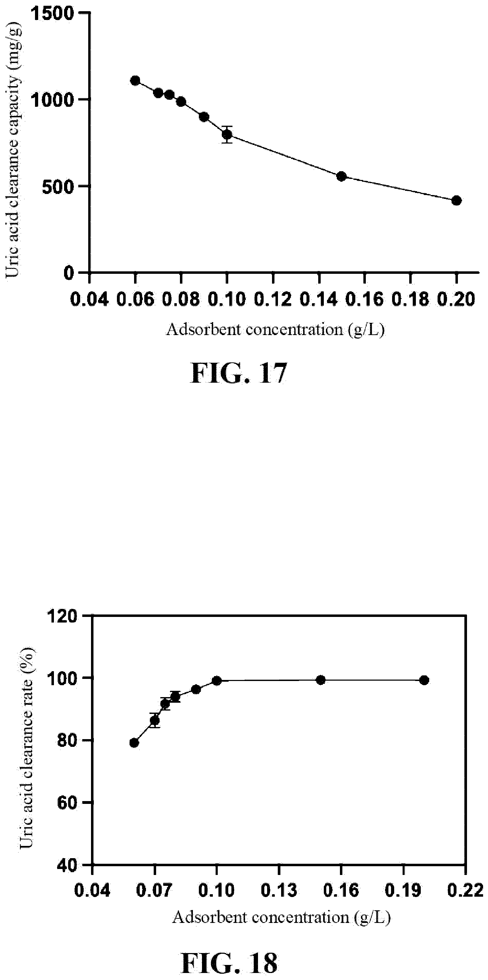

The results are shown in Fig. 17 and Fig. 18, respectively. Fig. 17 shows that different concentrations of $\delta$M4K adsorbent had a certain clearance effect on uric acid under the conditions of SIF assay with the lowest clearance capacity of about 400 mg/g, the highest clearance capacity of about 1100 mg/g, and the clearance capacity gradually decreased with the increasing concentration of adsorbents. Fig. 18 shows that under the SIF assay conditions, the uric acid clearance rate of the $\delta$M4K adsorbent at different concentrations gradually increased with the gradual increase in adsorbent concentration. When the adsorbent concentration reached 0.1 g/L, the uric acid clearance rate reached about 100%, and when the adsorbent concentration continued to increase, the uric acid clearance rate remained at about 100%.

**2.10 Cumulative uric acid clearance capacity measured in multiple SIF assays for insoluble manganese-containing compounds**

It is known that the content of uric acid in the intestinal tract is not constant. Specifically, the content of uric acid varies in different intestinal segments and at different time points, but is generally between 100 $\mu$M and 1300 $\mu$M. By conducting multiple adsorption assays of the adsorbent in a low-concentration uric acid solution (such as 100 $\mu$M uric acid solution), the ability of the adsorbent to continuously adsorb uric acid under conditions of low uric acid content in the environment when passing through the intestinal tract can be reflected.

The experimental method is as follows: 100 $\mu$M solution of uric acid in SIF was freshly formulated (concentration 1.68 mg/dL) with a pH of about 6.8 and containing about 50 mM phosphate. 4 mg of insoluble manganese-containing compound (δM4K) was weighed, 40 mL of solution of uric acid in SIF (absorbent concentration: 0.1 g/L) was added, and the mixture was placed in a shaker and oscillated for 0.5 h at 200 rpm at 37°C, which was recorded as the 1st adsorption. The solid was then filtered out and placed into a new solution of 40 mL of 100 μM uric acid in SIF for the 2nd adsorption under the same oscillating conditions. The above procedure was then repeated until a total of 12 times of adsorption had been completed. Thereafter, 1 mL of filtrate was pipetted from the filtrate from every time of adsorption, and centrifuged at 10,000 rpm for 5 min. 100 μL of the supernatant was taken and placed on a 96-well plate. The absorbance was measured at 291 nm using a microplate reader. Standard curves were made freshly, including 0, 25, 50, 100, 200 and 500 μM point values. The uric acid concentration C (μM) in the supernatant was calculated according to the standard curve, and the uric acid clearance capacity Q (mg/g) was calculated according to formula 7 and the uric acid clearance rate (%) was calculated according to formula 8.

$$\text{Formula 7: } Q = 0.168 * (100 - C) * 40/4$$

$$\text{Formula 8: Uric acid clearance rate} = (100 - C)/100 * 100\%$$

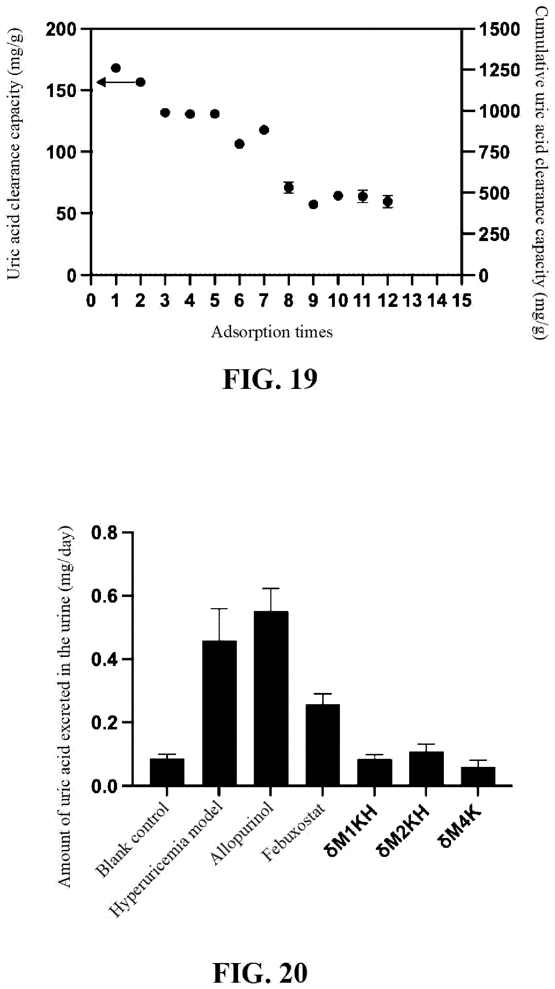

The result is shown in Fig. 19. Fig. 19 shows that δM4K exhibited excellent uric acid clearance in multiple adsorption assays in solutions with low concentrations of uric acid in SIF. Specifically, at the 1st adsorption, the uric acid clearance capacity of δM4K was about 170 mg/g, and the uric acid clearance rate was about 100%. With the increase of adsorption times, the uric acid clearance capacity of δM4K decreased gradually. Nevertheless, even at the 12th time of adsorption, the uric acid clearance capacity of δM4K was still about 60 mg/g, and the cumulative uric acid clearance capacity of a total of 12 times of adsorption was about 1250 mg/g. The results show that δM4K could adsorb uric acid continuously at low uric acid concentration, and could still maintain effective uric acid adsorption capacity after multiple times of adsorption.

**Example 3 Size and solubility considerations for manganese-containing compounds and calculation of oral safe doses**

Manganese is one of the essential trace elements for the normal body. As a component of many enzymes, manganese plays many important physiological roles. However, excessive intake of manganese can also cause damage to the body. For example, some manganese mine workers have been reported to suffer severe neurological damage due to long-term inhalation of air with excessive manganese levels. Inhaled manganese can be transported directly into brain tissue and can cause a permanent neurological disorder called "manganism." Insoluble inorganic manganese compounds, such as manganese oxide, are generally considered to be relatively safe by oral intake compared to inhalation, but there is still a risk of serious toxicity from excessive intake. For example, nanometer-sized manganese oxide and slightly soluble manganese ions can enter the body circulation through the gastrointestinal tract and become the main source of toxicity, and their toxicity depends mainly on the dose of manganese absorbed.

In this example, the applicant conducted an in vitro experiment simulating the dissolution of manganese in the gastrointestinal fluid, and measured the solubility of manganese oxide with various particle sizes of 1 micron and above (this size is designed to reduce the probability of nanoform manganese-containing compounds being absorbed into the body circulation) in simulated intestinal fluid (SIF) (expressed as the value of the manganese dissolution amount of manganese oxide in SIF measured in the "Method for manganese dissolution experiment" described below) to compare the dissolution of different types of manganese oxide. Since the amount of the manganese-containing compound dissolved in the gastrointestinal fluid basically determines the amount absorbed by the body, the applicant also calculated the safe dosage of these different types of manganese oxides when used orally in the human body based on the above results.

Method for manganese dissolution experiment

Different types of manganese oxide of different synthesis lots were dispersed in SIF at 2 g/L, incubated in a constant temperature shaker at 37°C for 4 h, and then filtered to obtain the filtrate. The manganese content in the filtrate was detected by inductively coupled plasma mass spectrometry (ICP-MS) to obtain the value of the manganese dissolution amount of manganese oxide in SIF, which was set as C mg/L.

Method for calculating safe oral dose

The oral safe dose was calculated based on the tolerable upper intake level (TUIL) of 0.16 mg/kg/day or 11 mg/day (recommended by the Agency for Toxic Substances and Disease Registry (ATSDR)). It is known that a volume (V) of about 9 liters of fluid passes through the gastrointestinal tract every day (Kvietys, P.R. and Granger, D.N. (2010), Role of intestinal lymphatics in interstitial volume regulation and transmucosal water transport. Annals of the New York Academy of Sciences, 1207: E29-E43. https://doi.org/10.1111/j.1749-6632.2010.05709.x). Assuming that it takes one day for manganese oxide to pass through the gastrointestinal tract and be excreted from the body, based on the amount of manganese dissolved in SIF (C mg/L) and an additional uncertainty factor (UF) of 10, the calculation formula for the oral safe dose of different types of manganese oxide can be expressed as follows:

$$\text{Oral safe dose (g)} = (\text{TUIL}*2 \text{ g}) / (C*V) / \text{UF}.$$

The amount of manganese dissolved in SIF for different types of manganese oxide measured in this example and their respective estimated oral safe doses are as follows:

| | Amount of manganese dissolved in SIF (mg/L) | | | Estimated oral safe dose (g/day) | | |
|---|---|---|---|---|---|---|
| | Lot 1 | Lot 2 | Lot 3 | Lot 1 | Lot 2 | Lot 3 |
| δM1K | <0.005 | <0.005 | <0.005 | >49 | >49 | >49 |
| δM1KH | 0.009 | 0.006 | <0.005 | 27 | 41 | >49 |
| δM2KH | 0.005 | <0.005 | 0.006 | 49 | >49 | 41 |
| AMKH | 0.027 | 0.045 | 0.020 | 9 | 5 | 12 |
| εMKH | 0.118 | 0.216 | 0.087 | 2 | 1 | 3 |
| λMLiH | 0.008 | 0.018 | 0.033 | 31 | 14 | 7 |
| αM1KH | 1.338 | 1.169 | 0.853 | 0.2 | 0.2 | 0.3 |
| βMKH | 0.947 | 0.793 | 1.025 | 0.3 | 0.3 | 0.2 |
| γMNH | 2.583 | 2.039 | 3.569 | 0.1 | 0.1 | 0.1 |
| RMH | 1.778 | 3.036 | 2.127 | 0.1 | 0.1 | 0.1 |
| βMH | 0.028 | 0.021 | 0.035 | 9 | 12 | 7 |
| βM | 0.007 | 0.010 | 0.013 | 35 | 24 | 19 |
| γMH | 0.009 | 0.011 | 0.009 | 27 | 22 | 27 |
| γM | 0.007 | 0.008 | 0.007 | 35 | 31 | 35 |

The above results indicate that the manganese oxides prepared by different methods all have extremely low solubility in simulated intestinal fluid, which allows them to be administered at very high oral safe doses without causing unacceptable side effects.

**Example 4 In vivo performance evaluation**

This example compares the uric acid-lowering properties of manganese oxide and a control substance in animals.
Specifically, the test substance and the control substance were administered to healthy rats, healthy mice, hyperuricemia model rats and hyperuricemia model mice, and the effect of the test substance in reducing the uric acid content in body fluids of the animals was examined.

**4.1 Experiment on healthy rats**

Experimental method

Male SD rats aged 7 to 8 weeks were randomly divided into 5 groups, with 6 rats in each group, including blank group (administered with sterile water), low-dose manganese oxide group, medium-dose manganese oxide group, high-dose manganese oxide group and ultra-high-dose manganese oxide group. From the 1st day to the 7th day, the blank group was gavaged with sterile water every day, and the other treatment groups were each administered with a fixed dose of the test substance suspension. On days 0 and 7, uric acid levels in rat body fluids were measured.

Results

After administration of manganese oxide, the rats in each treatment group well tolerated the drug and were free of adverse reactions. Manganese oxide can effectively reduce the uric acid content in rat body fluids.

**4.2 Experiment on healthy mice**

Experimental method

Male SD mice aged 7 to 8 weeks were randomly divided into 5 groups, with 6 rats in each group, including blank group (administered with sterile water), low-dose manganese oxide group, medium-dose manganese oxide group, high-dose manganese oxide group and ultra-high-dose manganese oxide group. From the 1st day to the 7th day, the blank group was gavaged with sterile water every day, and the other treatment groups were each administered with a fixed dose of the test substance suspension. On days 0 and 7, uric acid levels in mouse body fluids were measured.

Results

After administration of manganese oxide, the mice in each treatment group well tolerated the drug and were free of adverse reactions. Manganese oxide can effectively reduce the uric acid content in mouse body fluids.

**4.3 Experiment on hyperuricemia model rats**

The hyperuricemia rat model was established as follows (refer to J. Agric. Food Chem. 2019, 67, 220-228): the animals were gavaged with 450 mg/kg/day oteracil potassium (saline solution) + 100 mg/kg/d adenine (saline solution) for 7 days.

Experimental method

Male SD rats aged 7 to 8 weeks were randomly divided into 9 groups, with 6 rats in each group, including blank group (administered with sterile water), model group (administered with induction drug only), activated charcoal control group (administered with induction drug + activated charcoal), montmorillonite control group (administered with induction drug + montmorillonite), allopurinol control group (administered with induction drug + allopurinol), lesinurad control group (administered with induction drug + lesinurad), low-dose group (administered with induction drug + low-dose manganese oxide), medium-dose group (administered with induction drug + medium-dose manganese oxide), and high-dose group (administered with induction drug + high-dose manganese oxide). Rats were gavaged with corresponding drugs daily for 7 days. On the 7th day, the uric acid content in the body fluid of the hyperuricemia model rats was detected.

Results

Under drug induction, the level of uric acid in rat body fluids increased. However, manganese oxide effectively reduced uric acid levels in the body fluids of hyperuricemia model rats; at some doses, manganese oxide reduced uric acid levels better than or equivalent to allopurinol.

**4.4 Experiment on hyperuricemia model mice**

**4.4.1 Experiment on urate oxidase knockout (UOX KO) mice**

Experimental method

Urate oxidase knockout (UOX KO) mice aged 6 to 8 weeks were selected, and urine and blood were collected after one week of acclimation to test urine uric acid and blood uric acid levels. Based on the criterion of no significant difference in blood uric acid levels among the groups, the mice were randomly divided into 5 groups, with 9 mice in each group, including model group (administered with sterile water), positive control group (administered with allopurinol), low-dose group (administered with low-dose manganese oxide), medium-dose group (administered with medium-dose manganese oxide), and high-dose group (administered with high-dose manganese oxide). Mice were gavaged with corresponding drugs daily. After the start of drug administration, the blood of mice was collected every other week to prepare serum, and 24-hour urine was collected to detect the uric acid content in the urine using a uric acid assay kit.

<u>Results</u>

UOX KO mice had high levels of uric acid in blood and urine. Allopurinol was effective in reducing uric acid in UOX KO mice, and manganese oxide was superior to or equivalent to allopurinol in reducing uric acid at some doses.

**4.4.2 Experiment on oteracil potassium and adenine induced hyperuricemia model mice**

Oteracil potassium and adenine are known to be model drugs capable of inducing hyperuricemia in mice. Specifically, oteracil potassium is a urate oxidase inhibitor that can inhibit further metabolism of uric acid in mice, thereby increasing the concentration of uric acid in the blood of mice; adenine can damage the kidneys, thereby reducing the excretion of uric acid from the kidneys of mice. In this example, the healthy mice were randomly divided into seven groups, the mouse hyperuricemia model was established by administering oteracil potassium and adenine (both of which were doped into the basic mouse diet) to 6 groups of mice, and one group of healthy mice fed with only the base mouse diet was taken as the blank control group. The mice of the experimental groups were given the corresponding experimental substances according to the dosing regimen described in the following experimental method. Six days after the beginning of the experiment, 24-hour urine of each group of mice was collected and uric acid content in the urine was detected by the uric acid assay kit to investigate the effects of various experimental substances on the amount of uric acid excreted in the urine of mice.

<u>Experimental method</u>

Forty-two 6-week-old male KM mice were randomly divided into 7 groups with 6 mice in each group, including blank control group, hyperuricemia model group, allopurinol group, febuxostat group, $\delta$M1KH group, $\delta$M2KH group and $\delta$M4K group. The control group was fed with basic mouse diet the first day (i.e. the day of the experiment) to the seventh day after the start of the experiment, and the other groups were fed with modeling mouse diet (4.4 w/w% oteracil potassium and 0.44 w/w% adenine were added into the basic mouse diet) for inducing hyperuricemia. From the first day to the seventh day after the start of the experiment, the allopurinol group freely drank drinking water containing 50 mg/L allopurinol every day. The febuxostat group was given febuxostat (5 mg/kg/day) by gavage every day. The $\delta$M1KH group, $\delta$M2KH group and $\delta$M4K group were fed with mouse diet with the drug every day (1.16 w/w% of $\delta$M1KH, $\delta$M2KH or $\delta$M4K was added to the modeling mouse diet, respectively). On day 6 after the start of the experiment, the mice were placed in metabolic cages and 24-hour urine was collected to analyze uric acid levels. One to two drops of 8 mol/L aqueous NaOH solution were added to the urine collection container to prevent urate precipitation. The uric acid level in urine was measured using a uric acid assay kit (Solarbio, Beijing, China), and the amount of uric acid excreted through urine in 24 hours was calculated based on the urine volume.

<u>Results</u>

The result is shown in Fig. 20. Fig. 20 shows that administration of oteracil potassium and adenine to mice for 7 consecutive days significantly increased the amount of uric acid excreted in the urine of the mice. The amount of uric acid excreted in the urine in mice administered with allopurinol or febuxostat, respectively, was also at a high level. In contrast, the amount of uric acid excreted in the urine was significantly reduced in mice receiving manganese oxide. Without being bound by any theory, it is believed that the reason why manganese oxide (e.g. $\delta$-form manganese oxide) greatly reduces uric acid excretion through the urine is related to its effect of significantly increasing uric acid excretion through the digestive tract (via feces).

**4.4.3 Experiment on uric acid-induced hyperuricemia model mice**

Uric acid itself can also be used to establish a hyperuricemia model in mice. Specifically, after intraperitoneal injection, uric acid can be rapidly absorbed into the blood through the mesentery, thereby increasing uric acid levels in the blood. Therefore, intraperitoneal injection of uric acid can be used to establish a model of hyperuricemia due to parenteral causes. In this example, a mouse hyperuricemia model was established by intraperitoneal injection of uric acid into healthy mice (the experimental group was gavaged with manganese oxide and injected with uric acid simultaneously), blood was extracted from the mice at different time points after the injection to prepare serum, and the uric acid content in the serum was tested to examine the effect of manganese oxide on uric acid levels in the blood of model mice with hyperuricemia caused by parenteral causes.

Experimental method

Eighteen 6-week-old male KM mice were randomly divided into 3 groups, with 6 mice in each group, including blank control group, hyperuricemia model group and δM4K group. After one week of acclimation, at 9 a.m. on the day of the experiment, the blank control group was gavaged with pure water, the hyperuricemia model group was gavaged with pure water, and the δM4K group was gavaged with 500 mg/kg δM4K, and fasted for 16 h at night. At 8 a.m. on the second day after the start of the experiment, 100 $\mu$L of blood was collected from each group of mice through the fundus venous plexus, and the serum was separated. The serum uric acid level was measured using a uric acid assay kit and recorded as the serum uric acid level at 0 min. At 9 a.m., the blank control group was gavaged with pure water and intraperitoneally injected with normal saline, the hyperuricemia model group was gavaged with pure water and intraperitoneally injected with uric acid solution (uric acid dose 1000 mg/kg), and the δM4K group was gavaged with 500 mg/kg of δM4K and intraperitoneally injected with uric acid solution (uric acid dose 1000 mg/kg). For each group of mice, 100 $\mu$L of blood was collected from the fundus venous plexus at 15 min, 30 min, 60 min and 120 min after the injection of uric acid, respectively, and the serum was separated and the serum uric acid level was measured using a uric acid assay kit.

Results

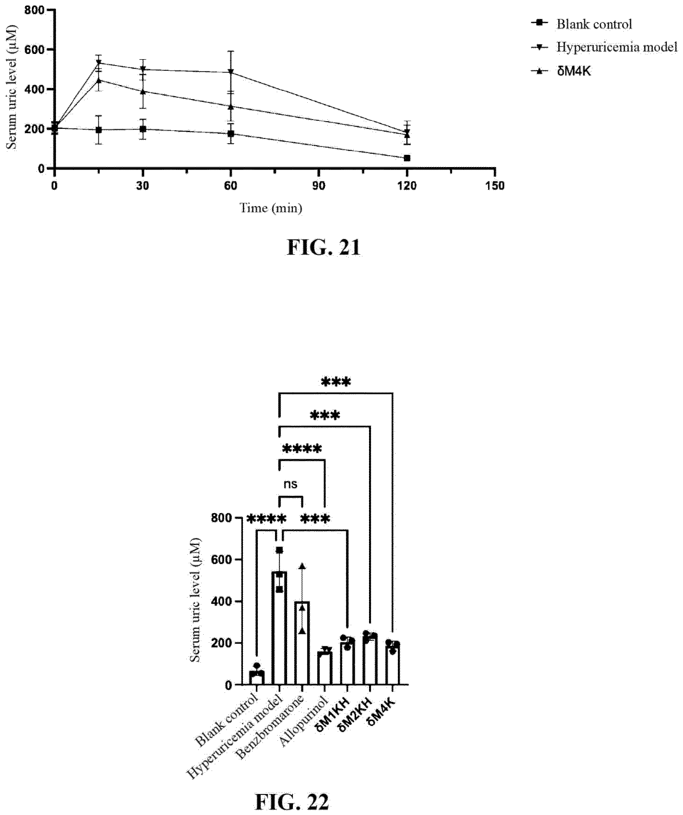

The result is shown in Fig. 21. Fig. 21 shows that after uric acid was injected into the peritoneal cavity of healthy mice, the serum uric acid level of the mice increased significantly. However, administration of manganese oxide by gavage can effectively offset the effect of intraperitoneal injection of uric acid on the serum uric acid level of mice: compared with mice not simultaneously administered with manganese oxide by gavage, administration of manganese oxide by gavage reduced the area under curve (AUC) of the serum uric acid level-time curve of mice by about 40%. The above results indicate that administration of manganese oxide (e.g. δ-form manganese oxide) by gavage can significantly reduce the uric acid level in the blood of mice after intraperitoneal injection of uric acid, and thus can be used to treat hyperuricemia caused by parenteral causes.

**4.4.4 Experiment on oteracil potassium and hypoxanthine induced hyperuricemia model mice**

Hypoxanthine can be metabolized into uric acid in the body, so it can also be used in combination with oteracil potassium as a model drug for inducing hyperuricemia in mice. In this example, the healthy mice were randomly divided into 7 groups, the mouse hyperuricemia model was established by administering oteracil potassium and hypoxanthine (they were injected intraperitoneally together) to 6 groups of mice, and 1 group of healthy mice injected intraperitoneally with normal saline was taken as the blank control group. The mice of the experimental groups were given the corresponding experimental substances according to the dosing regimen described in the following experimental method. On the 8th day after the start of the experiment, blood was drawn from each group of mice to prepare serum, and the uric acid content in the serum was tested to examine the effects of the experimental substances on the uric acid level in the blood of oteracil potassium and hypoxanthine induced hyperuricemia model mice.

Experimental method

Twenty-one 6-week-old male C57BL/6J mice were randomly divided into 7 groups with 3 mice in each group, including blank control group, hyperuricemia model group, benzbromarone group, allopurinol group, δM1KH group, δM2KH group and δM4K group. From the first day (i.e. the day of the experiment) to the eighth day after the start of the experiment, the blank control group was intraperitoneally injected with normal saline at 9:00 a.m. and 5:00 p.m. every day, and the remaining groups were intraperitoneally injected with modeling solution (100 mg/kg hypoxanthine + 100 mg/kg oteracil potassium) at 9:00 a.m. and 5:00 p.m. every day. The benzbromarone group was administered with benzbromarone solution (10 mg/kg) by gavage at 10 am every day. The allopurinol group was free to drink drinking water containing allopurinol (50 mg/L) every day, while the other groups were free to drink common drinking water. δM1KH group, δM2KH group and δM4K group were free to take mouse diet containing δM1KH, δM2KH and δM4K daily (1.14 w/w% δM1KH, δM2KH or δM4K were added to the basic mouse diet, respectively), and other groups were free to take the basic mouse diet. At 9 am on the 8th day after the start of the experiment, 4 h after the intraperitoneal injection (i.e. 1 pm), 100-200 $\mu$L of blood was collected from the fundus vein of each group of mice, and serum was obtained by centrifugation. The serum uric acid level was measured using a uric acid assay kit.

Results

The result is shown in Fig. 22. Fig. 22 shows that after oteracil potassium and hypoxanthine were administered to mice for 7 consecutive days, the serum uric acid level of the mice increased significantly. In comparison, administration of benzbromarone, allopurinol or manganese oxide significantly reduced the serum uric acid level in mice, among which the ability of manganese oxide to reduce the serum uric acid level in mice was better than that of benzbromarone and comparable to that of allopurinol. The above results indicate that the administration of manganese oxide (e.g. δ-form manganese oxide) can significantly reduce the increase in blood uric acid levels in oteracil potassium and hypoxanthine induced mice.

### 4.4.5 Experiment on oteracil potassium and uric acid induced hyperuricemia model mice

Oteracil potassium is a urate oxidase inhibitor, which can inhibit the further metabolism of uric acid in mice, thereby increasing the concentration of uric acid in the blood of mice. Administration of uric acid by gavage can increase intestinal uric acid uptake and also elevate uric acid levels in the blood. In this example, the healthy mice were randomly divided into 5 groups, the mouse hyperuricemia model was established by administering oteracil potassium (via intraperitoneal injection) and uric acid (by gavage) to the healthy mice, and 1 group of healthy mice injected intraperitoneally with normal saline and gavaged with SIF were used as the blank control group. The mice of the experimental groups were given the corresponding experimental substances according to the dosing regimen described in the following experimental method. Sixty minutes after completion of gavage, blood was drawn from the mice to prepare serum, and the uric acid content in the serum was tested to examine the effect of the experimental substances on the uric acid level in the blood of oteracil potassium and uric acid induced hyperuricemia model mice.

Experimental method

Thirty 6-week-old male KM mice were randomly divided into 5 groups, with 6 mice in each group, including blank control group, oteracil potassium group, hyperuricemia model group, sevelamer hydrochloride group and δM4K group. The blank control group was first intraperitoneally injected with normal saline, and then immediately gavaged with SIF. The oteracil potassium group was first intraperitoneally injected with oteracil potassium solution (300 mg/kg), and then immediately gavaged with SIF. The hyperuricemia model group was first intraperitoneally injected with oteracil potassium solution (300 mg/kg), and then immediately gavaged with SIF solution containing uric acid (30 mg/kg) to induce hyperuricemia. The sevelamer hydrochloride group was first intraperitoneally injected with oteracil potassium solution (300 mg/kg), and then immediately gavaged with SIF suspension containing uric acid and sevelamer hydrochloride (uric acid dose 30 mg/kg, sevelamer hydrochloride dose 50 mg/kg). The δM4K group was first intraperitoneally injected with oteracil potassium solution (300 mg/kg), and then immediately gavaged with SIF suspension containing uric acid and δM4K (uric acid dose 30 mg/kg, δM4K dose 50 mg/kg). The intraperitoneal injection and gavage operations for each mouse need to be completed consecutively, that is, the intraperitoneal injection and gavage operations for the first mouse must be completed consecutively, and then the corresponding operations for the second mouse are performed. For each mouse, venous blood was collected into a micro-blood collection tube 60 min after the completion of gavage, and centrifuged at 3000g for 15 min at 4°C. The serum uric acid level was measured using a uric acid assay kit.

Results

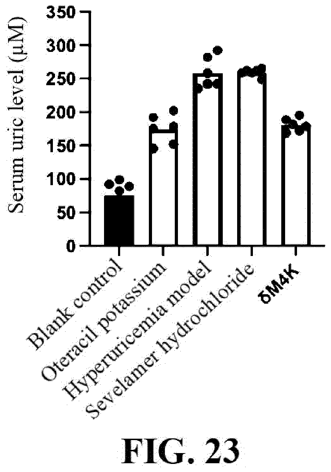

The result is shown in Fig. 23. Fig. 23 shows that after oteracil potassium or a combination of oteracil potassium and uric acid was administered to mice, the serum uric acid level of the mice was significantly increased. Administration of sevelamer hydrochloride had little effect on serum uric acid levels in mice. In contrast, administration of manganese oxide significantly reduced serum uric acid levels in mice. The above results indicate that the administration of manganese oxide (e.g. δ-form manganese oxide) can significantly reduce the increase of blood uric acid levels in oteracil potassium and uric acid induced mice.

### Example 5 In vivo safety evaluation for manganese-containing compounds

Experimental method

In this example, 8-week-old healthy C57BL/6J mice (half male and half female) were used as model animals to evaluate the safety of in vivo administration of manganese-containing compounds. Specifically, the mice were randomly divided into a control group (administered with normal saline), a low-dose group (administered with 250 mg/kg/day of δM4K), a medium-dose group (administered with 500 mg/kg/day of δM4K), a high-dose group (administered with 1000 mg/kg/day of δM4K), and an ultra-high-dose group (administered with 2000 mg/kg/day of δM4K), and then various experimental substances were administered by gavage to the mice in each group for 14 consecutive days according to the above-mentioned dosing regimen. During the experiment, the changes in body weight (g), food intake (g) and water intake (g) of mice in each group were monitored over time. After the experiment, blood was drawn from mice in each group for blood biochemical examination, then the mice were killed, and the heart, kidney, blood cells, spleen, liver, brain, lung and hair were separated and the Mn content (mg/kg) therein was determined.

Results

The results show that during the administration of manganese oxide by gavage for 14 consecutive days, there were no abnormalities in the mice's body weight, food intake, and water intake. After the experiment, it was found that there was no significant difference in the blood biochemical indexes (including WBC, RBC, HGB, W-MCR, W-LCR, W-SCC, HCT, MCV, MCH, W-MCC, W-LCC, RDW-SD, MCHC, PLT, W-SCR and RDW-CV) and the Mn content in various tissues (except the liver) of mice compared with the control group. Although a slight increase in liver Mn content was observed compared with the control group, further studies found that the liver Mn content returned to the same level as the control group 7 days after drug withdrawal. This indicates that manganese oxide is rarely absorbed by mice and can be quickly cleared without any accumulation effect in the body. In summary, the above results suggest that oral administration of manganese oxide (e.g. $\delta$-form manganese oxide) has high in vivo safety.

**Claims**

1.   Use of an insoluble manganese-containing compound in the preparation of a drug for preventing and/or treating increased uric acid levels in the blood and/or digestive tract or a disease or disorder associated with increased uric acid levels in the blood and/or digestive tract in a mammalian subject.

2.   The use of claim 1, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of water and saturated as measured at about 37°C and about 1 atmospheric pressure.

3.   The use of claim 1 or 2, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of simulated intestinal fluid (SIF) and saturated as measured at about 37°C and about 1 atmospheric pressure.

4.   The use of any one of claims 1-3, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fasted state simulated intestinal fluid (FaSSIF) and saturated as measured at about 37°C and about 1 atmospheric pressure.

5.   The use of any one of claims 1-4, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fed state simulated intestinal fluid (FeSSIF) and saturated as measured at about 37°C and about 1 atmospheric pressure.

6.   The use of any one of claims 1-5, wherein the insoluble manganese-containing compound is a manganese-containing compound that enters the blood circulation by absorption in the gastrointestinal tract in an amount of less than about 20%, 15%, 10%, 5%, 4%, 3%, 2%, 1%, 0.5%, 0.1%, 0.05%, 0.02%, or 0.01% of the dose administered.

7.   The use of any one of claims 1-6, wherein the insoluble manganese-containing compound is a manganese-containing compound that does not chemically interact with a surrounding substance or tissue in the body after administration, or only less than about 20%, 15%, 10%, 5%, 4%, 3%, 2%, 1%, 0.5%, 0.1%, 0.05%, 0.02%, or 0.01% of the dose administered of the manganese-containing compound chemically interacts with a surrounding substance or tissue in the body.

8.   The use of any one of claims 1-7, wherein the particle size of the insoluble manganese-containing compound is $\geq 1$ $\mu$m, e.g. 1 $\mu$m to 1 mm.

9. The use of any one of claims 1-8, whereinthe insoluble manganese-containing compound is selected from one or more of manganese oxide, manganese sulfide, manganese carbonate, manganese silicate, manganese borate, manganese ferricyanate, manganese oxalate, manganese selenite, manganese iodate, manganese tungstate and manganese phosphate, and solvates (e.g. hydrates) thereof.

10. The use of any one of claims 1-9, wherein the insoluble manganese-containing compound is manganese oxide or a solvate (e.g. a hydrate) thereof, wherein the manganese oxide is, for example, selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting only of manganese atoms, hydrogen atoms and oxygen atoms, such as manganese oxide selected from one or both of manganese oxyhydroxide and manganese hydroxide; and (3) manganese oxide consisting of manganese atoms, oxygen atoms, metal atoms and optionally hydrogen atoms, such as salt-type manganese oxide.

11. The use of any one of claims 1-10, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide.

12. The use of any one of claims 1-11, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixibyite, pyrochroite, and manganosite.

13. The use of any one of claims 1-12, wherein the manganese oxide is manganese oxide in the form of a polymorph, wherein the manganese oxide in the form of a polymorph is, for example, selected from one or more of $\alpha$-form crystalline manganese oxide, $\beta$-form crystalline manganese oxide, $\gamma$-form crystalline manganese oxide, $\delta$-form crystalline manganese oxide, $\lambda$-form crystalline manganese oxide, $\varepsilon$-form crystalline manganese oxide, R-form crystalline manganese oxide, amorphous manganese oxide, $\alpha$-form crystalline manganese oxyhydroxide, $\beta$-form crystalline manganese oxyhydroxide, and $\gamma$-form crystalline manganese oxyhydroxide.

14. The use of any one of claims 1-13, wherein the manganese oxide is manganese oxide in the form of a salt-type polymorph, wherein the manganese oxide in the form of a salt-type polymorph is, for example, selected from one or more of salt-type $\alpha$-form crystalline manganese oxide, salt-type $\beta$-form crystalline manganese oxide, salt-type $\gamma$-form crystalline manganese oxide, salt-type $\delta$-form crystalline manganese oxide, salt-type $\lambda$-form crystalline manganese oxide, salt-type $\varepsilon$-form crystalline manganese oxide, salt-type R-form crystalline manganese oxide, and salt-type amorphous manganese oxide, and wherein the salt-type $\alpha$-form crystalline manganese oxide is, for example, selected from one or both of potassium-type $\alpha$-form crystalline manganese oxide and hydrogen-type $\alpha$-form crystalline manganese oxide, the salt-type $\beta$-form crystalline manganese oxide is, for example, selected from one or both of potassium-type $\beta$-form crystalline manganese oxide and hydrogen-type $\beta$-form crystalline manganese oxide, the salt-type $\gamma$-form crystalline manganese oxide is, for example, selected from one or both of ammonium-type $\gamma$-form crystalline manganese oxide and hydrogen-type $\gamma$-form crystalline manganese oxide, the salt-type $\delta$-form crystalline manganese oxide is, for example, selected from one or more of potassium-type $\delta$-form crystalline manganese oxide, hydrogen-type $\delta$-form crystalline manganese oxide, sodium-type $\delta$-form crystalline manganese oxide, ammonium-type $\delta$-form crystalline manganese oxide, calcium-type $\delta$-form crystalline manganese oxide, magnesium-type $\delta$-form crystalline manganese oxide, iron-type $\delta$-form crystalline manganese oxide, ferrous-type $\delta$-form crystalline manganese oxide, zinc-type $\delta$-form crystalline manganese oxide, lanthanum-type $\delta$-form crystalline manganese oxide, bismuth-type $\delta$-form crystalline oxide, lithium-type $\delta$-form crystalline manganese oxide, and silver-type $\delta$-form crystalline oxide, the salt-type $\lambda$-form crystalline manganese oxide is, for example, selected from one or both of lithium-type $\lambda$-form crystalline manganese oxide and hydrogen-type $\lambda$-form crystalline manganese oxide, and the salt-type $\varepsilon$-form crystalline manganese oxide is, for example, selected from one or both of potassium-type $\varepsilon$-form crystalline manganese oxide and hydrogen-type $\varepsilon$-form crystalline manganese oxide, the salt-type R-form crystalline manganese oxide is, for example, hydrogen-type R-form crystalline manganese oxide, and the salt-type amorphous manganese oxide is, for example, selected from one or both of potassium-type amorphous manganese oxide and hydrogen-type amorphous manganese oxide.

15. The use of any one of claims 1-14, wherein the insoluble manganese-containing compound has a uric acid clearance capacity of about 50 mg/g to about 1000 mg/g as measured in a simulated intestinal fluid (SIF) assay.

16. The use of any one of claims 1-15, wherein the insoluble manganese-containing compound has a uric acid clearance capacity of about 100 mg/g to about 900 mg/g as measured in a simulated intestinal fluid (SIF) assay.

17. The use of any one of claims 1-16, wherein the insoluble manganese-containing compound has a uric acid clearance capacity of about 200 mg/g to about 800 mg/g as measured in a simulated intestinal fluid (SIF) assay.

18. The use of any one of claims 1-17, wherein the insoluble manganese-containing compound has a uric acid clearance capacity of about 200 mg/g to about 800 mg/g measured in a fasted state simulated intestinal fluid (FaSSIF) assay.

19. The use of any one of claims 1-18, wherein the insoluble manganese-containing compound has a uric acid clearance capacity of about 200 mg/g to about 800 mg/g measured in a fed state simulated intestinal fluid (FeSSIF) assay.

20. The use of any one of claims 1-19, wherein the drug is an oral formulation.

21. The use of any one of claims 1-20, wherein the oral formulation is a solid oral formulation or a liquid oral formulation.

22. The use of any one of claims 1-21, wherein the solid oral formulation is powder, granule, tablet, capsule, pill, or lozenge.

23. The use of any one of claims 1-22, wherein the drug does not contain an additional therapeutic agent.

24. The use of any one of claims 1-23, wherein the drug comprises an effective amount of an insoluble manganese-containing compound.

25. The use of any one of claims 1-24, wherein the drug comprises about 3 mg to about 3000 mg, about 3 mg to about 1500 mg, about 5 mg to about 500 mg, or about 10 mg to about 200 mg of an insoluble manganese-containing compound.

26. The use of any one of claims 1-25, wherein the mammal is a human.

27. The use of any one of claims 1-26, wherein the digestive tract is the intestinal tract.

28. The use of any one of claims 1-27, wherein the disease or disorder associated with increased uric acid levels in the blood and/or digestive tract is at least one selected from the group consisting of: gout, gout complications, hyperuricemia, higher uric acid levels not reaching levels typically diagnosed as hyperuricemia, cardiovascular disease, diabetes, diabetes-related disorders, insulin resistance, metabolic syndrome, hypothyroidism, hyperparathyroidism, obesity, inflammation, muscle spasms, local swelling, joint pain, malignancies, tumor lysis syndrome, polycythemia vera, cognitive disorder, psoriasis, sarcoidosis, non-alcoholic fatty liver disease, stroke, hemolytic anemia, congenital metabolic genetic errors, poisoning, epididymitis and orchitis, and transplantation of blood, bone marrow, or solid organs.

29. The use of any one of claims 1-28, wherein the gout is acute gout, chronic gout, or refractory gout.

30. The use of any one of claims 1-29, wherein the gout complication is at least one of gouty arthritis, gouty urinary calculus, and gouty nephropathy.

31. The use of any one of claims 1-30, wherein the gouty nephropathy is at least one of nephritis, pyelonephritis, hydronephrosis, uric acid nephrolithiasis, renal dysfunction, renal failure, and uremia.

32. The use of any one of claims 1-31, wherein the cardiovascular disease is at least one of hypertension, coronary heart disease, heart failure, congenital heart disease, deep vein thrombosis, pulmonary embolism, aortic aneurysm, aortic dissection, hyperlipidemia, myocardial infarction and atherosclerosis.

33. The use of any one of claims 1-32, wherein the hyperuricemia is primary hyperuricemia or secondary hyperuricemia.

34. The use of any one of claims 1-33, wherein the secondary hyperuricemia is at least one of drug-related hyperuricemia and hyperuricemia related to other medical conditions.

35. The use of any one of claims 1-34, wherein the diabetes-related condition is at least one of diabetic nephropathy, diabetic peripheral neuropathy, diabetic retinopathy, diabetic macroangiopathy, diabetic microangiopathy, diabetic foot, and diabetic ketoacidosis.

36. The use of any one of claims 1-35, wherein the malignancy is a hematological malignancy.

37. The use of any one of claims 1-36, wherein the hematological malignancy is at least one of leukemia and multiple myeloma.

38. The use of any one of claims 1-37, wherein the nonalcoholic fatty liver disease is nonalcoholic steatohepatitis.

39. The use of any one of claims 1-38, wherein the poisoning is at least one of chloroform poisoning, carbon tetrachloride poisoning, and lead poisoning.

40. The use of any one of claims 1-39, wherein the congenital metabolic genetic error is Lesch-Nyhan syndrome.

41. The use of any one of claims 1-40, wherein prior to administration of the drug to the subject, the subject's blood and/or serum uric acid level is higher than 240 $\mu$mol/L, 260 $\mu$mol/L, 280 $\mu$mol/L, 300 $\mu$mol/L, 320 $\mu$mol/L, 340 $\mu$mol/L, 360 $\mu$mol/L, 380 $\mu$mol/L, 400 $\mu$mol/L or 420 $\mu$mol/L by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100%.

42. The use of any one of claims 1-41, wherein the subject's blood and/or serum uric acid level is reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% after administration of the drug compared to that prior to administration of the drug.

43. The use of any one of claims 1-42, wherein the subject's blood and/or serum uric acid level after administration of the drug is between 80 $\mu$mol/L and 460 $\mu$mol/L, between 100 $\mu$mol/L and 450 $\mu$mol/L, between 150 $\mu$mol/L and 440 $\mu$mol/L, between 200 $\mu$mol/L and 430 $\mu$mol/L, between 210 $\mu$mol/L and 420 $\mu$mol/L, between 80 $\mu$mol/L and 380 $\mu$mol/L, between 100 $\mu$mol/L and 370 $\mu$mol/L or between 150 $\mu$mol/L and 360 $\mu$mol/L.

44. Use of a manganese-containing compound in the preparation of a drug for adsorbing uric acid in the digestive tract of mammals.

45. The use of claim 44, wherein the manganese-containing compound is an insoluble manganese-containing compound.

46. The use of claim 44 or 45, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of water and saturated as measured at about 37°C and about 1 atmospheric pressure.

47. The use of any one of claims 44-46, wherein the insoluble manganese-containing compound is a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng or 1 ng when dissolved in 1 mL of simulated intestinal fluid (SIF) and saturated as measured at about 37°C and about 1 atmospheric pressure.

48. The use of any one of claims 44-47, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g, 5 $\mu$g, 1 $\mu$g, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fasted state simulated intestinal fluid (FaSSIF) and saturated as measured at about 37°C and about 1 atmospheric pressure.

49. The use of any one of claims 44-48, wherein the insoluble manganese-containing compound refers to a manganese-containing compound having a mass of less than about 100 $\mu$g, 75 $\mu$g, 50 $\mu$g, 40 $\mu$g, 30 $\mu$g, 25 $\mu$g, 20 $\mu$g, 15 $\mu$g, 10 $\mu$g,

5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fed state simulated intestinal fluid (FeSSIF) and saturated as measured at about 37°C and about 1 atmospheric pressure.

50. The use of any one of claims 44-49, wherein the insoluble manganese-containing compound is a manganese-containing compound that enters the blood circulation by absorption in the gastrointestinal tract in an amount less than about 20%, 15%, 10%, 5%, 4%, 3%, 2%, 1%, 0.5%, 0.1%, 0.05%, 0.02%, or 0.01% of the dose administered.

51. The use of any one of claims 44-50, wherein the insoluble manganese-containing compound is a manganese-containing compound that does not chemically interact with a surrounding substance or tissue in the body after administration or only less than about 20%, 15%, 10%, 5%, 4%, 3%, 2%, 1%, 0.5%, 0.1%, 0.05%, 0.02%, or 0.01% of the dose administered of the manganese-containing compound chemically interacts with a surrounding substance or tissue in the body.

52. The use of any one of claims 44-51, wherein the particle size of the insoluble manganese-containing compound is ≥1 μm, e.g. 1 μm to 1 mm.

53. The use of any one of claims 44-52, wherein the insoluble manganese-containing compound is selected from one or more of manganese oxide, manganese sulfide, manganese carbonate, manganese silicate, manganese borate, manganese ferricyanate, manganese oxalate, manganese selenite, manganese iodate, manganese tungstate and manganese phosphate, and solvates (e.g. hydrates) thereof.

54. The use of any one of claims 44-53, wherein the insoluble manganese-containing compound is manganese oxide or a solvate (e.g. a hydrate) thereof, wherein the manganese oxide is, for example, selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting only of manganese atoms, hydrogen atoms and oxygen atoms, such as manganese oxide selected from one or both of manganese oxyhydroxide and manganese hydroxide; and (3) manganese oxide consisting of manganese atoms, oxygen atoms, metal atoms and optionally hydrogen atoms, such as salt-type manganese oxide.

55. The use of any one of claims 44-54, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide.

56. The use of any one of claims 44-55, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixibyite, pyrochroite, and manganosite.

57. The use of any one of claims 44-56, wherein the manganese oxide is manganese oxide in the form of a polymorph, wherein the manganese oxide in the form of a polymorph is, for example, selected from one or more of α-form crystalline manganese oxide, β-form crystalline manganese oxide, γ-form crystalline manganese oxide, δ-form crystalline manganese oxide, λ-form crystalline manganese oxide, ε-form crystalline manganese oxide, R-form crystalline manganese oxide, amorphous manganese oxide, α-form crystalline manganese oxyhydroxide, β-form crystalline manganese oxyhydroxide, and γ-form crystalline manganese oxyhydroxide.

58. The use of any one of claims 44-57, wherein the manganese oxide is manganese oxide in the form of a salt-type polymorph, wherein the manganese oxide in the form of a salt-type polymorph is, for example, selected from one or more of salt-type α-form crystalline manganese oxide, salt-type β-form crystalline manganese oxide, salt-type γ-form crystalline manganese oxide, salt-type δ-form crystalline manganese oxide, salt-type λ-form crystalline manganese oxide, salt-type ε-form crystalline manganese oxide, salt-type R-form crystalline manganese oxide, and salt-type amorphous manganese oxide, and wherein the salt-type α-form crystalline manganese oxide is, for example, selected from one or both of potassium-type α-form crystalline manganese oxide and hydrogen-type α-form crystalline manganese oxide, the salt-type β-form crystalline manganese oxide is, for example, selected from one or both of potassium-type β-form crystalline manganese oxide and hydrogen-type β-form crystalline manganese oxide, the salt-type γ-form crystalline manganese oxide is, for example, selected from one or both of ammonium-type γ-form crystalline manganese oxide and hydrogen-type γ-form crystalline manganese oxide, and the salt-type δ-form crystalline manganese oxide is, for example, selected from potassium-type δ-form crystalline manganese oxide, hydrogen-type δ-form crystalline manganese oxide, sodium-type δ-form crystalline manganese oxide, ammonium-type δ-form crystalline manganese oxide, calcium-type δ-form crystalline manganese oxide, magnesium-type δ-form crystalline oxide, iron-type δ-form crystalline oxide, Manganese, ferrous δ-form crystalline manganese oxide, zinc δ-form crystalline manganese oxide, one or more of lanthanum-type δ-form crystalline manganese oxide, bismuth-type δ-form crystalline manganese oxide, lithium-type δ-form crystalline manganese oxide, and silver-type δ-form crystalline manganese oxide, the salt-type λ-form crystalline manganese oxide is, for example, selected from one or both of lithium-type λ-form crystalline manganese oxide and hydrogen-type λ-form crystalline manganese oxide, the salt-type ε-form crystalline manganese oxide is, for example, selected from one or both of potassium-type ε-form crystalline manganese oxide and hydrogen-type ε-form crystalline manganese oxide, the salt-type R-form crystalline manganese oxide is, the salt-type amorphous manganese oxide is, for example, selected from one or both of potassium-type amorphous manganese oxide and hydrogen-type amorphous manganese oxide.

59. The use of any one of claims 44-58, wherein the insoluble manganese-containing compound has a uric acid clearance capacity of about 50 mg/g to about 1000 mg/g as measured in a simulated intestinal fluid (SIF) assay.

60. The use of any one of claims 44-59, wherein the insoluble manganese-containing compound has a uric acid clearance capacity of about 100 mg/g to about 900 mg/g as measured in a simulated intestinal fluid (SIF) assay.

61. The use of any one of claims 44-60, wherein the insoluble manganese-containing compound has a uric acid clearance capacity of about 200 mg/g to about 800 mg/g as measured in a simulated intestinal fluid (SIF) assay.

62. The use of any one of claims 44-61, wherein the insoluble manganese-containing compound has a uric acid clearance capacity of about 200 mg/g to about 800 mg/g as measured in a fasted state simulated intestinal fluid (FaSSIF) assay.

63. The use of any one of claims 44-62, wherein the insoluble manganese-containing compound has a uric acid clearance capacity of about 200 mg/g to about 800 mg/g measured in a fed state simulated intestinal fluid (FeSSIF) assay.

64. The use of any one of claims 44-63, wherein the drug is an oral formulation.

65. The use of any one of claims 44-64, wherein the oral formulation is a solid oral formulation or a liquid oral formulation.

66. The use of any one of claims 44-65, wherein the solid oral formulation is powder, granule, tablet, capsule, pill, or lozenge.

67. The use of any one of claims 44-66, wherein the drug does not contain an additional therapeutic agent.

68. The use of any one of claims 44-67, wherein the drug comprises an effective amount of an insoluble manganese-containing compound.

69. The use of any one of claims 44-68, wherein the drug comprises about 3 mg to about 3000 mg, about 3 mg to about 1500 mg, about 5 mg to about 500 mg, or about 10 mg to about 200 mg of an insoluble manganese-containing compound.

70. The use of any one of claims 44-69, wherein the mammal is a human.

71. The use of any one of claims 44-70, wherein the digestive tract is the intestinal tract.

72. A pharmaceutical composition for preventing and/or treating a disease or disorder associated with increased uric acid levels in the blood and/or digestive tract of a mammalian subject, wherein the pharmaceutical composition comprises an insoluble manganese-containing compound and a pharmaceutically acceptable excipient.

73. The pharmaceutical composition of claim 71, wherein the insoluble manganese-containing compound refers to a manganese-containing compound having a mass of less than about 100 μg, 75 μg, 50 μg, 40 μg, 30 μg, 25 μg, 20 μg,

15 µg, 10 µg, 5 µg, 1 µg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of water and saturated as measured at about 37°C and about 1 atmospheric pressure.

74. The pharmaceutical composition of claim 72 or 73, wherein the insoluble manganese-containing compound is a manganese-containing compound having a mass of less than about 100 µg, 75 µg, 50 µg, 40 µg, 30 µg, 25 µg, 20 µg, 15 µg, 10 µg, 5 µg, 1 µg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng or 1 ng when dissolved in 1 mL of simulated intestinal fluid (SIF) and saturated as measured at about 37°C and about 1 atmospheric pressure.

75. The pharmaceutical composition of any one of claims 72-74, wherein the insoluble manganese-containing compound refers to a manganese-containing compound having a mass of less than about 100 µg, 75 µg, 50 µg, 40 µg, 30 µg, 25 µg, 20 µg, 15 µg, 10 µg, 5 µg, 1 µg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fasted state simulated intestinal fluid (FaSSIF) and saturated as measured at about 37°C and about 1 atmospheric pressure.

76. The pharmaceutical composition of any one of claims 72-75, wherein the insoluble manganese-containing compound refers to a manganese-containing compound having a mass of less than about 100 µg, 75 µg, 50 µg, 40 µg, 30 µg, 25 µg, 20 µg, 15 µg, 10 µg, 5 µg, 1 µg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fed state simulated intestinal fluid (FeSSIF) and saturated as measured at about 37°C and about 1 atmospheric pressure.

77. The pharmaceutical composition of any one of claims 72-76, wherein the insoluble manganese-containing compound is a manganese-containing compound that enters the blood circulation by absorption in the gastrointestinal tract in an amount less than about 20%, 15%, 10%, 5%, 4%, 3%, 2%, 1%, 0.5%, 0.1%, 0.05%, 0.02%, or 0.01% of the dose administered.

78. The pharmaceutical composition of any one of claims 72-77, wherein the insoluble manganese-containing compound is a manganese-containing compound that does not chemically interact with a surrounding substance or tissue in the body after administration or only less than about 20%, 15%, 10%, 5%, 4%, 3%, 2%, 1%, 0.5%, 0.1%, 0.05%, 0.02%, or 0.01% of the dose administered of the manganese-containing compound chemically interacts with a surrounding substance or tissue in the body.

79. The pharmaceutical composition of any one of claims 72-78, wherein the particle size of the insoluble manganese-containing compound is ≥1 µm, e.g. 1 µm to 1 mm.

80. The pharmaceutical composition of any one of claims 72-79, wherein the insoluble manganese-containing compound is selected from one or more of manganese oxide, manganese sulfide, manganese carbonate, manganese silicate, manganese borate, manganese ferricyanate, manganese oxalate, manganese selenite, manganese iodate, manganese tungstate and manganese phosphate, and solvates (e.g. hydrates) thereof.

81. The pharmaceutical composition of any one of claims 72-80, wherein the insoluble manganese-containing compound is manganese oxide or a solvate (e.g. a hydrate) thereof, wherein the manganese oxide is, for example, selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting only of manganese atoms, hydrogen atoms and oxygen atoms, such as manganese oxide selected from one or both of manganese oxyhydroxide and manganese hydroxide; and (3) manganese oxide consisting of manganese atoms, oxygen atoms, metal atoms and optionally hydrogen atoms, such as salt-type manganese oxide.

82. The pharmaceutical composition of any one of claims 72-81, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide.

83. The pharmaceutical composition of any one of claims 72-82, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixibyite, pyrochroite, and manganosite.

84. The pharmaceutical composition of any one of claims 72-83, wherein the manganese oxide is manganese oxide in the form of a polymorph, wherein the manganese oxide in the form of a polymorph is, for example, selected from one or more of $\alpha$-form crystalline manganese oxide, $\beta$-form crystalline manganese oxide, $\gamma$-form crystalline manganese oxide, $\delta$-form crystalline manganese oxide, $\lambda$-form crystalline manganese oxide, $\epsilon$-form crystalline manganese oxide, R-form crystalline manganese oxide, amorphous manganese oxide, $\alpha$-form crystalline manganese oxyhydroxide, $\beta$-form crystalline manganese oxyhydroxide, and $\gamma$-form crystalline manganese oxyhydroxide.

85. The pharmaceutical composition of any one of claims 72-84, wherein the manganese oxide is manganese oxide in the form of a salt-type polymorph, wherein the manganese oxide in the form of a salt-type polymorph is, for example, selected from one or more of salt-type $\alpha$-form crystalline manganese oxide, salt-type $\beta$-form crystalline manganese oxide, salt-type $\gamma$-form crystalline manganese oxide, salt-type $\delta$-form crystalline manganese oxide, salt-type $\lambda$-form crystalline manganese oxide, salt-type $\epsilon$-form crystalline manganese oxide, salt-type R-form crystalline manganese oxide, and salt-type amorphous manganese oxide, and wherein the salt-type $\alpha$-form crystalline manganese oxide is, for example, selected from one or both of potassium-type $\alpha$-form crystalline manganese oxide and hydrogen-type $\alpha$-form crystalline manganese oxide, the salt-type $\beta$-form crystalline manganese oxide is, for example, selected from one or both of potassium-type $\beta$-form crystalline manganese oxide and hydrogen-type $\beta$-form crystalline manganese oxide, the salt-type $\gamma$-form crystalline manganese oxide is, for example, selected from one or both of ammonium-type $\gamma$-form crystalline manganese oxide and hydrogen-type $\gamma$-form crystalline manganese oxide, and the salt-type $\delta$-form crystalline manganese oxide is, for example, selected from potassium-type $\delta$-form crystalline manganese oxide, hydrogen-type $\delta$-form crystalline manganese oxide, sodium-type $\delta$-form crystalline manganese oxide, ammonium-type $\delta$-form crystalline manganese oxide, calcium-type $\delta$-form crystalline manganese oxide, magnesium-type $\delta$-form crystalline oxide, iron-type $\delta$-form crystalline oxide, Manganese, ferrous $\delta$-form crystalline manganese oxide, zinc $\delta$-form crystalline manganese oxide, one or more of lanthanum-type $\delta$-form crystalline manganese oxide, bismuth-type $\delta$-form crystalline manganese oxide, lithium-type $\delta$-form crystalline manganese oxide, and silver-type $\delta$-form crystalline manganese oxide, the salt-type $\lambda$-form crystalline manganese oxide is, for example, selected from one or both of lithium-type $\lambda$-form crystalline manganese oxide and hydrogen-type $\lambda$-form crystalline manganese oxide, the salt-type $\epsilon$-form crystalline manganese oxide is, for example, selected from one or both of potassium-type $\epsilon$-form crystalline manganese oxide and hydrogen-type $\epsilon$-form crystalline manganese oxide, the salt-type R-form crystalline manganese oxide is, the salt-type amorphous manganese oxide is, for example, selected from one or both of potassium-type amorphous manganese oxide and hydrogen-type amorphous manganese oxide.

86. The pharmaceutical composition of any one of claims 72-85, wherein the insoluble manganese-containing compound has a uric acid clearance capacity of about 50 mg/g to about 1000 mg/g as measured in a simulated intestinal fluid (SIF) assay.

87. The pharmaceutical composition of any one of claims 72-86, wherein the insoluble manganese-containing compound has a uric acid clearance capacity of about 100 mg/g to about 900 mg/g as measured in a simulated intestinal fluid (SIF) assay.

88. The pharmaceutical composition of any one of claims 72-87, wherein the insoluble manganese-containing compound has a uric acid clearance capacity of about 200 mg/g to about 800 mg/g as measured in a simulated intestinal fluid (SIF) assay.

89. The pharmaceutical composition of any one of claims 72-88, wherein the insoluble manganese-containing compound has a uric acid clearance capacity of about 200 mg/g to about 800 mg/g measured in a fasted state simulated intestinal fluid (FaSSIF) assay.

90. The pharmaceutical composition of any one of claims 72-89, wherein the insoluble manganese-containing compound has a uric acid clearance capacity of about 200 mg/g to about 800 mg/g measured in a fed state simulated intestinal fluid (FeSSIF) assay.

91. The pharmaceutical composition of any one of claims 72-90, wherein the pharmaceutical composition is an oral formulation.

**92.** The pharmaceutical composition of any one of claims 72-91, wherein the oral formulation is a solid oral formulation or a liquid oral formulation.

**93.** The pharmaceutical composition of any one of claims 72-92, wherein the solid oral formulation is powder, granule, tablet, capsule, pill, or lozenge.

**94.** The pharmaceutical composition of any one of claims 72-93, wherein the pharmaceutical composition does not comprise an additional therapeutic agent.

**95.** The pharmaceutical composition of any one of claims 72-94, wherein the pharmaceutical composition comprises an effective amount of an insoluble manganese-containing compound.

**96.** The pharmaceutical composition of any one of claims 72-95, wherein the pharmaceutical composition comprises about 3 mg to about 3000 mg, about 3 mg to about 1500 mg, about 5 mg to about 500 mg, or about 10 mg to about 200 mg of an insoluble manganese-containing compound.

**97.** The pharmaceutical composition of any one of claims 72-96, wherein the mammal is a human.

**98.** The pharmaceutical composition of any one of claims 72-97, wherein the digestive tract is the intestinal tract.

**99.** The pharmaceutical composition to any one of claims 72-98, wherein the disease or disorder associated with increased uric acid levels in the blood and/or digestive tract is at least one selected from the group consisting of: gout, gout complications, hyperuricemia, higher uric acid levels not reaching levels typically diagnosed as hyperuricemia, cardiovascular disease, diabetes, diabetes-related disorders, insulin resistance, metabolic syndrome, hypothyroidism, hyperparathyroidism, obesity, inflammation, muscle spasms, local swelling, joint pain, malignancies, tumor lysis syndrome, polycythemia vera, cognitive disorder, psoriasis, sarcoidosis, non-alcoholic fatty liver disease, stroke, hemolytic anemia, congenital metabolic errors, poisoning, epididymitis and orchitis, and transplantation of blood, bone marrow, or solid organs.

**100.**
The pharmaceutical composition of any one of claims 72-99, wherein the gout is acute gout, chronic gout, or refractory gout.

**101.**
The pharmaceutical composition of any one of claims 72-100, wherein the gout complication is at least one of gouty arthritis, gouty urinary calculus, and gouty nephropathy.

**102.**
The pharmaceutical composition of any one of claims 72-101, wherein the gouty nephropathy is at least one of nephritis, pyelonephritis, hydronephrosis, uric acid nephrolithiasis, renal dysfunction, renal failure, and uremia.

**103.**
The pharmaceutical composition of any one of claims 72-102, wherein the cardiovascular disease is at least one of hypertension, coronary heart disease, heart failure, congenital heart disease, deep vein thrombosis, pulmonary embolism, aortic aneurysm, aortic dissection, hyperlipidemia, myocardial infarction and atherosclerosis.

**104.**
The pharmaceutical composition of any one of claims 72-103, wherein the hyperuricemia is primary hyperuricemia or secondary hyperuricemia.

**105.**
The pharmaceutical composition of any one of claims 72-104, wherein the secondary hyperuricemia is at least one of drug-related hyperuricemia and hyperuricemia related to other medical conditions.

**106.**
The pharmaceutical composition of any one of claims 72-105, wherein the diabetes-related condition is at least one of diabetic nephropathy, diabetic peripheral neuropathy, diabetic retinopathy, diabetic macroangiopathy, diabetic microangiopathy, diabetic foot, and diabetic ketoacidosis.

**107.**

The pharmaceutical composition of any one of claims 72-106, wherein the malignancy is a hematological malignancy.

**108.**

The pharmaceutical composition of any one of claims 72-107, wherein the hematological malignancy is at least one of leukemia and multiple myeloma.

**109.**

The pharmaceutical composition of any one of claims 72-108, wherein the nonalcoholic fatty liver disease is nonalcoholic steatohepatitis.

**110.**

The pharmaceutical composition of any one of claims 72-109, wherein the poisoning is at least one of chloroform poisoning, carbon tetrachloride poisoning, and lead poisoning.

**111.** The pharmaceutical composition of any one of claims 72-110, wherein the congenital metabolic genetic error is Lesch-Nyhan syndrome.

**112.**

The pharmaceutical composition of any one of claims 72-111, wherein prior to administration of the pharmaceutical composition to the subject, the subject's blood and/or serum uric acid level is higher than 240 $\mu$mol/L, 260 $\mu$mol/L, 280 $\mu$mol/L, 300 $\mu$mol/L, 320 $\mu$mol/L, 340 $\mu$mol/L, 360 $\mu$mol/L, 380 $\mu$mol/L, 400 $\mu$mol/L or 420 $\mu$mol/L by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100%.

**113.**

The pharmaceutical composition of any one of claims 72-112, wherein the subject's blood and/or serum uric acid level is reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% after administration of the pharmaceutical composition compared to that prior to administration of the pharmaceutical composition.

**114.**

The pharmaceutical composition of any one of claims 72-113, wherein the subject's blood and/or serum uric acid level after administration of the pharmaceutical composition is between 80 $\mu$mol/L and 460 $\mu$mol/L, between 100 $\mu$mol/L and 450 $\mu$mol/L, between 150 $\mu$mol/L and 440 $\mu$mol/L, between 200 $\mu$mol/L and 430 $\mu$mol/L, between 210 $\mu$mol/L and 420 $\mu$mol/L, between 80 $\mu$mol/L and 380 $\mu$mol/L, between 100 $\mu$mol/L and 370 $\mu$mol/L or between 150 $\mu$mol/L and 360 $\mu$mol/L.

**115.**
A kit comprising:

(i) the pharmaceutical composition of any one of claims 72-114; and
(ii) drug instructions and/or drug labels.

**116.**

A method for preventing and/or treating increased uric acid levels in the blood and/or digestive tract or a disease or disorder associated with increased uric acid levels in the blood and/or digestive tract in a mammalian subject, comprising administrating an effective amount of an insoluble manganese-containing compound to a subject in need thereof.

**117.**

A method for preventing and/or treating increased uric acid levels in the blood or a disease or disorder associated with increased uric acid levels in the blood in a mammalian subject, comprising administrating an effective amount of an insoluble manganese-containing compound to a subject in need thereof over an extended period of time.

**118.**

A method for maintaining uric acid levels in the blood in a mammalian subject, comprising administering to an subject in need thereof an effective amount of an insoluble manganese-containing compound over an extended period of time.

**119.**

The method of any one of claims 116-118, wherein the method maintains the subject's blood and/or serum uric acid level between 80 μmol/L and 460 μmol/L, between 100 μmol/L and 450 μmol/L, between 150 μmol/L and 440 μmol/L, between 200 μmol/L and 430 μmol/L, between 210 μmol/L and 420 μmol/L, between 80 μmol/L and 380 μmol/L, between 100 μmol/L and 370 μmol/L or between 150 μmol/L and 360 μmol/L.

**120.**
The method of any of claims 116-119, wherein the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of water and saturated as measured at about 37°C and about 1 atmospheric pressure.

**121.**
The method of any one of claims 116-120, wherein the insoluble manganese-containing compound is a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng or 1 ng when dissolved in 1 mL of simulated intestinal fluid (SIF) and saturated as measured at about 37°C and about 1 atmospheric pressure.

**122.**
The method of any one of claims 116-121, wherein the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fasted state simulated intestinal fluid (FaSSIF) and saturated as measured at about 37°C and about 1 atmospheric pressure.

**123.**
The method of any one of claims 116-122, wherein the insoluble manganese-containing compound refers to a manganese-containing compound having a mass of less than about 100 μg, 75 μg, 50 μg, 40 μg, 30 μg, 25 μg, 20 μg, 15 μg, 10 μg, 5 μg, 1 μg, 750 ng, 500 ng, 400 ng, 300 ng, 250 ng, 200 ng, 150 ng, 100 ng, 75 ng, 50 ng, 40 ng, 30 ng, 25 ng, 20 ng, 15 ng, 10 ng, 5 ng, or 1 ng when dissolved in 1 mL of fed state simulated intestinal fluid (FeSSIF) and saturated as measured at about 37°C and about 1 atmospheric pressure.

**124.**
The method of any one of claims 116-123, wherein the insoluble manganese-containing compound is a manganese-containing compound that enters the blood circulation by absorption in the gastrointestinal tract in an amount less than about 20%, 15%, 10%, 5%, 4%, 3%, 2%, 1%, 0.5%, 0.1%, 0.05%, 0.02%, or 0.01% of the dose administered.

**125.**
The method of any one of claims 116-124, wherein the insoluble manganese-containing compound is a manganese-containing compound that does not chemically interact with a surrounding substance or tissue in the body after administration or only less than about 20%, 15%, 10%, 5%, 4%, 3%, 2%, 1%, 0.5%, 0.1%, 0.05%, 0.02%, or 0.01% of the dose administered of the manganese-containing compound chemically interacts with a surrounding substances or tissue in the body.

**126.**
The method of any one of claims 116-125, wherein the particle size of the insoluble manganese-containing compound is ≥1 μm, e.g. 1 μm to 1 mm.

**127.**
The method of any one of claims 116-126, wherein the insoluble manganese-containing compound is selected from one or more of manganese oxide, manganese sulfide, manganese carbonate, manganese silicate, manganese borate, manganese ferricyanate, manganese oxalate, manganese selenite, manganese iodate, manganese tungstate and manganese phosphate, and solvates (e.g. hydrates) thereof.

**128.**
The method of any one of claims 116-127, wherein the insoluble manganese-containing compound is manganese oxide or a solvate (e.g. a hydrate) thereof, wherein the manganese oxide is, for example, selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting solely of manganese atoms, hydrogen atoms and oxygen atoms, such as manganese oxide selected from one or both of manganese oxyhydroxide and manganese hydroxide; and (3) Manganese oxide consisting of manganese atoms, oxygen atoms, metal atoms and optionally hydrogen atoms, such as salt-type manganese oxide.

**129.**

The method of any one of claims 116-128, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide.

**130.**

The method of any one of claims 116-129, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixbyite, pyrochroite, and manganosite.

**131.**

The method of any one of claims 116-130, wherein the manganese oxide is manganese oxide in the form of a polymorph, wherein the manganese oxide in the form of a polymorph is, for example, selected from one or more of $\alpha$-form crystalline manganese oxide, $\beta$-form crystalline manganese oxide, $\gamma$-form crystalline manganese oxide, $\delta$-form crystalline manganese oxide, $\lambda$-form crystalline manganese oxide, $\varepsilon$-form crystalline manganese oxide, R-form crystalline manganese oxide, amorphous manganese oxide, $\alpha$-form crystalline manganese oxyhydroxide, $\beta$-form crystalline manganese oxyhydroxide, and $\gamma$-form crystalline manganese oxyhydroxide.

**132.**

The method of any one of claims 116-131, wherein the manganese oxide is manganese oxide in the form of a salt-type polymorph, wherein the manganese oxide in the form of a salt-type polymorph is, for example, selected from one or more of salt-type $\alpha$-form crystalline manganese oxide, salt-type $\beta$-form crystalline manganese oxide, salt-type $\gamma$-form crystalline manganese oxide, salt-type $\delta$-form crystalline manganese oxide, salt-type $\lambda$-form crystalline manganese oxide, salt-type $\varepsilon$-form crystalline manganese oxide, salt-type R-form crystalline manganese oxide, and salt-type amorphous manganese oxide, and wherein the salt-type $\alpha$-form crystalline manganese oxide is, for example, selected from one or both of potassium-type $\alpha$-form crystalline manganese oxide and hydrogen-type $\alpha$-form crystalline manganese oxide, the salt-type $\beta$-form crystalline manganese oxide is, for example, selected from one or both of potassium-type $\beta$-form crystalline manganese oxide and hydrogen-type $\beta$-form crystalline manganese oxide, the salt-type $\gamma$-form crystalline manganese oxide is, for example, selected from one or both of ammonium-type $\gamma$-form crystalline manganese oxide and hydrogen-type $\gamma$-form crystalline manganese oxide, and the salt-type $\delta$-form crystalline manganese oxide is, for example, selected from potassium-type $\delta$-form crystalline manganese oxide, hydrogen-type $\delta$-form crystalline manganese oxide, sodium-type $\delta$-form crystalline manganese oxide, ammonium-type $\delta$-form crystalline manganese oxide, calcium-type $\delta$-form crystalline manganese oxide, magnesium-type $\delta$-form crystalline oxide, iron-type $\delta$-form crystalline oxide, Manganese, ferrous $\delta$-form crystalline manganese oxide, zinc $\delta$-form crystalline manganese oxide, one or more of lanthanum-type $\delta$-form crystalline manganese oxide, bismuth-type $\delta$-form crystalline manganese oxide, lithium-type $\delta$-form crystalline manganese oxide, and silver-type $\delta$-form crystalline manganese oxide, the salt-type $\lambda$-form crystalline manganese oxide is, for example, selected from one or both of lithium-type $\lambda$-form crystalline manganese oxide and hydrogen-type $\lambda$-form crystalline manganese oxide, the salt-type $\varepsilon$-form crystalline manganese oxide is, for example, selected from one or both of potassium-type $\varepsilon$-form crystalline manganese oxide and hydrogen-type $\varepsilon$-form crystalline manganese oxide, the salt-type R-form crystalline manganese oxide is, the salt-type amorphous manganese oxide is, for example, selected from one or both of potassium-type amorphous manganese oxide and hydrogen-type amorphous manganese oxide.

**133.**

The method of any one of claims 116-132, wherein the insoluble manganese-containing compound has a uric acid clearance capacity of about 50 mg/g to about 1000 mg/g as measured in a simulated intestinal fluid (SIF) assay.

**134.**

The method of any one of claims 116-133, wherein the insoluble manganese-containing compound has a uric acid clearance capacity of about 100 mg/g to about 900 mg/g as measured in a simulated intestinal fluid (SIF) assay.

**135.**

The method of any one of claims 116-134, wherein the insoluble manganese-containing compound has a uric acid clearance capacity of about 200 mg/g to about 800 mg/g as measured in a simulated intestinal fluid (SIF) assay.

**136.**

The method of any one of claims 116-135, wherein the insoluble manganese-containing compound has a uric acid clearance capacity of about 200 mg/g to about 800 mg/g as measured in a fasted state simulated intestinal fluid (FaSSIF) assay.

**137.**

The method of any one of claims 116-136, wherein the insoluble manganese-containing compound has a uric acid clearance capacity of about 200 mg/g to about 800 mg/g as measured in a fed state simulated intestinal fluid (FeSSIF) assay.

**138.**

The method of any one of claims 116-137, wherein the effective amount is from about 3 mg to about 3000 mg of manganese oxide per day.

**139.**

The method of any one of claims 116-138, wherein the effective amount is from about 3 mg to about 1500 mg of manganese oxide per day.

**140.**

The method of any one of claims 116-139, wherein the effective amount is from about 5 mg to about 500 mg of manganese oxide per day.

**141.**

The method of any one of claims 116-140, wherein the effective amount is from about 10 mg to about 200 mg of manganese oxide per day.

**142.**

The method of any one of claims 116-141, wherein the insoluble manganese-containing compound is administered orally.

**143.**

The method of any one of claims 116-142, wherein the insoluble manganese-containing compound is formulated as an oral formulation.

**144.**

The method of any one of claims 116-143, wherein the oral formulation is a solid oral formulation or a liquid oral formulation.

**145.**

The method of any one of claims 116-144, wherein the solid oral formulation is powder, granule, tablet, capsule, pill, or lozenge.

**146.**

The method of any one of claims 116-145, wherein the oral formulation comprises an effective amount of an insoluble manganese-containing compound.

**147.**

The method of any one of claims 116-146, wherein the oral formulation comprises about 3 mg to about 3000 mg, about 3 mg to about 1500 mg, about 5 mg to about 500 mg, or about 10 mg to about 200 mg of an insoluble manganese-containing compound.

**148.**

The method of any one of claims 116-147, wherein the insoluble manganese-containing compound is administered to the subject once, twice, or more times daily.

**149.**

The method of any one of claims 116-148, wherein the administration lasts 1 day, 2 days, 3 days, 4 days, 5 days, 6 days, 1 week, 2 weeks, 3 weeks, 1 month, 2 months, 3 months, 4 months, 5 months, 6 months, 8 months, 10 months, 1 year, 1.5 years, 2 years, 2.5 years, 3 years, 4 years, 5 years, 10 years, or more.

**150.**

The method of any one of claims 116-149, wherein the extended period is 1 day, 2 days, 3 days, 4 days, 5 days, 6 days, 1 week, 2 weeks, 3 weeks, 1 month, 2 months, 3 months, 4 months, 5 months, 6 months, 8 months, 10 months, 1 year, 1.5 years, 2 years, 2.5 years, 3 years, 4 years, 5 years, 10 years, or more.

**151.**

The method of any one of claims 116-150, wherein the insoluble manganese-containing compound and the food are taken simultaneously or at intervals of less than 1min, 2min, 3min, 4min, 5min, 10min, 15min, 20min, 25min, or 30min.

**152.**

The method of any one of claims 116-151, wherein the insoluble manganese-containing compound is administered 30 min, 40 min, 50 min, 1h, 1.5h, 2h, or more after meals.

**153.**

The method of any one of claims 116-152, wherein the mammal is a human.

**154.**

The method of any one of claims 116-153, wherein the digestive tract is the intestinal tract.

**155.**

The method of any one of claims 116-154, wherein the disease or disorder associated with increased uric acid levels in the blood and/or digestive tract is at least one selected from the group consisting of: gout, gout complications, hyperuricemia, higher uric acid levels not reaching levels typically diagnosed as hyperuricemia, cardiovascular disease, diabetes, diabetes-related disorders, insulin resistance, metabolic syndrome, hypothyroidism, hyperparathyroidism, obesity, inflammation, muscle spasms, local swelling, joint pain, malignancies, tumor lysis syndrome, polycythemia vera, cognitive disorder, psoriasis, sarcoidosis, non-alcoholic fatty liver disease, stroke, hemolytic anemia, congenital metabolic errors, poisoning, epididymitis and orchitis, and transplantation of blood, bone marrow, or solid organs.

**156.**

The method of any one of claims 116-155, wherein the gout is acute gout, chronic gout, or refractory gout.

**157.**

The method of any one of claims 116-156, wherein the gout complication is at least one of gouty arthritis, gouty urinary calculus, and gouty nephropathy.

**158.**

The method of any one of claims 116-157, wherein the gouty nephropathy is at least one of nephritis, pyelonephritis, hydronephrosis, uric acid nephrolithiasis, renal dysfunction, renal failure, and uremia.

**159.**

The method of any one of claims 116-158, wherein the cardiovascular disease is at least one of hypertension, coronary heart disease, heart failure, congenital heart disease, deep vein thrombosis, pulmonary embolism, aortic aneurysm, aortic dissection, hyperlipidemia, myocardial infarction and atherosclerosis.

**160.**

The method of any one of claims 116-159, wherein the hyperuricemia is primary hyperuricemia or secondary hyperuricemia.

**161.**

The method of any one of claims 116-160, wherein the secondary hyperuricemia is at least one of drug-related hyperuricemia and hyperuricemia related to other medical conditions.

**162.**

The method of any one of claims 116-161, wherein the diabetes-related condition is at least one of diabetic nephropathy, diabetic peripheral neuropathy, diabetic retinopathy, diabetic macroangiopathy, diabetic microangiopathy, diabetic foot, and diabetic ketoacidosis.

**163.**

The method of any one of claims 116-162, wherein the malignancy is a hematological malignancy.

**164.**

The method of any one of claims 116-163, wherein the hematological malignancy is at least one of leukemia and multiple myeloma.

**165.**

The method of any one of claims 116-164, wherein the nonalcoholic fatty liver disease is nonalcoholic steatohepatitis.

**166.**

The method of any one of claims 116-165, wherein the poisoning is at least one of chloroform poisoning, carbon tetrachloride poisoning, and lead poisoning.

**167.**

The method of any one of claims 116-166, wherein the congenital metabolic genetic error is Lesch-Nyhan syndrome.

**168.**

The method of any one of claims 116-167, wherein prior to administration of the insoluble manganese-containing compound to the subject, the subjects blood and/or serum uric acid level is higher than 240 $\mu$mol/L, 260 $\mu$mol/L, 280 $\mu$mol/L, 300 $\mu$mol/L, 320 $\mu$mol/L, 340 $\mu$mol/L, 360 $\mu$mol/L, 380 $\mu$mol/L, 400 $\mu$mol/L or 420 $\mu$mol/L by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100%.

**169.**

The method of any one of claims 116-168, wherein the subjects blood and/or serum uric acid level is reduced by at least about 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or 100% after administration of the insoluble manganese-containing compound compared to that prior to administration of the insoluble manganese-containing compound.

**170.**

The method of any one of claims 116-169, wherein the subjects blood and/or serum uric acid level after administration of the insoluble manganese-containing compound is between 80 $\mu$mol/L and 460 $\mu$mol/L, between 100 $\mu$mol/L and 450 $\mu$mol/L, between 150 $\mu$mol/L and 440 $\mu$mol/L, between 200 $\mu$mol/L and 430 $\mu$mol/L, between 210 $\mu$mol/L and 420 $\mu$mol/L, between 80 $\mu$mol/L and 380 $\mu$mol/L, between 100 $\mu$mol/L and 370 $\mu$mol/L or between 150 $\mu$mol/L and 360 $\mu$mol/L.

**171.**

A pharmaceutical composition comprising manganese oxide, wherein the amount of manganese oxide is sufficient to reduce blood and/or serum uric acid levels by at least about 5% in a mammalian subject whose blood and/or serum uric acid levels are at least 10% higher than 360 $\mu$mol/L or 420 $\mu$mol/L within 24 hours after administration of the pharmaceutical composition.

**172.**

The pharmaceutical composition of claim 171, wherein the particle size of manganese oxide is $\geq$1 $\mu$m, e.g. 1 $\mu$m to 1 mm.

**173.**

The pharmaceutical composition of claim 171 or 172, wherein the manganese oxide is selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting solely of manganese atoms, hydrogen atoms and oxygen atoms, such as manganese oxide selected from one or both of manganese oxyhydroxide and manganese hydroxide; and (3) manganese oxide consisting of manganese atoms, oxygen atoms, metal atoms and optionally hydrogen atoms, such as salt-type manganese oxide.

**174.**
The pharmaceutical composition of any one of claims 171-173, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type manganese oxide.

**175.**
The pharmaceutical composition of any one of claims 171-174, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixbyite, pyrochroite, and manganosite.

**176.**
The pharmaceutical composition of any one of claims 171-175, wherein the manganese oxide is manganese oxide in the form of a polymorph, wherein the manganese oxide in the form of a polymorph is, for example, selected from one or more of $\alpha$-form crystalline manganese oxide, $\beta$-form crystalline manganese oxide, $\gamma$-form crystalline manganese oxide, $\delta$-form crystalline manganese oxide, $\lambda$-form crystalline manganese oxide, $\varepsilon$-form crystalline manganese oxide, R-form crystalline manganese oxide, amorphous manganese oxide, $\alpha$-form crystalline manganese oxyhydroxide, $\beta$-form crystalline manganese oxyhydroxide, and $\gamma$-form crystalline manganese oxyhydroxide.

**177.**
The pharmaceutical composition of any one of claims 171-176, wherein the manganese oxide is manganese oxide in the form of a salt-type polymorph, wherein the manganese oxide in the form of a salt-type polymorph is, for example, selected from one or more of salt-type $\alpha$-form crystalline manganese oxide, salt-type $\beta$-form crystalline manganese oxide, salt-type $\gamma$-form crystalline manganese oxide, salt-type $\delta$-form crystalline manganese oxide, salt-type $\lambda$-form crystalline manganese oxide, salt-type $\varepsilon$-form crystalline manganese oxide, salt-type R-form crystalline manganese oxide, and salt-type amorphous manganese oxide, and wherein the salt-type $\alpha$-form crystalline manganese oxide is, for example, selected from one or both of potassium-type $\alpha$-form crystalline manganese oxide and hydrogen-type $\alpha$-form crystalline manganese oxide, the salt-type $\beta$-form crystalline manganese oxide is, for example, selected from one or both of potassium-type $\beta$-form crystalline manganese oxide and hydrogen-type $\beta$-form crystalline manganese oxide, the salt-type $\gamma$-form crystalline manganese oxide is, for example, selected from one or both of ammonium-type $\gamma$-form crystalline manganese oxide and hydrogen-type $\gamma$-form crystalline manganese oxide, and the salt-type $\delta$-form crystalline manganese oxide is, for example, selected from potassium-type $\delta$-form crystalline manganese oxide, hydrogen-type $\delta$-form crystalline manganese oxide, sodium-type $\delta$-form crystalline manganese oxide, ammonium-type $\delta$-form crystalline manganese oxide, calcium-type $\delta$-form crystalline manganese oxide, magnesium-type $\delta$-form crystalline oxide, iron-type $\delta$-form crystalline oxide, Manganese, ferrous $\delta$-form crystalline manganese oxide, zinc $\delta$-form crystalline manganese oxide, one or more of lanthanum-type $\delta$-form crystalline manganese oxide, bismuth-type $\delta$-form crystalline manganese oxide, lithium-type $\delta$-form crystalline manganese oxide, and silver-type $\delta$-form crystalline manganese oxide, the salt-type $\lambda$-form crystalline manganese oxide is, for example, selected from one or both of lithium-type $\lambda$-form crystalline manganese oxide and hydrogen-type $\lambda$-form crystalline manganese oxide, the salt-type $\varepsilon$-form crystalline manganese oxide is, for example, selected from one or both of potassium-type $\varepsilon$-form crystalline manganese oxide and hydrogen-type $\varepsilon$-form crystalline manganese oxide, the salt-type R-form crystalline manganese oxide is, the salt-type amorphous manganese oxide is, for example, selected from one or both of potassium-type amorphous manganese oxide and hydrogen-type amorphous manganese oxide.

**178.**
The pharmaceutical composition of any one of claims 171-177, wherein the manganese oxide has a uric acid clearance capacity of about 50 mg/g to about 1000 mg/g as measured in a simulated intestinal fluid (SIF) assay.

**179.**
The pharmaceutical composition of any one of claims 171-178, wherein the manganese oxide has a uric acid clearance capacity of about 100 mg/g to about 900 mg/g as measured in a simulated intestinal fluid (SIF) assay.

**180.**

The pharmaceutical composition of any one of claims 171-179, wherein the manganese oxide has a uric acid clearance capacity of about 200 mg/g to about 800 mg/g as measured in a simulated intestinal fluid (SIF) assay.

**181.**

The pharmaceutical composition of any one of claims 171-180, wherein the manganese oxide has a uric acid clearance capacity of about 200 mg/g to about 800 mg/g as measured in a fasted state simulated intestinal fluid (FaSSIF) assay.

**182.**

The pharmaceutical composition of any one of claims 171-181, wherein the manganese oxide has a uric acid clearance capacity of about 200 mg/g to about 800 mg/g as measured in a fed state simulated intestinal fluid (FeSSIF) assay.

**183.**

The pharmaceutical composition of any one of claims 171-182, wherein the pharmaceutical composition is an oral formulation.

**184.**

The pharmaceutical composition of any one of claims 171-183, wherein the oral formulation is a solid oral formulation or a liquid oral formulation.

**185.**

The pharmaceutical composition of any one of claims 171-184, wherein the solid oral formulation is powder, granule, tablet, capsule, pill, or lozenge.

**186.**

The pharmaceutical composition of any one of claims 171-185, wherein the pharmaceutical composition does not comprise an additional therapeutic agent.

**187.**

The pharmaceutical composition of any one of claims 171-186, wherein the pharmaceutical composition comprises an effective amount of manganese oxide.

**188.**

The pharmaceutical composition of any one of claims 171-187, wherein the pharmaceutical composition comprises about 3 mg to about 3000 mg, about 3 mg to about 1500 mg, about 5 mg to about 500 mg, or about 10 mg to about 200 mg of manganese oxide.

**189.**

The pharmaceutical composition of any one of claims 171-188, wherein the mammal is a human.

**190.**

The pharmaceutical composition of any one of claims 171-189, wherein the blood and/or serum uric acid level in the mammalian subject is above 360 $\mu$mol/L or 420 $\mu$mol/L by at least about 15%, 20%, 25%, 30%, 35%, 40%, 45%, 50%, 55%, 60%, 65%, 70%, 75%, 80%, 85%, 90%, 95% or 100%.

**191.**

The pharmaceutical composition of any one of claims 171-190, wherein the blood and/or serum uric acid level in the mammalian subject is reduced by at least about 10%, 15%, 20%, 25%, 30%, 35%, 40%, 45%, 50%, 55%, 60%, 65%, 70%, 75%, 80%, 85%, 90%, 95% or 100% within 24 hours after administration of the pharmaceutical composition.

**192.**

A pharmaceutical composition formulated for oral administration to a mammalian subject in at least one solid dosage unit, wherein the solid dosage unit comprises manganese oxide, and wherein the manganese oxide has a uric acid clearance capacity measured in a fasted state simulated intestinal fluid (FaSSIF) or fed state simulated intestinal fluid (FeSSIF) assay that is at least about 100 mg/g higher than the uric acid clearance capacity of activated charcoal measured under the same conditions.

**193.**

The pharmaceutical composition of claim 192, wherein the manganese oxide has a uric acid clearance capacity measured in a fasted state simulated intestinal fluid (FaSSIF) or fed state simulated intestinal fluid (FeSSIF) assay that is at least about 200mg/g, 300mg/g, 400mg/g, 500mg/g, or 600mg/g higher than the uric acid clearance capacity of activated charcoal measured under the same conditions.

**194.**

The pharmaceutical composition of claim 192 or 193, wherein the fasted state simulated intestinal fluid (FaSSIF) or fed state simulated intestinal fluid (FeSSIF) assay is performed using fasted state simulated intestinal fluid (FaSSIF) or fed state simulated intestinal fluid (FeSSIF) with a concentration of uric acid of about 8.4 $\pm$ 0.2 mg/dL, a concentration of manganese oxide of about 0.1 g/L to 2 g/L at a temperature between about 25°C and 37°C, and under oscillating conditions.

**195.**

The pharmaceutical composition of any of claims 192-194, wherein the particle size of manganese oxide is $\geq$ 1 $\mu$m, e.g. 1 $\mu$m to 1 mm

**196.**

The pharmaceutical composition of any one of claims 192-195, wherein the manganese oxide is selected from one or more of: (1) manganese oxide consisting only of manganese atoms and oxygen atoms, such as manganese oxide selected from one or more of manganese monoxide, manganese dioxide, monomanganese trioxide, diamanganese trioxide, pentamanganese octoxide, trimanganese tetraoxide, dimanganese heptaoxide, heptamanganese dodecoxide, and heptamanganese tridecaoxide; (2) manganese oxide consisting solely of manganese atoms, hydrogen atoms and oxygen atoms, such as manganese oxide selected from one or both of manganese oxyhydroxide and manganese hydroxide; and (3) manganese oxide consisting of manganese atoms, oxygen atoms, metal atoms and optionally hydrogen atoms, such as salt-type manganese oxide.

**197.**

The pharmaceutical composition of any one of claims 192-196, wherein the salt-type manganese oxide is selected from one or more of potassium-type manganese oxide, hydrogen-type manganese oxide, ammonium-type manganese oxide, sodium-type manganese oxide, calcium-type manganese oxide, magnesium-type manganese oxide, iron-type manganese oxide, ferrous-type manganese oxide, zinc-type manganese oxide, lanthanum-type manganese oxide, bismuth-type manganese oxide, lithium-type manganese oxide, and silver-type R-form manganese oxide.

**198.**

The pharmaceutical composition of any one of claims 192-197, wherein the manganese oxide is manganese oxide in the form of a natural mineral, wherein the manganese oxide in the form of a natural mineral is, for example, selected from one or more of hollandite, cryptomelane, pyrolusite, nsutit, birnessite, buserite, vernadite, ramsdellite, romanechite, todorokite, manganite, groutite, feitknechtite, hydrohausmannite, lithiophorite, chalcophanite, hausmannite, bixbyite, pyrochroite, and manganosite.

**199.**

The pharmaceutical composition of any one of claims 192-198, wherein the manganese oxide is manganese oxide in the form of a polymorph, wherein the manganese oxide in the form of a polymorph is, for example, selected from one or more of $\alpha$-form crystalline manganese oxide, $\beta$-form crystalline manganese oxide, $\gamma$-form crystalline manganese oxide, $\delta$-form crystalline manganese oxide, $\lambda$-form crystalline manganese oxide, $\varepsilon$-form crystalline manganese oxide, R-form crystalline manganese oxide, amorphous manganese oxide, $\alpha$-form crystalline manganese oxyhydroxide, $\beta$-form crystalline manganese oxyhydroxide, and $\gamma$-form crystalline manganese oxyhydroxide.

**200.**

The pharmaceutical composition of any one of claims 192-199, wherein the manganese oxide is manganese oxide in the form of a salt-type polymorph, wherein the manganese oxide in the form of a salt-type polymorph is, for example, selected from one or more of salt-type $\alpha$-form crystalline manganese oxide, salt-type $\beta$-form crystalline manganese oxide, salt-type $\gamma$-form crystalline manganese oxide, salt-type $\delta$-form crystalline manganese oxide, salt-type $\lambda$-form crystalline manga-nese oxide, salt-type $\varepsilon$-form crystalline manganese oxide, salt-type R-form crystalline manganese oxide, and salt-type amorphous manganese oxide, and wherein the salt-type $\alpha$-form crystalline manganese oxide is, for example, selected from one or both of potassium-type $\alpha$-form crystalline manganese oxide and hydrogen-type $\alpha$-form crystalline manganese oxide, the salt-type $\beta$-form crystalline manganese oxide is, for example, selected from one or both of potassium-type $\beta$-form crystalline manganese oxide and hydrogen-type $\beta$-form crystalline manganese oxide, the salt-type $\gamma$-form crystalline

manganese oxide is, for example, selected from one or both of ammonium-type γ-form crystalline manganese oxide and hydrogen-type γ-form crystalline manganese oxide, and the salt-type δ-form crystalline manganese oxide is, for example, selected from potassium-type δ-form crystalline manganese oxide, hydrogen-type δ-form crystalline manganese oxide, sodium-type δ-form crystalline manganese oxide, ammonium-type δ-form crystalline manganese oxide, calcium-type δ-form crystalline manganese oxide, magnesium-type δ-form crystalline oxide, iron-type δ-form crystalline oxide, Manganese, ferrous δ-form crystalline manganese oxide, zinc δ-form crystalline manganese oxide, one or more of lanthanum-type δ-form crystalline manganese oxide, bismuth-type δ-form crystalline manganese oxide, lithium-type δ-form crystalline manganese oxide, and silver-type R-form δ-form crystalline manganese oxide, the salt-type λ-form crystalline manganese oxide is, for example, selected from one or both of lithium-type λ-form crystalline manganese oxide and hydrogen-type λ-form crystalline manganese oxide, the salt-type ε-form crystalline manganese oxide is, for example, selected from one or both of potassium-type ε-form crystalline manganese oxide and hydrogen-type ε-form crystalline manganese oxide, the salt-type R-form crystalline manganese oxide is, the salt-type amorphous manganese oxide is, for example, selected from one or both of potassium-type amorphous manganese oxide and hydrogen-type amorphous manganese oxide.

**201.**

The pharmaceutical composition of any one of claims 192-200, wherein the manganese oxide has a uric acid clearance capacity of about 200 mg/g to about 800 mg/g as measured in a fasted state simulated intestinal fluid (FaSSIF) assay.

**202.**

The pharmaceutical composition of any one of claims 192-201, wherein the manganese oxide has a uric acid clearance capacity of about 200 mg/g to about 800 mg/g as measured in a fed state simulated intestinal fluid (FeSSIF) assay.

**203.**

The pharmaceutical composition of any one of claims 192-202, wherein the solid dosage unit is powder, granule, tablet, capsule, pill, or lozenge.

**204.**

The pharmaceutical composition of any one of claims 192-203, wherein the pharmaceutical composition does not comprise an additional therapeutic agent.

**205.**

The pharmaceutical composition of any one of claims 192-204, wherein the pharmaceutical composition comprises an effective amount of manganese oxide.

**206.**

The pharmaceutical composition of any one of claims 192-205, wherein the pharmaceutical composition comprises about 3 mg to about 3000 mg, about 3 mg to about 1500 mg, about 5 mg to about 500 mg, or about 10 mg to about 200 mg of manganese oxide.

**207.**

The pharmaceutical composition of any one of claims 192-206, wherein the mammal is a human.

FIG. 1

FIG. 2

**FIG. 3**

**FIG. 4**

**FIG. 5**

**FIG. 6**

**FIG. 7**

**FIG. 8**

FIG. 9

FIG. 10

FIG. 11

**FIG. 12**

**FIG. 13**

**FIG. 14**

**FIG. 15**

**FIG. 16**

**FIG. 17**

**FIG. 18**

**FIG. 19**

**FIG. 20**

**FIG. 21**

**FIG. 22**

FIG. 23

# EP 4 556 011 A1

<table>
<tr><td colspan="2"><b>INTERNATIONAL SEARCH REPORT</b></td><td>International application No.<br><br><b>PCT/CN2023/107543</b></td></tr>
</table>

**A. CLASSIFICATION OF SUBJECT MATTER**

A61K33/32(2006.01)i; A61K33/00(2006.01)i; A61P19/06(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, VCN, VEN, CJFD, CNKI, ISI-WEB OF SCIENCE, PUBMED, BING, STNext, 百度学术, Baidu Scholar, 读秀, DUXIU: 上海石趣医药, SHIQU PHARMACEUTICAL, 张诗宜, 赵鑫, 曾小东, 氧化 1w 锰, 氧化锰, 硫化锰, 碳酸锰, 硅酸锰, 硼酸锰, 铁氰酸锰, 草酸锰, 亚硒酸锰, 碘酸锰, 钨酸锰, 磷酸锰, 锰矿, 锰钡矿, 布塞尔矿, 锰楣石, romanechite, todorokite, manganite, groutite, feitknechtite, hydrohausmannite, lithiophorite, chalcophanite, hausmannite, bixbyite, pyrochroite, manganosite, manganese dioxide, 不溶 3d 锰, 难溶 3d 锰, uricase, gout, gouty, hyperuricemia, 痛风, 高尿酸, 心血管疾病, 糖尿病, 胰岛素抗性, 代谢综合征, 甲状腺功能减退, 甲状旁腺功能亢进, 肥胖, 炎症, 肌肉痉挛, 局部肿胀, 关节疼痛, 肿瘤, 癌, 真性红细胞增多症, 认知障碍, 银屑病, 牛皮癣, 结节病, 非酒精性脂肪性肝, 卒中, 溶血性贫血, 先天性代谢遗传错误, 中毒, 子痫, 器官 3w 移植, 肾炎, 肾盂积水, 肾功能障碍, 肾衰, 尿毒症, 高血压, 冠心病, 心力衰竭, 先天性心脏病, 深静脉血栓, 肺栓塞, 主动脉瘤, 动脉夹层, 高脂血症, 心肌梗塞, 动脉粥样硬化

**C. DOCUMENTS CONSIDERED TO BE RELEVANT**

| Category* | Citation of document, with indication, where appropriate, of the relevant passages | Relevant to claim No. |
|---|---|---|
| X | PARMEKAR, M.V. et al. "Highly Tuned Cobalt-doped MnO2 Nanozyme as Remarkably Efficient Uricase Mimic"<br>*Applied Nanoscience*, Vol. vol. 10, 02 August 2019 (2019-08-02),<br>pp. 317-328 | 1-207 |
| X | US 2020315980 A1 (GWANGJU INSTITUTE OF SCIENCE AND TECHNOLOGY) 08 October 2020 (2020-10-08)<br>description, paragraphs 0010-0011, 0014, 0017, 0034 and 0037 | 1-207 |
| X | US 2020390806 A1 (UNIVERSITY OF FLORIDA RESEARCH FOUNDATION, INCORPORATED) 17 December 2020 (2020-12-17)<br>abstract, description, paragraphs 0007, 0017, 0028-0030 and 0042 | 1-43, 72-117, 119-207 |

☑ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **23 October 2023** | **31 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/107543** |

**C.  DOCUMENTS CONSIDERED TO BE RELEVANT**

| Category* | Citation of document, with indication, where appropriate, of the relevant passages | Relevant to claim No. |
| --- | --- | --- |
| X | CN 111821316 A (PEKING UNIVERSITY) 27 October 2020 (2020-10-27) <br> claims 1-4 and 24-30 | 1-43, 72-117, 119-207 |
| X | CN 112691120 A (PEKING UNIVERSITY) 23 April 2021 (2021-04-23) <br> claims 7-8, and description, paragraph 0023 | 1-43, 72-117, 119-207 |
| X | CN 114522222 A (TIANJIN UNIVERSITY) 24 May 2022 (2022-05-24) <br> claims 1-10 | 1-43, 72-117, 119-207 |
| X | CN 113694083 A (ANHUI MEDICAL UNIVERSITY) 26 November 2021 (2021-11-26) <br> claims 1-7, and description, paragraph 0003 | 1-43, 72-117, 119-207 |
| X | CN 105997979 A (BEIJING HEALTHGEM BIOTECHNOLOGY CO., LTD.) 12 October 2016 (2016-10-12) <br> claims 1-12 | 44 |
| PX | US 2022313732 A1 (OHM CREATIONS, LLC.) 06 October 2022 (2022-10-06) <br> claims 1-16, and description, paragraphs 0069 and 0073-0074 | 1-43, 72-117, 119-207 |

Form PCT/ISA/210 (second sheet) (July 2022)

## INTERNATIONAL SEARCH REPORT

| International application No. |
| --- |
| **PCT/CN2023/107543** |

**Box No. II     Observations where certain claims were found unsearchable (Continuation of item 2 of first sheet)**

This international search report has not been established in respect of certain claims under Article 17(2)(a) for the following reasons:

1. ☑  Claims Nos.: **116-170**
   because they relate to subject matter not required to be searched by this Authority, namely:

   The subject matter of claims 116-170 relates to a method for treatment of a human body. Therefore, said claims do not comply with PCT Rule 39.1(iv). The search report is provided on the basis of the designation of the subject matter of claims 116-170 being a corresponding pharmaceutical use.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/107543**

| Patent document cited in search report | | | Publication date (day/month/year) | Patent family member(s) | | | Publication date (day/month/year) |
|---|---|---|---|---|---|---|---|
| US | 2020315980 | A1 | 08 October 2020 | KR | 102135053 | B1 | 20 July 2020 |
| US | 2020390806 | A1 | 17 December 2020 | WO | 2019164892 | A1 | 29 August 2019 |
| | | | | US | 11179416 | B2 | 23 November 2021 |
| CN | 111821316 | A | 27 October 2020 | | None | | |
| CN | 112691120 | A | 23 April 2021 | | None | | |
| CN | 114522222 | A | 24 May 2022 | | None | | |
| CN | 113694083 | A | 26 November 2021 | | None | | |
| CN | 105997979 | A | 12 October 2016 | | None | | |
| US | 2022313732 | A1 | 06 October 2022 | US | 11759479 | B2 | 19 September 2023 |

Form PCT/ISA/210 (patent family annex) (July 2022)

# EP 4 556 011 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Current hypertension reports*, 2016, vol. 18 (10), 1-6
- *Mini Reviews in Medicinal Chemistry*, 2020, vol. 20 (18), 1857-1866
- Remington's Pharmaceutical Sciences. Mack Printing Company, 1990, 1289-1329
- *CHEMICAL ABSTRACTS*, 9050-97-9
- *CHEMICAL ABSTRACTS*, 39389-20-3
- *CHEMICAL ABSTRACTS*, 12627-85-9
- *CHEMICAL ABSTRACTS*, 9017-79-2
- *CHEMICAL ABSTRACTS*, 39339-85-0
- **KVIETYS, P.R.** ; **GRANGER, D.N.** Role of intestinal lymphatics in interstitial volume regulation and transmucosal water transport. *Annals of the New York Academy of Sciences*, 2010, vol. 1207, E29-E43, https://doi.org/10.1111/j.1749-6632.2010.05709.x
- *J. Agric. Food Chem.*, 2019, vol. 67, 220-228